# FASCICULE DE BREVET EUROPEEN

(11) **EP 2 448 939 B1**
(45) Date de publication et mention de la délivrance du brevet: **01.03.2017**
(21) Numéro de dépôt: 10742012.7
(22) Date de dépôt: 01.07.2010
(51) Int. Cl.: C07D 487/04, A61K 31/519

(54) **DERIVES DE 2,3-DIHYDRO-1H-IMIDAZO {1,2-A} PYRIMIDIN-5-ONE, LEUR PREPARATION ET LEUR UTILISATION PHARMACEUTIQUE**
2,3-DIHYDRO-1H-IMIDAZO {1,2-A} PYRIMIDIN-5-ON-DERIVATE, DEREN HERSTELLUNG UND PHARMAZEUTISCHE VERWENDUNG
2,3-DIHYDRO-1H-IMIDAZO {1,2-A} PYRIMIDIN-5-ONE DERIVATIVES, THE PREPARATION AND PHARMACEUTICAL USE THEREOF

(30) Priorité: 02.07.2009 FR 0903236; 10.09.2009 US 241097 P; 09.10.2009 FR 0957067
(43) Date de publication de la demande: 09.05.2012
(73) Titulaire: SANOFI, 75008 Paris (FR)
(72) Inventeur: BROLLO, Maurice, F-75013 Paris (FR); CLAUSS, Annie, F-75013 Paris (FR); EL AHMAD, Youssef, F-75013 Paris (FR); FILOCHE-ROMME, Bruno, F-75013 Paris (FR); HALLEY, Frank, F-75013 Paris (FR); KARLSSON, Karl Andreas, F-75013 Paris (FR); MARCINIAK, Gilbert, F-75013 Paris (FR); RONAN, Baptiste, F-75013 Paris (FR); SCHIO, Laurent, F-75013 Paris (FR); VIVET, Bertrand, F-75013 Paris (FR); VIVIANI, Fabrice, F-75013 Paris (FR); ZIMMERMANN, André, F-75013 Paris (FR)
(74) Mandataire: Nony
(86) Numéro de dépôt international: PCT/FR2010/051373
(87) Numéro de publication internationale: WO 2011/001112

(56) Documents cités:
- WO-A-02/18386
- WO-A-03/027116
- WO-A-03/072579
- WO-A-2006/109081
- WO-A-2008/148074
- WYSOCKI, WALDEMAR ET AL: "6-Benzyl-7-hydroxy-1-(2-methoxyphenyl)-2, 3-dihydro-1H,7H-imidazo[1,2- a]pyrimidin-5-one", ACTA CRYSTALLOGRAPHICA, SECTION E: STRUCTURE REPORTS ONLINE , E62(6), O2548-O2550 CODEN: ACSEBH; ISSN: 1600-5368 URL: HTTP://JOURNALS.IUCR.ORG/E/ISSUES/2006/06/ 00/BX2005/INDEX.HTML, 2006, XP002574142,

## Description

La présente invention concerne de nouveaux composés chimiques (2,3-dihydro-1H-imidazo{1,2-a} pyrimidin-5-one), dérivés de pyrimidinones, leur procédé de préparation, les nouveaux intermédiaires obtenus, leur application à titre de médicaments, les compositions pharmaceutiques les renfermant et la nouvelle utilisation de tels dérivés.

La présente invention concerne ainsi également l'utilisation desdits dérivés pour la préparation d'un médicament destiné au traitement de l'homme.

Plus particulièrement, l'invention concerne, de nouveaux dérivés de pyrimidinones et leur utilisation pharmaceutique pour la prévention et le traitement d'affections capables d'être modulées par l'inhibition de la voie PI3K/AKT/mTOR. AKT est un acteur clé dans cette voie de signalisation. Un niveau élevé de phosphorylation d'AKT est le marqueur de l'activation de la voie qui est retrouvée dans de nombreux cancers humains.

Les produits de la présente invention peuvent ainsi notamment être utilisés pour la prévention ou le traitement d'affections capables d'être modulées par l'inhibition de la phosphorylation d'AKT (P-AKT). L'inhibition de P-AKT peut être notamment obtenue par l'inhibition de la voie PI3K/AKT/mTOR, et en particulier par l'inhibition de kinases appartenant à cette voie comme les récepteurs à activité tyrosine kinase tels EGFR, IGFR, ErbB2, la 3'-phosphoinositide-dependent protein kinase-1 (PDK1), la phosphoinositide kinase PI3K, la serine-threonine kinase AKT, la kinase mTOR.

L'inhibition et la régulation de la voie PI3K/AKT/mTOR constitue notamment un nouveau et puissant mécanisme d'action pour le traitement d'un grand nombre de maladies cancéreuses incluant des tumeurs solides et liquides. De telles affections que peuvent traiter les produits de la présente demande sont les tumeurs humaines solides ou liquides.

Cette invention concerne egalement, de nouveaux dérivés de pyrimidinones et leur utilisation pharmaceutique pour la prévention et le traitement d'affections impactées par la modulation de l'autophagie. L'inhibition et la régulation de l'autophagie constitue un nouveau mécanisme d'action pour le traitement d'un grand nombre de maladies cancéreuses incluant des tumeurs solides et liquides.

Cette invention concerne egalement, de nouveaux dérivés de pyrimidinones et leur utilisation pharmaceutique pour le traitement de maladies parasitaires telles que la malaria, la maladie du sommeil, la maladie de Chagas, les leishmanioses.

### Rôle de la voie PI3K/AKT/mTOR

La voie de signalisation PI3K/AKT/mTOR est un réseau complexe qui régule de multiples fonctions cellulaires, comme la croissance, la survie, la prolifération et la motilité cellulaire, qui sont des processus clés de la tumorigénèse.

Cette voie de signalisation est une cible importante dans le traitement du cancer car la plupart de ses effecteurs sont altérés dans les tumeurs humaines. Les principaux effecteurs contribuant à l'activation de la voie sont i) les oncogènes tels que ErbB1 (EGFR), ErbB2 (HER2), PIK3CA et AKT activés par mutation, amplification ou surexpression ; ii) la déficience des gènes suppresseurs de tumeurs tels que PTEN, TSC1/2, LKB et PML qui sont inactivés suite à des mutations ou à des délétions (Jiang L-Z & Liu L-Z, Biochim Biophys Acta, 2008, 1784 :150 ; Vivanco I & Sawyers CL, 2002, Nat Rev Cancer, 2 :489 ; Cully M et al., Nature Rev. Cancer, 2006, 6 :184).

L'activation des oncogènes de cette voie de signalisation est retrouvée dans de nombreuses maladies cancéreuses humaines.
- les mutations activatrices de PIK3CA sont présentes dans 15-30% des cancers du colon, du sein, de l'endomètre, du foie, de l'ovaire et de la prostate (TL Yuan and LC Cantley, Oncogene, 2008, 27:5497; Y. Samuels et al. Science, 2004, 304:554; KE. Bachman et al. Cancer Biol Ther, 2004, 3:772; DA Levine et al. Clin Canc Res. 2005, 11:2875; C. Hartmann et al. Acta Neuropathol. 2005, 109:639).
- les amplifications, mutations activatrices et surexpressions des RTKs tels qu'EGFR et HER2 dans les cancers du cerveau, du sein et du poumon (NSCLC)
- l'amplification et la surexpression activatrice d'AKT dans les cancers du cerveau, du poumon (NSCLC), du sein, du rein, de l'ovaire et du pancréas (Testa JR. and Bellacosa A., Proct. Natl. Acad. Sci. USA 2001, 98:10983 ; Cheng et al., Proct. Natl. Acad. Sci. USA 1992, 89: 9267 ; Bellacosa et al., Int. J. Cancer, 1995, 64:280 ; Cheng et al., Proct. Natl. Acad. Sci. USA 1996, 93 :3636 ; Yuan et al., Oncogene, 2000, 19 :2324).
La déficience des gènes suppresseurs de tumeurs de cette voie de signalisation est également retouvée dans de nombreuses maladies cancéreuses humaines:
o la délétion de *PTEN* dans 50% des cancers du poumon (NSCLC), du foie, du rein, de la prostate, du sein, du cerveau, du pancréas, de l'endomètre et du colon (Maxwell GL et al. Canc. Res. 1998, 58 :2500 ; Zhou X-P et al. Amer. J. Pathol., 2002, 161 :439 ; Endersby R & Baker SJ, Oncogene, 2008, 27:5416; Li et al. Science, 1997, 275:1943; Steack PA et al., Nat. Genet., 1997, 15 :356)
o les mutations de *TSC1*/*2* dans plus de 50% des scléroses tubéreuses
o les mutations ou délétions de *LKB1* (or *STK11*) qui prédisposent aux cancers du tractus gastro-intestinal et au cancer du pancreas et qui sont retouvées en particulier dans 10-38% des adenocarcinomes du poumon (Shah U. et al. Cancer Res. 2008, 68 :3562)
o les modification de *PML* notament par translocation dans les tumeurs humaines (Gurrieri C et al, J. NAtl Cancer Inst. 2004, 96 :269).
De plus cette voie de signalisation est un facteur majeur de résistance à la chimiothérapie, la radiothérapie et à des thérapies ciblées tels que les inhibiteurs d'EGFR et HER2 par exemple (C. Sawyers et al. Nat Rev 2002).

### Role d'AKT

AKT (protéine kinase B ; PKB) est une sérine-thréonine kinase qui occupe une place centrale dans une des voies majeures de signalisation cellulaire, la voie PI3K/AKT. AKT est notamment impliquée dans la croissance, la prolifération et la survie des cellules tumorales. L'activation d'AKT se fait en deux étapes (i) par phosphorylation de la thréonine 308 (P-T308) par PDK1 et (2) par phosphorylation de la sérine 473 (P-S473) par mTORC2 (ou complexe mTOR-Rictor), résultant en une activation totale. AKT à son tour régule un grand nombre de protéines dont mTOR (mammalian target of Rapamycin), BAD, GSK3, p21, p27, FOXO ou FKHRL1 (Manning BD & Cantley LC, Cell, 2007 129 :1261). L'activation d'AKT promeut l'internalisation des nutriments, ce qui déclenche un processus de métabolisation anabolisante soutenant la croissance et la prolifération cellulaire. En particulier, AKT contrôle l'initiation de la synthèse protéique à travers une cascade d'interactions qui procède par l'intermédiaire de TSC1/2 (complexe de sclérose tubéreuse), Rheb, et TOR pour aboutir à deux cibles critiques de la voie de signalisation, p70S6K et 4EBP. AKT induit également une phosphorylation inhibitrice du facteur de transcription Forkhead et l'inactivation de GSK3β qui conduisent à l'inhibition de l'apoptose et à la progression du cycle cellulaire (Franke TF, Oncogene, 2008, 27 :6473). AKT est donc une cible pour la thérapie anti-cancéreuse et l'inhibition de l'activation d'AKT par l'inhibition de sa phosphorylation peut induire l'apoptose des cellules malignes et par la même, présenter un traitement pour le cancer.

### Les récepteurs à activité tyrosyne kinase comme IGF1R

Des niveaux anormalement élevés d'activité protéine kinase ont été impliqués dans de nombreuses maladies résultant de fonctions cellulaires anormales. Ceci peut provenir soit directement soit indirectement, d'un disfonctionnement dans les mécanismes de contrôle de l'activité kinase, lié par exemple à une mutation, une sur-expression ou une activation inappropriée de l'enzyme, ou par une sur- ou sous-production de cytokines ou des facteurs de croissance, également impliqués dans la transduction des signaux en amont ou en aval des kinases. Dans tous ces cas, une inhibition sélective de l'action des kinases laisse espérer un effet bénéfique.

Le récepteur de type 1 pour l'insulin-like growth factor (IGF-I-R) est un récepteur transmembranaire à activité tyrosine kinase qui se lie en premier lieu à l'IGFI mais aussi à l'IGFII et à l'insuline avec une plus faible affinité. La liaison de l'IGF1 à son récepteur entraîne une oligomérisation du récepteur, l'activation de la tyrosine kinase, l'autophosphorylation intermoléculaire et la phosphorylation de substrats cellulaires (principaux substrats : IRS1 et Shc). Le récepteur activé par son ligand induit une activité mitogènique dans les cellules normales. Cependant IGF-I-R joue un rôle important dans la croissance dite anormale.

Plusieurs rapports cliniques soulignent le rôle important de la voie IGF-I dans le développement des cancers humains :
IGF-I-R est souvent trouvé sur-exprimé dans de nombreux types tumoraux (sein, colon, poumon, sarcome, prostate, myelome multiple) et sa présence est souvent associée à un phénotype plus agressif.

De fortes concentrations d'IGF1 circulant sont fortement corrélées à un risque de cancer de la prostate, poumon et sein.

De plus, il a été largement documenté que IGF-I-R est nécessaire à l'établissement et au maintient du phénotype transformé in vitro comme in vivo [Baserga R, Exp. Cell. Res., 1999, 253, pages 1-6]. L'activité kinase d'IGF-I-R est essentielle à l'activité de transformation de plusieurs oncogènes: EGFR, PDGFR, l'antigène grand T du virus SV40, Ras activé, Raf, et v-Src. L'expression d'IGF-I-R dans des fibroblastes normaux induit un phénotype néoplasique, qui peut ensuite entraîner la formation de tumeur in vivo. L'expression d'IGF-I-R joue un rôle important dans la croissance indépendante du substrat. IGF-I-R a également été montré comme un protecteur dans l'apoptose induite par chimiothérapie, radiation, et l'apoptose induite par des cytokines. De plus, l'inhibition d'IGF-I-R endogène par un dominant négatif, la formation de triple hélice ou l'expression d'un antisens provoque une suppression de l'activité transformante in vitro et la diminution de la croissance de tumeurs dans les modèles animaux.

### PDK1

La 3'-phosphoinositide-dependent protein kinase-1 (PDK1) est une des composantes essentielles de la voie de signalisation PI3K-AKT. C'est une sérine-thréonine (Ser/Thr) kinase dont le rôle est de phosphoryler et d'activer d'autres Ser/Thr kinases de la famille des AGC impliquées dans le contrôle de la croissance, la prolifération, la survie cellulaire et dans la régulation du métabolisme. Ces kinases incluent la protéine kinase B (PKB ou AKT), SGK (ou serum and glucocorticoïd regulated kinase), RSK (ou p90 ribosomal S6 kinase), p70S6K (ou p70 ribosomal S6 kinase) ainsi que diverses isoformes de la protéine kinase C (PKC) (Vanhaesebroeck B. & Alessi DR., Biochem J, 2000, 346:561). Un des rôles clés de PDK1 est donc l'activation d'AKT : en présence de PIP3, le second messager généré par PI3K, PDK-1 est recruté à la membrane plasmique via son domaine PH (plekstrin homology) et phosphoryle AKT sur la thréonine 308 situé dans la boucle d'activation, une modification essentielle de l'activation d'AKT. PDK1 est exprimée de façon ubiquitaire et est une kinase constitutivement active. PDK1 est un élément clé dans la voie de signalisation PI3K/AKT pour la régulation de processus clés dans la tumorigénèse comme la prolifération et la survie cellulaire. Cette voie étant activée dans plus de 50% des cancers humains, PDK1 représente une cible pour la thérapie anticancéreuse. L'inhibition de PDK1 devrait résulter en une inhibition effective de la proliferation et de la survie des cellules cancéreuses et donc apporter un bénéfice thérapeutique pour les cancers humains (Bayascas JR, Cell cycle, 2008, 7 :2978 ; Peifer C. & Alessi DR, ChemMedChem, 2008, 3 :1810).

### Les phosphoinositides-3 kinases (PI3Ks)

La lipide kinase PI3K est une cible importante dans cette voie de signalisation pour l'oncologie. Les PI3Ks de la classe I sont réparties en classe Ia (PI3Kα,β,δ) activée par les récepteurs à activité tyrosine kinase (RTKs), les récepteurs couplés aux protéines G (GPCRs), les GTPases de la famille Rho, p21-Ras et en classe Ib (PI3Kγ) activé par les GPCRs et par p21-Ras. Les PI3Ks de la classe la sont des hétérodimères qui consistent en une sous unité catalytique p110α, β ou δ et une sous unité régulatrice p85 ou p55. La classe Ib (p110γ) est monomérique. Les PI3Ks de la classe I sont des lipides/protéines kinases qui sont activées par les RTKs, les GPCRs ou Ras après recrutement à la membrane. Ces PI3Ks de la classe I phosphorylent le phosphatidylinositol 4,5 diphosphate (PIP2) sur la position 3 de l'inositol pour donner le phosphatidylinositol 3,4,5 triphosphate (PIP3), messager secondaire clé de cette voie de signalisation. A son tour, PIP3 recrute AKT et PDK1 à la membrane où ils se fixent par leur domaine homologue à la pleckstrine (domaine PH), conduisant à l'activation d'AKT par phosphorylation de PDK1 sur la thréonine 308. AKT phosphoryle de nombreux substrats, jouant ainsi un rôle clé dans de nombreux processus aboutissant à la transformation cellulaire comme la prolifération, la croissance et la survie cellulaire ainsi que l'angiogénèse.

Les PI3Ks de classe I sont impliquées dans les cancers humains : des mutations somatiques du gène PIK3CA qui code pour PI3Kα se retrouvent dans 15-35% des tumeurs humaines avec notamment deux mutations oncogéniques principales H1047R (dans le domaine kinase) et E545K/E542K (dans le domaine hélical) (Y. Samuels et al. Science, 2004, 304:554; TL Yuan and LC Cantley, Oncogene, 2008, 27:5497). Des inhibiteurs de PI3K sont attendus efficaces pour le traitement de nombreux cancers humains présentant des altérations génétiques aboutissant à l'activation de la voie PI3K/AKT/mTOR (Vogt P. et al., Virology, 2006, 344 :131 ; Zhao L & Vogt PK, Oncogene, 2008, 27 :5486).

### mTOR

mTOR (mammalian target of rapamycin) est une serine-threonine kinase apparentée aux lipide kinases de la famille PI3K. mTOR a été impliquée dans divers processus biologiques incluant la croissance, la prolifération, la motilité et la survie cellulaires. mTOR est une kinase multifonctionnelle intégrant à la fois les signaux venant des facteurs de croissance et ceux venant des nutriments pour réguler la traduction des protéines, la capture des nutriments, l'autophagie et la fonction mitochondriale. mTOR existe sous la forme de deux complexes différent appelés mTORC1 et mTORC2. mTORC1 contient la sous-unité raptor et mTORC2, la sous-unité rictor. Ces deux complexes sont régulés de façon différente: mTORC1 phosphoryle la kinase S6 (S6K) et 4EBP1, stimulant ainsi la traduction et la biogenèse des ribosomes pour faciliter la croissance des cellules et la progression dans le cycle cellulaire. S6K agit aussi dans une voie de rétro-contrôle pour atténuer l'activation d'AKT. mTORC1 est sensible à la rapamycine alors que mTORC2 est généralement insensible à la rapamycine. mTORC2 modulerait la signalisation des facteurs de croissance en phosphorylant AKT sur le résidu serine 473. mTOR a été impliquée dans diverses pathologies incluant notamment le cancer, le diabète, l'obésité, les maladies cardio-vasculaires et les désordres neurologiques. mTOR module de nombreux processus biologiques incluant la traduction, l'autophagie, et la biogenèse des ribosomes en intégrant des signaux intra et extra-cellulaires comme les signaux transportés par les facteurs de croissance, les nutriments, les niveau d'énergie et le stress cellulaire (Guertin D.A. and Sabatini D., Cancer Cell, 2007, 12 :9 ; Menon S. and Manning B.D., Oncogene, 2009, 27 :S43).

### Role de l'autophagie

L'autophagie est un mécanisme de dégradation intracellulaire (organelles, protéines de longues vies...) dépendant des lysosomes. Le processus autophagie implique la formation de vésicules particulières appelées autophagosomes. La lipide kinase PI3K de classe III (Vps34) est impliquée dans la formation des autophagosomes. Cette PI3K de la classe III phosphoryle le phosphatidylinositol (PI) sur la position 3 de l'inositol pour donner le phosphatidylinositol 3 triphosphate (PI3P). Le PI3P est un messager secondaire clé dans la formation des autophagosomes via le recrutement de protéines telles que WIPI, DFCP1 et Alfy. L'autophagie est un mécanisme de survie cellulaire permettant à la cellule de survivre en situation de stress, comme par exemple face à un stress métabolique. Dans le cas du cancer, l'autophagie est impliquée dans la résistance des cellules tumorales face aux stress envirronnementaux tels que : l'hypoxie, les stress oxydatifs, la carence en nutriments, mais aussi face aux stress thérapeutiques : traitements par agents anticancéreux, radiations ionisantes.

### Application en chimiothérapie anti-paludique

Le paludisme est l'une des premières causes infectieuses de mortalité au monde et touche chaque année 100 à 200 millions de personnes. La forte recrudescence de la maladie observée depuis quelques années est due à plusieurs facteurs, dont :
- les vecteurs, à savoir les anophèles, qui deviennent résistants aux insecticides classiques et bon marché,
- l'augmentation de la population dans les zones à risque et, principalement,
- la résistance de nombreuses souches de Plasmodium falciparum, parasite responsable des formes mortelles de la maladie, aux médicaments classiquement utilisés, tels que la chloroquine et la méfloquine.

La propagation de la résistance parmi les souches de Plasmodium, en particulier P. falciparum, contre la plupart des médicaments anti-paludéens démontre le besoin urgent de développer de nouveaux composés possédant un nouveau mode d'action et permettant ainsi une diminution du risque de résistance croisée. Les kinases humaines sont des cibles validées dans le traitement de nombreuses pathologies et le kinome de P. falciparum a été proposé comme un réservoir de nouvelles cibles pour le développement de nouveaux médicaments, non encore explorées dans le traitement du paludisme.

Le kinome de Plasmodium falciparum est composé de 64 kinases, dont certaines sont orthologues de kinases humaines. Des inhibiteurs des voies de signalisation kinases ont été testés pour leur capacité à inhiber in vitro et in vivo la croissance de P. falciparum et d'autres espècess pathogènes à l'origine du paludisme.

Les molécules de l'invention inhibent la croissance de P. falciparum (hautement résistant à la chloroquine souche Fcm29-Cameroun) à 1 uM et 0,1 uM dans un test in vitro utilisant des érythrocytes humains infectés, comme indiqué dans le tableau 2.

Des kinomes similaires sont présents dans toutes les espèces de Plasmodium, tels que P. falciparum, P. vivax, P. malariae, P. ovale et P. knowlesi. Les composés de l'invention peuvent donc être utiles dans le traitement du paludisme induit par tous les parasites mentionnés ci-dessus. En outre, les kinases se trouvent dans d'autres parasites, tels que Trypanosoma (par exemple T. brucei, T. cruzei) et Leishmania (par exemple L. major, L. donovani). Les composés de l'invention peuvent donc être utilisés dans le traitement de la maladie du sommeil, la maladie de Chagas, les différentes formes de leishmaniose et d'autres infections parasitaires.

Des dérivés Morpholino pyrimidinones inhibiteurs de kinases sont connus de l'homme de l'art.

L'application WO2008/148074 décrit des produits qui possèdent une activité inhibitrice de mTOR. Ces produits sont des pyrido[1,2-a]pyrimidin-4-ones qui diffèrent des produits de la présente invention en raison de leur caractère entièrement aromatique et de leurs substitutions.

L'application WO2008/064244 décrit l'application des produits TGX-221 et TGX-155 inhibiteurs de PI3Kβ utiles dans le traitement du cancer et notamment dans le cancer du sein. Ces produits sont des pyrido[1,2-a]pyrimidin-4-ones précédemment décrits dans les applications WO2004/016607 et WO2001/053266 qui diffèrent des produits de la présente invention en raison de leur caractère entièrement aromatique et de leurs substitutions.

Les applications WO2006/109081, WO2006/109084 et WO2006/126010 décrivent des produits inhibiteurs de DNA-PK utiles pour le traitement des cancers ATM déficients. Ces produits sont des pyrido[1,2-a]pyrimidin-4-ones qui diffèrent des produits de la présente invention en raison de leur caractère entièrement aromatique et de leurs substitutions

L'application WO2003/024949 décrit des produits inhibiteurs de DNA-PK utiles pour le traitement des cancers ATM déficients. Ces produits sont des pyrido[1,2-a]pyrimidin-4-ones qui diffèrent des produits de la présente invention en raison de leur caractère entièrement aromatique et de leurs substitutions.

On connaît également, des applications WO 03/027116, WO02/18386 et WO03/072579, des dérivés pyrimidone régulant l'activité de GSK3β ou GSK3β et cdk5/p25, dans le cas de maladies neurodégénératives comme la maladie d'Alzheimer. Ces produits sont des pyrimidin-4-ones et des pyrimidin-5(1H)ones qui diffèrent des produits de la présente invention en raison de la nature de leur chaîne carbonée et de leurs substitutions.

La présente invention a pour objet les produits de formule (I): 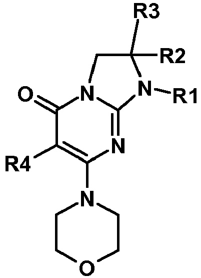 dans laquelle :
R1 représente un radical -L-aryle ou -L-hétéroaryle, tel que L représente : soit un radical alkyle linéaire ou ramifié renfermant de 1 à 6 atomes de carbone et éventuellement substitué par un radical hydroxyle,
   soit un groupe CO,
soit un groupe L'-X où L' représente un radical alkyle linéaire ou ramifié renfermant de 1 à 6 atomes de carbone et X un atome d'oxygène ou de soufre ;
les radicaux aryle et hétéroaryle étant éventuellement substitués par un ou plusieurs radicaux identiques ou différents choisis parmi les atomes d'halogène et les radicaux hydroxyle, CN, nitro, -COOH, -COOalk, -NRxRy, - CONRxRy, -NRxCORy, -NRxCO₂Rz, -CORy, alcoxy, phénoxy, alkylthio, alkyle, cycloalkyle et hétérocycloalkyle ;
ces derniers radicaux alcoxy, phénoxy, alkylthio, alkyle et hétérocycloalkyle, étant eux-mêmes éventuellement substitués par un ou plusieurs radicaux identiques ou différents choisis parmi les atomes d'halogène et NRvRw ;
les radicaux hétérocycloalkyle et hétéroaryle pouvant de plus renfermer un radical oxo ;
R2 représente un atome d'hydrogène ou un radical alkyle ;
R3 représente un radical alkyle éventuellement substitué par un ou plusieurs atomes d'halogène ;
R4 représente un atome d'hydrogène ou un atome d'halogène ;
NRxRy étant tel que Rx représente un atome d'hydrogène ou un radical alkyle et Ry représente un atome d'hydrogène, un radical cycloalkyle ou un radical alkyle éventuellement substitué par un ou plusieurs radicaux identiques ou différents choisis parmi les radicaux hydroxyle, alcoxy, NRvRw et hétérocycloalkyle ; soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
NRvRw étant tel que Rv représente un atome d'hydrogène ou un radical alkyle et Rw représente un atome d'hydrogène, un radical cycloalkyle ou un radical alkyle éventuellement substitué par un ou plusieurs radicaux identiques ou différents choisis parmi les radicaux hydroxyle, alcoxy, hétérocycloalkyle ; soit Rv et Rw forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
les radicaux cycliques que peuvent former Rx et Ry ou Rv et Rw respectivement avec l'atome d'azote auquel ils sont liés, étant éventuellement substitués par un ou plusieurs radicaux identiques ou différents choisis parmi les atomes d'halogène, les radicaux alkyle, hydroxyle, oxo, alcoxy, NH2; NHalk et N(alk)2 ;
Rz représente les valeurs de Ry à l'exception de hydrogène ;
Rx, Ry et Rz dans les radicaux -NRxCORy, -CORy et NRxCO₂Rz étant choisis parmi les significations indiquées ci-dessus pour Rx, Ry, et Rz ;
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Dans les produits de formule (I) :
- le terme radical alkyle (ou alk) désigne les radicaux, linéaires et le cas échéant ramifiés, méthyle, éthyle, propyle, isopropyle, butyle, isobutyle, sec-butyle, tert-butyle, pentyle, isopentyle, hexyle, isohexyle et également heptyle, octyle, nonyle et décyle ainsi que leurs isomères de position linéaires ou ramifiés : on préfère les radicaux alkyles renfermant de 1 à 6 atomes de carbone et plus particulièrement les radicaux alkyle renfermant de 1 à 4 atomes de carbone de la liste ci-dessus ;
- le terme radical alcoxy désigne les radicaux linéaires et le cas échéant ramifiés, méthoxy, éthoxy, propoxy, isopropoxy, butoxy linéaire, secondaire ou tertiaire, pentoxy ou hexoxy ainsi que leurs isomères de position linéaires ou ramifiés : on préfère les radicaux alkoxy renfermant de 1 à 4 atomes de carbone de la liste ci-dessus ;
- le terme radical alkylthio désigne les radicaux linéaires et le cas échéant ramifiés, méthylthio, éthylthio, propylthio , isopropylthio, butylthio linéaire, secondaire ou tertiaire, pentylthio ou hexylthio ainsi que leurs isomères de position linéaires ou ramifiés : on préfère les radicaux alkylthio renfermant de 1 à 4 atomes de carbone de la liste ci-dessus ;
- le terme atome d'halogène désigne les atomes de chlore, de brome, d'iode ou de fluor et de préférence l'atome de chlore, de brome ou de fluor.
- le terme radical cycloalkyle désigne un radical carbocyclique saturé renfermant 3 à 10 atomes de carbone et désigne ainsi notamment les radicaux cyclopropylee, cyclobutyle, cyclopentyle et cyclohexyle et tout particulièrement les radicaux cyclopropyle, cyclopentyle et cyclohexyle ;
- dans le radical -O-cycloalkyle, cycloalkyle est tel que défini ci-dessus ;
- le terme radical hétérocycloalkyle désigne ainsi un radical carbocyclique monocyclique ou bicyclique, renfermant de 3 à 10 chaînons interrompu par un ou plusieurs hétéroatomes, identiques ou différents, choisis parmi les atomes d'oxygène, d'azote ou de soufre : on peut citer par exemple les radicaux morpholinyle, thiomorpholinyle, homomorpholinyle, aziridyle, azétidyle, pipérazinyle, pipéridyle, homopipérazinyle, pyrrolidinyle, imidazolidinyle, pyrazolidinyle, tétrahydrofuryle, tétrahydrothiényle, tétrahydropyranne, oxodihydropyridazinyle, ou encore oxétanyle tous ces radicaux étant éventuellement substitués ; on peut citer notamment les radicaux morpholinyle, thiomorpholinyle, homomorpholinyle, pipérazinyle, pipéridyle, homopipérazinyle ou encore pyrrolidinyle,
- les termes aryle et hétéroaryle désignent des radicaux insaturés ou partiellement insaturés, respectivement carbocycliques et hétérocycliques, monocycliques ou bicycliques, renfermant au plus 12 chaînons, pouvant éventuellement contenir un chaînon -C(O), les radicaux hétérocycliques contenant un ou plusieurs hétéroatomes identiques ou différents choisis parmi O, N, ou S avec N, le cas échéant, éventuellement substitué ;
- le terme radical aryle désigne ainsi des radicaux monocycliques ou bicycliques renfermant 6 à 12 chaînons tels que par exemple les radicaux phényle, naphtyle, biphényle, indényle, fluorényle et anthracényle, plus particulièrement les radicaux phényle et naphtyle et encore plus particulièrement le radical phényle. On peut noter qu'un radical carbocyclique contenant un chaînon -C(O) est par exemple le radical tétralone ;
- le terme radical hétéroaryle désigne ainsi des radicaux monocycliques ou bicycliques renfermant 5 à 12 chaînons : des radicaux hétéroaryles monocycliques tels que par exemple les radicaux thiényle tel que 2-thiényle et 3-thiényle, furyle tel que 2-furyle, 3-furyle, pyrannyle, pyrrolyle, pyrrolinyle, pyrazolinyle, imidazolyle, pyrazolyle, pyridyle tel que 2-pyridyle, 3-pyridyle et 4-pyridyle, pyrazinyle, pyrimidinyle, pyridazinyle, oxazolyle, thiazolyle, isothiazolyle, diazolyle, thiadiazolyle, thiatriazolyle, oxadiazolyle, isoxazolyle tel que 3- ou 4-isoxazolyle, furazannyle, tétrazolyle libre ou salifié, tous ces radicaux étant éventuellement substitués parmi lesquels plus particulièrement les radicaux thiényle tel que 2-thiényle et 3-thiényle, furyle tel que 2-furyle, pyrrolyle, pyrrolinyle, pyrazolinyle, imidazolyle, pyrazolyle, oxazolyle, isoxazolyle, pyridyle, pyridazinyle, ces radicaux étant éventuellement substitués ; des radicaux hétéroaryles bicycliques tels que par exemple les radicaux benzothiényle tel que 3-benzothiényle, benzothiazolyle, quinolyle, isoquinolyle, dihydroquinolyle, quinolone, tétralone, adamentyl, benzofuryle, isobenzofuryle, dihydrobenzofuranne, éthylènedioxyphényle, thianthrényle, benzopyrrolyle, benzimidazolyle, benzoxazolyle, thionaphtyle, indolyle, azaindolyle, indazolyle, purinyle, thiénopyrazolyle, tétrahydroindazolyle, tétrahydrocyclopentapyrazolyle, dihydrofuropyrazolyle, tétrahydropyrrolopyrazolyle, oxotétrahydropyrrolopyrazolyle, tétrahydropyranopyrazolyle, tétrahydropyridinopyrazolyle ou oxodihydropyridino-pyrazolyle, tous ces radicaux étant éventuellement substitués ;

Comme exemples de radicaux hétéroaryles ou bicycliques, on peut citer plus particulièrement les radicaux pyrimidinyle, pyridyle, pyrrolyle, azaindolyle, indazolyle ou pyrazolyle, benzothiazolyle ou benzimidazolyle éventuellement substitués par un ou plusieurs substituants identiques ou différents comme indiqué ci-dessus.

Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus.

La présente invention a pour objet les produits de formule (I) tels que définis ci-dessus dans laquelle :
R1 représente un radical -L-phényle ou -L-hétéroaryle, tel que L représente : soit un radical alkyle linéaire ou ramifié renfermant de 1 à 6 atomes de carbone et éventuellement substitué par un radical hydroxyle,
   soit un groupe CO,
soit un groupe L'-X où L' représente un radical alkyle linéaire ou ramifié renfermant de 1 à 6 atomes de carbone et X un atome d'oxygène ou de soufre ;
les radicaux phényle et hétéroaryle étant éventuellement substitués par un ou plusieurs radicaux identiques ou différents choisis parmi les atomes d'halogène et les radicaux -NRxRy, alcoxy et alkyle ;
ces derniers radicaux alcoxy et alkyle étant eux-mêmes éventuellement substitués par un ou plusieurs radicaux choisis parmi les atomes d'halogène ;
R2 représente un radical alkyle ;
R3 représente un radical alkyle éventuellement substitué par un ou plusieurs atomes d'halogène ;
R4 représente un atome d'hydrogène ou un atome de fluor ;
NRxRy étant tel que Rx représente un atome d'hydrogène ou un radical alkyle et Ry représente un atome d'hydrogène ou un radical alkyle; soit Rx et Ry forment avec l'atome d'azote auquel ils sont liés un radical morpholino ;
tous les radicaux alkyle (alk) ou alcoxy ci-dessus étant linéaires ou ramifiés et renfermant de 1 à 6 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Notamment, lorsque NRxRy ou NRvRw forme un cycle comme défini ci-dessus, un tel cycle aminé peut être choisi notamment parmi les radicaux pyrrolidinyle, pyrazolidinyle, pyrazolinyle, pipéridyle, azépinyle, morpholinyle, homomorpholinyle, pipérazinyle ou homopipérazinyle, ces radicaux étant eux-mêmes éventuellement substitués comme indiqué ci-dessus ou ci-après.

Le cycle NRxRy ou NRvRw peut plus particulièrement être choisi parmi les radicaux pyrrolidinyle, morpholinyle éventuellement substitué par un ou deux radicaux alkyle ou pipérazinyle éventuellement substitué sur le second atome d'azote par un radical alkyle, phényle, ou et CH2-phényle, eux-mêmes éventuellement substitués par un ou plusieurs radicaux identiques ou différents choisis parmi les atomes d'halogène et les radicaux alkyle, hydroxyle et alcoxy.

La présente invention a tout particulièrement pour objet les produits de formule (I) tels que définis ci-dessus répondant aux formules suivantes :
- (2S)-1-[2-(4-méthoxyphényl)éthyl]-2-méthyl-7-(morpholin-4-yl)-2-(trifluorométhyl)-2,3-dihydroimidazo[1,2-a]pyrimidin-5(1H)-one
- 1-[2-(4-méthoxyphényl)éthyl]-2-méthyl-7-(morpholin-4-yl)-2-(trifluorométhyl)-2,3-dihydroimidazo[1,2-a]pyrimidin-5(1H)-one
- (2S)-1-benzyl-2-méthyl-7-(morpholin-4-yl)-2-(trifluorométhyl)-2,3-dihydroimidazo[1,2-a]pyrimidin-5(1H)-one
- (2S)-2-méthyl-7-(morpholin-4-yl)-1-(2-phényléthyl)-2-(trifluorométhyl)-2,3-dihydroimidazo[1,2-a]pyrimidin-5(1H)-one
- (2S)-2-méthyl-7-(morpholin-4-yl)-1-(3-phénylpropyl)-2-(trifluorométhyl)-2,3-dihydroimidazo[1,2-a]pyrimidin-5(1H)-one
- (2S)-2-méthyl-7-(morpholin-4-yl)-1-(2-phénoxyéthyl)-2-(trifluorométhyl)-2,3-dihydroimidazo[1,2-a]pyrimidin-5(1H)-one
- (2S)-2-méthyl-7-(morpholin-4-yl)-1-[2-(phénylsulfanyl)éthyl]-2-(trifluorométhyl)-2,3-dihydroimidazo[1,2-a]pyrimidin-5(1H)-one
- (2S)-2-méthyl-7-(morpholin-4-yl)-1-[(2R)-2-phénylpropyl]-2-(trifluorométhyl)-2,3-dihydroimidazo[1,2-a]pyrimidin-5(1H)-one
- (2S)-2-méthyl-7-(morpholin-4-yl)-1-[(2S)-2-phénylpropyl]-2-(trifluorométhyl)-2,3-dihydroimidazo[1,2-a]pyrimidin-5(1H)-one
- (2S)-1-[(2S)-2-hydroxy-2-phényléthyl]-2-méthyl-7-(morpholin-4-yl)-2-(trifluorométhyl)-2,3-dihydroimidazo[1,2-a]pyrimidin-5(1H)-one
- (2S)-1-[(2R)-2-hydroxy-2-phényléthyl]-2-méthyl-7-(morpholin-4-yl)-2-(trifluorométhyl)-2,3-dihydroimidazo[1,2-a]pyrimidin-5(1H)-one
- (2S)-2-méthyl-7-(morpholin-4-yl)-1-[(2S)-1-phénylpropan-2-yl]-2-(trifluorométhyl)-2,3-dihydroimidazo[1,2-a]pyrimidin-5(1H)-one
- (2S)-2-méthyl-7-(morpholin-4-yl)-1-[(1S)-1-phénylpropyl]-2-(trifluorométhyl)-2,3-dihydroimidazo[1,2-a]pyrimidin-5(1H)-one
- (2S)-2-méthyl-7-(morpholin-4-yl)-1-[(1R)-1-phénylpropyl]-2-(trifluorométhyl)-2,3-dihydroimidazo[1,2-a]pyrimidin-5(1H)-one
- (2S)-2-méthyl-7-(morpholin-4-yl)-1-{2-[4-(morpholin-4-yl)phényl]éthyl}-2-(trifluorométhyl)-2,3-dihydroimidazo[1,2-a]pyrimidin-5(1H)-one
- (2S)-2-méthyl-7-(morpholin-4-yl)-1-(1-phényléthyl)-2-(trifluorométhyl)-2,3-dihydroimidazo[1,2-a]pyrimidin-5(1H)-one
- 1-[2-(4-méthoxyphényl)éthyl]-2,2-diméthyl-7-(morpholin-4-yl)-2,3-dihydroimidazo[1,2-a]pyrimidin-5(1H)-one
ainsi que les sels d'addition avec les acides minéraux et organiques ou avec les bases minérales et organiques desdits produits de formule (I).

La présente invention a notamment pour objet les produits de formule (I) tels que définis ci-dessus répondant aux formules suivantes :
- (2S)-6-fluoro-1-[2-(4-méthoxyphényl)éthyl]-2-méthyl-7-(morpholin-4-yl)-2-(trifluorométhyl)-2,3-dihydroimidazo[1,2-a]pyrimidin-5(1H)-one
- (2S)-1-benzyl-6-fluoro-2-méthyl-7-(morpholin-4-yl)-2-(trifluorométhyl)-2,3-dihydroimidazo[1,2-a]pyrimidin-5(1H)-one
- (2S)-6-fluoro-1-[2-(4-méthoxyphényl)éthyl]-2-méthyl-7-(morpholin-4-yl)-2-(trifluorométhyl)-2,3-dihydroimidazo[1,2-a]pyrimidin-5(1H)-one
- (2S)-1-benzyl-6-fluoro-2-méthyl-7-(morpholin-4-yl)-2-(trifluorométhyl)-2,3-dihydroimidazo[1,2-a]pyrimidin-5(1H)-one
- (2S)-1-[(5-chloro-1-benzothiophén-3-yl)méthyl]-2-méthyl-7-(morpholin-4-yl)-2-(trifluorométhyl)-2,3-dihydroimidazo[1,2-a]pyrimidin-5(1H)-one
-(2S)-2-méthyl-7-(morpholin-4-yl)-1-(phénylcarbonyl)-2-(trifluorométhyl)-2,3-dihydroimidazo[1,2-a]pyrimidin-5(1H)-one
- (2S)-1-[(1R ou 1S)-1-(3-fluorophényl)éthyl]-2-méthyl-7-(morpholin-4-yl)-2-(trifluorométhyl)-2,3-dihydroimidazo[1,2-a]pyrimidin-5(1H)-one
-trifluoro acétate de (2S)-1-{[4-chloro-2-(trifluorométhyl)quinoléin-6-yl]méthyl}-2-méthyl-7-(morpholin-4-yl)-2-(trifluorométhyl)-2,3-dihydroimidazo[1,2-a]pyrimidin-5(1H)-one
-(2S)-1-(3-bromo-4-fluorobenzyl)-2-méthyl-7-(morpholin-4-yl)-2-(trifluorométhyl)-2,3-dihydroimidazo[1,2-a]pyrimidin-5(1H)-one
- (2S)-1-(2,3-difluorobenzyl)-2-méthyl-7-(morpholin-4-yl)-2-(trifluorométhyl)-2,3-dihydroimidazo[1,2-a]pyrimidin-5(1H)-one
-(2S)-1-[2-(3-méthoxyphényl)éthyl]-2-méthyl-7-(morpholin-4-yl)-2-(trifluorométhyl)-2,3-dihydroimidazo[1,2-a]pyrimidin-5(1H)-one
- (2S)-1-[2-(2-chlorophényl)éthyl]-2-méthyl-7-(morpholin-4-yl)-2-(trifluorométhyl)-2,3-dihydroimidazo[1,2-a]pyrimidin-5(1H)-one
- (2S)-1-[2-(4-chlorophényl)éthyl]-2-méthyl-7-(morpholin-4-yl)-2-(trifluorométhyl)-2,3-dihydroimidazo[1,2-a]pyrimidin-5(1H)-one
- (2S)-1-[2-(3-chlorophényl)éthyl]-2-méthyl-7-(morpholin-4-yl)-2-(trifluorométhyl)-2,3-dihydroimidazo[1,2-a]pyrimidin-5(1H)-one
- (2S)-1-(1,3-benzoxazol-2-ylméthyl)-2-méthyl-7-(morpholin-4-yl)-2-(trifluorométhyl)-2,3-dihydroimidazo[1,2-a]pyrimidin-5(1H)-one
- (2S)-2-méthyl-7-(morpholin-4-yl)-1-[(1 R ou 1 S)-1-phényléthyl]-2-(trifluorométhyl)-2,3-dihydroimidazo[1,2-a]pyrimidin-5(1H)-one
- (2S)-2-méthyl-7-(morpholin-4-yl)-1-[(1 R ou 1 S)-1-phényléthyl]-2-(trifluorométhyl)-2,3-dihydroimidazo[1,2-a]pyrimidin-5(1H)-one
- (2S)-1-(1 H-indol-3-ylméthyl)-2-méthyl-7-(morpholin-4-yl)-2-(trifluorométhyl)-2,3-dihydroimidazo[1,2-a]pyrimidin-5(1H)-one
- (2S)-1-[(2-chlorophényl)carbonyl]-2-méthyl-7-(morpholin-4-yl)-2-(trifluorométhyl)-2,3-dihydroimidazo[1,2-a]pyrimidin-5(1H)-one
- (2S)-2-méthyl-1-[(2-méthylphényl)carbonyl]-7-(morpholin-4-yl)-2-(trifluorométhyl)-2,3-dihydroimidazo[1,2-a]pyrimidin-5(1H)-one
- (2S)-1-[(1 R ou 1S)-1-(2-fluorophényl)éthyl]-2-méthyl-7-(morpholin-4-yl)-2-(trifluorométhyl)-2,3-dihydroimidazo[1,2-a]pyrimidin-5(1H)-one
- (2S)-1-[(1 R ou 1S)-1-(2-fluorophényl)éthyl]-2-méthyl-7-(morpholin-4-yl)-2-(trifluorométhyl)-2,3-dihydroimidazo[1,2-a]pyrimidin-5(1H)-one
- (S)-1-[2-(2-Fluoro-4,5-dimethoxy-phenyl)-2-hydroxy-ethyl]-2-methyl-7-morpholin-4-yl-2-trifluoromethyl-2,3-dihydro-1H-imidazo[1,2-a]pyrimidin-5-one
- (S)-1-[(S)-2-Hydroxy-2-(2-methoxy-phenyl)-ethyl]-2-methyl-7-morpholin-4-yl-2-trifluoromethyl-2,3-dihydro-1H-imidazo[1,2-a]pyrimidin-5-one
(S)-1-[(S)-2-(4-Chloro-2-methoxy-phenyl)-2-hydroxy-ethyl]-2-methyl-7-morpholin-4-yl-2-trifluoromethyl-2,3-dihydro-1H-imidazo[1,2-a]pyrimidin-5-one
(S)-1-[(S)-2-(4-Fluoro-2-methoxy-phenyl)-2-hydroxy-ethyl]-2-methyl-7-morpholin-4-yl-2-trifluoromethyl-2,3-dihydro-1H-imidazo[1,2-a]pyrimidin-5-one
(S)-1-[(S)-2-(2-Chloro-4-methoxy-phenyl)-2-hydroxy-ethyl]-2-methyl-7-morpholin-4-yl-2-trifluoromethyl-2,3-dihydro-1H-imidazo[1,2-a]pyrimidin-5-one
ainsi que les sels d'addition avec les acides minéraux et organiques ou avec les bases minérales et organiques desdits produits de formule (I).

La présente invention a encore pour objet tout procédé de préparation des produits de formule (I) tels que définis ci-dessus.

Les produits selon l'invention peuvent être préparés à partir de méthodes conventionnelles de chimie organique.

### Préparation de composés de formule (I)

Le schéma général 1 ci-dessous est illustratif des méthodes utilisées pour la préparation des produits de formule (I). A ce titre, elles ne sauraient constituer une limitation de la portée de l'invention, en ce qui concerne les méthodes de préparation des composés revendiqués.

Les produits de formule (I) tels que définis ci-dessus selon la présente invention peuvent ainsi notamment être préparés selon le procédé décrit dans le schéma général 1.

La présente invention a ainsi également pour objet le procédé de préparation de produits de formule (I) selon le schéma général 1 tel que défini ci-après. 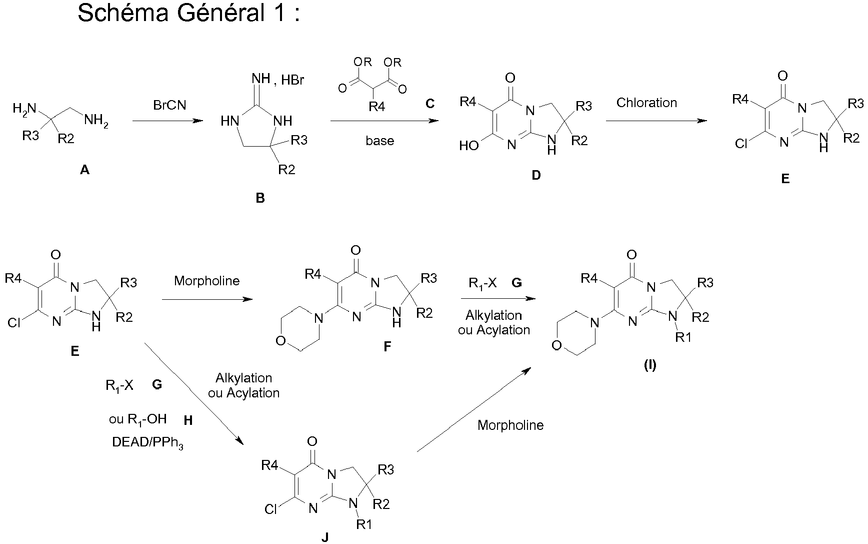 Dans le Schéma Général 1 :

Les diamines A sont soit commerciales, soit préparées, en version chirale ou racémique, selon le procédé décrit par Brigaud, T. et coll. dans J. Org. Chem. 2006, 71(18), 7075-7078, lorsque R2 = CF3 et R3 = Me ou par analogie avec cette même référence dans les autres cas.

Les guanidines B peuvent être obtenus par réaction d'une diamine A et du bromure de cyanogène dans un solvant tel l'eau ou l'acétonitrile, à une température comprise entre 0°C et le point d'ébullition du solvant, selon les conditions décrites par exemple par Gallet, T. et coll. (EP1340761 2003).

Les composés D peuvent être obtenus par condensation d'une guanidine B avec un malonate de dialkyle (de préférence de diéthyle) C, en présence d'une base telle que le méthylate de sodium, à une température comprise entre 60°C et 100°C, comme décrit par exemple par Badawey E.-S.A.M. et coll. (Eur J Med Chem, 1998, 33(5), 349-361.

Les composés E peuvent être obtenus à partir d'un composé D par traitement avec un agent de chloration tel que l'oxychlorure de phosphore, en l'absence de solvant, à une température comprise entre 20°C et 120°C, ou en présence d'un solvent tel que le dichloroéthane, à une température comprise entre 20°C et la température d'ébullition du solvant, comme par exemple dans les conditions décrites par Yamashita, A. et coll. (Syn. Commun. (2004), 34(5), 795-803)

Les composés F peuvent être obtenus à partir d'un composé E par réaction avec la morpholine, en l'absence de solvant, à une température comprise entre 20°C et 120°C, ou en présence d'un solvant tel que l'acétonitrile, à une température comprise entre 20°C et la température de reflux du solvant, comme décrit par exemple par Aliabiev S.B. (Lett. Org. Chem. (2007), 4(4), 273-280.

Les composés (I) peuvent être obtenus par une réaction d'alkylation ou d'acylation, par addition d'un composé G (R1-X avec R1 = L-Aryl ou Hétéroaryl tel que défini ci-dessus et X = Cl, Br, I ou OTf dans le cas d'une alkylation et X = Cl dans le cas d'une acylation) sur un mélange d'un composé F et d'une base telle que l'hydrure de sodium ou le carbonate de césium en excès, dans un solvant tel que le tétrahydrofuranne, la N,N-diméthylformamide ou l'acétonitrile, à une température comprise entre 0°C et 80°C, tel que décrit par exemple par Ting P.C. et coll. (J. Med. Chem. (1990), 33(10), 2697-2706) dans le cas de la réaction d'alkylation.

En suivant la procédure décrite par E. P. Seest et al. dans Tet. Assymetry 17 (2006) 2154-2182, les composés G correspondants à des 1-aryl-2-chloroéthanols ou 1-hétéroaryl-2-chloroéthanols chiraux on été synthétisés à partir des dérivés chlorocétone correspondants qui sont eux-mêmes issues de la chloration dans des conditions standards des dérivés acétyles disponibles commercialement.

Alternativement, les composés (I) peuvent être obtenus à partir d'un composé J par réaction avec la morpholine, en l'absence de solvant, à une température comprise entre 20°C et 120°C, ou en présence d'un solvant, tel que l'acétonitrile, à une température comprise entre 20°C et la température de reflux du solvant, comme décrit par exemple par Aliabiev S.B. (Lett. Org. Chem. (2007), 4(4), 273-280.
les composés J peuvent être obtenus par une réaction d'alkylation ou d'acylation, par addition d'un composé G (R1-X avec R1 = L-Aryl ou Hétéroaryl tel que défini ci-dessus et X = Cl, Br, I ou OTf dans le cas d'une alkylation et X = Cl dans le cas d'une acylation) sur un mélange d'un composé F et d'une base telle que l'hydrure de sodium ou le carbonate de césium en excès, dans un solvant tel que le tétrahydrofuranne, la N,N-diméthylformamide ou l'acétonitrile, à une température comprise entre 0°C et 80°C, tel que décrit par exemple par Ting P.C. et coll. (J. Med. Chem. (1990), 33(10), 2697-2706) dans le cas de la réaction d'alkylation.

Alternativement, les composés J peuvent être obtenus par réaction de Mitsunobu entre un composé E et un alcool H, en présence de l'azo-dicarboxylate de diéthyle et de triphénylphosphine (éventuellement supportée sur résine), dans un solvant tel que le tétrahydrofuranne, à une température comprise entre 0°C et 65°C, tel que décrit par exemple par Mitsunobu O. et coll. (Synthesis (1981), 1-28).

Dans les cas où R2 est différent de R3 et si la synthèse n'est pas stéréosélective, les énantiomères ou les éventuels diastéréoisomères des intermédiaires de synthèse ou des composés (I) peuvent être séparés par chromatographie sur support chiral.

Parmi les produits de départs de formule A, B ou C certains sont connus et peuvent être obtenus soit commercialement, soit selon les méthodes usuelles connues de l'homme du métier, par exemple à partir de produits commerciaux.

Il est entendu pour l'homme du métier que, pour la mise en oeuvre des procédés selon l'invention décrits précédemment, il peut être nécessaire d'introduire des groupements protecteurs des fonctions amino, carboxyle et alcool afin d'éviter des réactions secondaires.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyles, trityle, benzyle, tert-butoxycarbonyle, BOC, benzyloxycarbonyle, phtalimido ou d'autres radicaux connus dans la chimie des peptides,

Les fonctions acide peuvent être protégées par exemple sous forme d'esters formés avec les esters facilement clivables tels que les esters benzyliques ou ter butyliques ou des esters connus dans la chimie des peptides.

On trouvera une liste de différents groupements protecteurs utilisables dans les manuels connus de l'homme du métier et par exemple dans le brevet BF 2 499 995.

On peut noter que l'on peut soumettre, si désiré et si nécessaire, des produits intermédiaires ou des produits de formule (I) ainsi obtenus par les procédés indiqués ci-dessus, pour obtenir d'autres intermédiaires ou d'autres produits de formule (I), à une ou plusieurs réactions de transformations connues de l'homme du métier telles que par exemple :
a) une réaction d'estérification de fonction acide,
b) une réaction de saponification de fonction ester en fonction acide,
c) une réaction de réduction de la fonction carboxy libre ou estérifié en fonction alcool,
d) une réaction de transformation de fonction alcoxy en fonction hydroxyle, ou encore de fonction hydroxyle en fonction alcoxy,
e) une réaction d'élimination des groupements protecteurs que peuvent porter les fonctions réactives protégées,
f) une réaction de salification par un acide minéral ou organique ou par une base pour obtenir le sel correspondant,
g) une réaction de dédoublement des formes racémiques en produits dédoublés,
lesdits produits de formule (I) ainsi obtenus étant sous toutes les formes isomères possibles racémiques, énantiomères et diastéréoisomères.

Les réactions a) à g) peuvent être réalisées dans les conditions usuelles connues de l'homme du métier telles que, par exemple, celles indiquées ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
   La réaction de saponification peut être réalisée selon les méthodes usuelles connues de l'homme du métier, telles que par exemple dans un solvant tel que le méthanol ou l'éthanol, le dioxanne ou le diméthoxyéthane, en présence de soude ou de potasse.
c) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxanne ou l'éther éthylique.
   Les éventuelles fonctions carboxy libre des produits décrits ci-dessus peuvent être, si désiré, réduites en fonction alcool notamment par de l'hydrure de bore.
d) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.
e) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
f) Les produits décrits ci-dessus peuvent, si désiré, faire l'objet de réactions de salification par exemple par un acide minéral ou organique ou par une base minérale ou organique selon les méthodes usuelles connues de l'homme du métier : une telle réaction de salification peut être réalisée par exemple en présence d'acide chlorhydrique par exemple ou encore d'acide tartrique, citrique ou méthane sulfonique, dans un alcool tel que par exemple l'éthanol ou le méthanol.
g) Les éventuelles formes optiquement actives des produits décrits ci-dessus peuvent être préparées par dédoublement des racémiques selon les méthodes usuelles connues de l'homme du métier.

Les produits de formule (I) tels que définis ci-dessus ainsi que leurs sels d'addition avec les acides présentent d'intéressantes propriétés pharmacologiques notamment en raison de leurs propriétés inhibitrices de kinases ainsi qu'il est indiqué ci-dessus.

Les produits de la présente invention sont notamment utiles pour la thérapie de tumeurs.

Les produits de l'invention peuvent également ainsi augmenter les effets thérapeutiques d'agents anti-tumoraux couramment utilisés.

Ces propriétés justifient leur application en thérapeutique et l'invention a particulièrement pour objet à titre de médicaments, les produits de formule (I) telle que définie ci-dessus, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

L'invention a tout particulièrement pour objet, à titre de médicaments, les produits répondant aux formules suivantes :
- (2S)-1-[2-(4-méthoxyphényl)éthyl]-2-méthyl-7-(morpholin-4-yl)-2-(trifluorométhyl)-2,3-dihydroimidazo[1,2-a]pyrimidin-5(1H)-one
- 1-[2-(4-méthoxyphényl)éthyl]-2-méthyl-7-(morpholin-4-yl)-2-(trifluorométhyl)-2,3-dihydroimidazo[1,2-a]pyrimidin-5(1H)-one
- (2S)-1-benzyl-2-méthyl-7-(morpholin-4-yl)-2-(trifluorométhyl)-2,3-dihydroimidazo[1,2-a]pyrimidin-5(1H)-one
- (2S)-2-méthyl-7-(morpholin-4-yl)-1-(2-phényléthyl)-2-(trifluorométhyl)-2,3-dihydroimidazo[1,2-a]pyrimidin-5(1H)-one
- (2S)-2-méthyl-7-(morpholin-4-yl)-1-(3-phénylpropyl)-2-(trifluorométhyl)-2,3-dihydroimidazo[1,2-a]pyrimidin-5(1H)-one
- (2S)-2-méthyl-7-(morpholin-4-yl)-1-(2-phénoxyéthyl)-2-(trifluorométhyl)-2,3-dihydroimidazo[1,2-a]pyrimidin-5(1H)-one
- (2S)-2-méthyl-7-(morpholin-4-yl)-1-[2-(phénylsulfanyl)éthyl]-2-(trifluorométhyl)-2,3-dihydroimidazo[1,2-a]pyrimidin-5(1H)-one
- (2S)-2-méthyl-7-(morpholin-4-yl)-1-[(2R)-2-phénylpropyl]-2-(trifluorométhyl)-2,3-dihydroimidazo[1,2-a]pyrimidin-5(1H)-one
- (2S)-2-méthyl-7-(morpholin-4-yl)-1-[(2S)-2-phénylpropyl]-2-(trifluorométhyl)-2,3-dihydroimidazo[1,2-a]pyrimidin-5(1H)-one
- (2S)-1-[(2S)-2-hydroxy-2-phényléthyl]-2-méthyl-7-(morpholin-4-yl)-2-(trifluorométhyl)-2,3-dihydroimidazo[1,2-a]pyrimidin-5(1H)-one
- (2S)-1-[(2R)-2-hydroxy-2-phényléthyl]-2-méthyl-7-(morpholin-4-yl)-2-(trifluorométhyl)-2,3-dihydroimidazo[1,2-a]pyrimidin-5(1H)-one
-(2S)-2-méthyl-7-(morpholin-4-yl)-1-[(2S)-1-phénylpropan-2-yl]-2-(trifluorométhyl)-2,3-dihydroimidazo[1,2-a]pyrimidin-5(1H)-one
- (2S)-2-méthyl-7-(morpholin-4-yl)-1-[(1S)-1-phénylpropyl]-2-(trifluorométhyl)-2,3-dihydroimidazo[1,2-a]pyrimidin-5(1H)-one
- (2S)-2-méthyl-7-(morpholin-4-yl)-1-[(1R)-1-phénylpropyl]-2-(trifluorométhyl)-2,3-dihydroimidazo[1,2-a]pyrimidin-5(1H)-one
-(2S)-2-méthyl-7-(morpholin-4-yl)-1-{2-[4-(morpholin-4-yl)phényl]éthyl}-2-(trifluorométhyl)-2,3-dihydroimidazo[1,2-a]pyrimidin-5(1H)-one
- (2S)-2-méthyl-7-(morpholin-4-yl)-1-(1-phényléthyl)-2-(trifluorométhyl)-2,3-dihydroimidazo[1,2-a]pyrimidin-5(1H)-one
- 1-[2-(4-méthoxyphényl)éthyl]-2,2-diméthyl-7-(morpholin-4-yl)-2,3-dihydroimidazo[1,2-a]pyrimidin-5(1H)-one
- (2S)-6-fluoro-1-[2-(4-méthoxyphényl)éthyl]-2-méthyl-7-(morpholin-4-yl)-2-(trifluorométhyl)-2,3-dihydroimidazo[1,2-a]pyrimidin-5(1H)-one
- (2S)-1-benzyl-6-fluoro-2-méthyl-7-(morpholin-4-yl)-2-(trifluorométhyl)-2,3-dihydroimidazo[1,2-a]pyrimidin-5(1H)-one
- (2S)-1-[(5-chloro-1-benzothiophén-3-yl)méthyl]-2-méthyl-7-(morpholin-4-yl)-2-(trifluorométhyl)-2,3-dihydroimidazo[1,2-a]pyrimidin-5(1H)-one
-(2S)-2-méthyl-7-(morpholin-4-yl)-1-(phénylcarbonyl)-2-(trifluorométhyl)-2,3-dihydroimidazo[1,2-a]pyrimidin-5(1H)-one
- (2S)-1-[(1R ou 1S)-1-(3-fluorophényl)éthyl]-2-méthyl-7-(morpholin-4-yl)-2-(trifluorométhyl)-2,3-dihydroimidazo[1,2-a]pyrimidin-5(1H)-one
-trifluoro acétate de (2S)-1-{[4-chloro-2-(trifluorométhyl)quinoléin-6-yl]méthyl}-2-méthyl-7-(morpholin-4-yl)-2-(trifluorométhyl)-2,3-dihydroimidazo[1,2-a]pyrimidin-5(1H)-one
-(2S)-1-(3-bromo-4-fluorobenzyl)-2-méthyl-7-(morpholin-4-yl)-2-(trifluorométhyl)-2,3-dihydroimidazo[1,2-a]pyrimidin-5(1H)-one
- (2S)-1-(2,3-difluorobenzyl)-2-méthyl-7-(morpholin-4-yl)-2-(trifluorométhyl)-2,3-dihydroimidazo[1,2-a]pyrimidin-5(1H)-one
-(2S)-1-[2-(3-méthoxyphényl)éthyl]-2-méthyl-7-(morpholin-4-yl)-2-(trifluorométhyl)-2,3-dihydroimidazo[1,2-a]pyrimidin-5(1H)-one
- (2S)-1-[2-(2-chlorophényl)éthyl]-2-méthyl-7-(morpholin-4-yl)-2-(trifluorométhyl)-2,3-dihydroimidazo[1,2-a]pyrimidin-5(1H)-one
- (2S)-1-[2-(4-chlorophényl)éthyl]-2-méthyl-7-(morpholin-4-yl)-2-(trifluorométhyl)-2,3-dihydroimidazo[1,2-a]pyrimidin-5(1H)-one
- (2S)-1-[2-(3-chlorophényl)éthyl]-2-méthyl-7-(morpholin-4-yl)-2-(trifluorométhyl)-2,3-dihydroimidazo[1,2-a]pyrimidin-5(1H)-one
- (2S)-1-(1,3-benzoxazol-2-ylméthyl)-2-méthyl-7-(morpholin-4-yl)-2-(trifluorométhyl)-2,3-dihydroimidazo[1,2-a]pyrimidin-5(1H)-one
- (2S)-2-méthyl-7-(morpholin-4-yl)-1-[(1R ou 1S)-1-phényléthyl]-2-(trifluorométhyl)-2,3-dihydroimidazo[1,2-a]pyrimidin-5(1H)-one
- (2S)-2-méthyl-7-(morpholin-4-yl)-1-[(1R ou 1S)-1-phényléthyl]-2-(trifluorométhyl)-2,3-dihydroimidazo[1,2-a]pyrimidin-5(1H)-one
- (2S)-1-(1H-indol-3-ylméthyl)-2-méthyl-7-(morpholin-4-yl)-2-(trifluorométhyl)-2,3-dihydroimidazo[1,2-a]pyrimidin-5(1H)-one
- (2S)-1-[(2-chlorophényl)carbonyl]-2-méthyl-7-(morpholin-4-yl)-2-(trifluorométhyl)-2,3-dihydroimidazo[1,2-a]pyrimidin-5(1H)-one
- (2S)-2-méthyl-1-[(2-méthylphényl)carbonyl]-7-(morpholin-4-yl)-2-(trifluorométhyl)-2,3-dihydroimidazo[1,2-a]pyrimidin-5(1H)-one
- (2S)-1-[(1 R ou 1S)-1-(2-fluorophényl)éthyl]-2-méthyl-7-(morpholin-4-yl)-2-(trifluorométhyl)-2,3-dihydroimidazo[1,2-a]pyrimidin-5(1H)-one
- (2S)-1-[(1 R ou 1S)-1-(2-fluorophényl)éthyl]-2-méthyl-7-(morpholin-4-yl)-2-(trifluorométhyl)-2,3-dihydroimidazo[1,2-a]pyrimidin-5(1H)-one
- (S)-1-[2-(2-Fluoro-4,5-dimethoxy-phenyl)-2-hydroxy-ethyl]-2-methyl-7-morpholin-4-yl-2-trifluoromethyl-2,3-dihydro-1H-imidazo[1,2-a]pyrimidin-5-one
- (S)-1-[(S)-2-Hydroxy-2-(2-methoxy-phenyl)-ethyl]-2-methyl-7-morpholin-4-yl-2-trifluoromethyl-2,3-dihydro-1H-imidazo[1,2-a]pyrimidin-5-one
(S)-1-[(S)-2-(4-Chloro-2-methoxy-phenyl)-2-hydroxy-ethyl]-2-methyl-7-morpholin-4-yl-2-trifluoromethyl-2,3-dihydro-1H-imidazo[1,2-a]pyrimidin-5-one
(S)-1-[(S)-2-(4-Fluoro-2-methoxy-phenyl)-2-hydroxy-ethyl]-2-methyl-7-morpholin-4-yl-2-trifluoromethyl-2,3-dihydro-1H-imidazo[1,2-a]pyrimidin-5-one
(S)-1-[(S)-2-(2-Chloro-4-methoxy-phenyl)-2-hydroxy-ethyl]-2-methyl-7-morpholin-4-yl-2-trifluoromethyl-2,3-dihydro-1H-imidazo[1,2-a]pyrimidin-5-one
ainsi que les sels d'addition avec les acides minéraux et organiques ou avec les bases minérales et organiques pharmaceutiquement acceptables desdits produits de formule (I).

L'invention concerne aussi des compositions pharmaceutiques contenant à titre de principe actif l'un au moins des produits de formule (I) tels que définis ci-dessus ou un sel pharmaceutiquement acceptable de ce produit ou un prodrug de ce produit et, le cas échéant, un support pharmaceutiquement acceptable.

L'invention s'étend ainsi aux compositions pharmaceutiques contenant à titre de principe actif l'un au moins des médicaments tels que définis ci-dessus.

De telles compositions pharmaceutiques de la présente invention peuvent également, le cas échéant, renfermer des principes actifs d'autres médicaments antimitotiques tels que notamment ceux à base de taxol, cis-platine, les agents intercalants de l'ADN et autres.

Ces compositions pharmaceutiques peuvent être administrées par voie buccale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les pilules, les tablettes, les gélules, les gouttes, les granulés, les préparations injectables, les pommades, les crèmes ou les gels ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 0,05 à 5 g par jour chez l'adulte, ou de préférence de 0,1 à 2 g par jour.

La présente invention a également pour objet l'utilisation de produits de formule (I) tels que définis ci-dessus pour la préparation d'un médicament destiné au traitement ou à la prévention d'une maladie caractérisée par le dérèglement de l'activité d'une protéine ou d'une lipide kinase.

La présente invention a notamment pour objet l'utilisation d'un produit de formule (I) tel que défini ci-dessus pour la préparation d'un médicament destiné à la prévention ou au traitement de différentes maladies comme les maladies cardiovasculaires incluant notamment la thrombose.

Un tel médicament peut notamment être destiné au traitement ou à la prévention d'une maladie chez un mammifère.

La présente invention a notamment pour objet l'utilisation d'un produit de formule (I) tel que défini ci-dessus pour la préparation d'un médicament destiné à la prévention ou au traitement de maladies liées à une prolifération non contrôlée.

La présente invention a ainsi tout particulièrement pour objet l'utilisation d'un produit de formule (I) tel que défini ci-dessus pour la préparation d'un médicament destiné au traitement ou à la prévention de maladies en oncologie et notamment destiné au traitement de cancers.

Parmi ces cancers, on s'intéresse au traitement de tumeurs solides ou liquides, au traitement de cancers résistant à des agents cytotoxiques

Les produits de la présente invention cités peuvent notamment être utilisés pour le traitement de tumeurs primaires et/ou de métastases en particulier dans les cancers gastriques, hépatiques, rénaux, ovariens, du colon, de la prostate, de l'endomètre, du poumon (NSCLC et SCLC), les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas, dans les hamartomes. Sont concernées notamment les maladies présentant des anomalies génétiques aboutissant à l'activation de la voie PI3K/AKT/mTOR et/ou à l'activation de la voie MAP Kinase.

La présente invention a aussi pour objet l'utilisation des produits de formule (I) telle que définie ci-dessus pour la préparation de médicaments destinés à la chimiothérapie de cancers.
La présente invention a ainsi pour objet les produits de formule (I) tels que définis ci-dessus pour leur utilisation pour le traitement de cancers.
La présente invention a pour objet les produits de formule (I) tels que définis ci-dessus pour leur utilisation pour le traitement de tumeurs solides ou liquides.
La présente invention a donc pour objet les produits de formule (I) tels que définis ci-dessus pour leur utilisation pour le traitement de cancers résistant à des agents cytotoxiques.
La présente invention a donc pour objet les produits de formule (I) tels que définis ci-dessus pour leur utilisation pour le traitement de tumeurs primaires et/ou de métastases en particulier dans les cancers gastriques, hépatiques, rénaux, ovariens, du colon, de la prostate, de l'endomètre, du poumon (NSCLC et SCLC), les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas, dans les hamartomes.
La présente invention a donc pour objet les produits de formule (I) tels que définis ci-dessus pour leur utilisation pour la chimiothérapie de cancers.
La présente invention a donc pour objet les produits de formule (I) tels que définis ci-dessus pour leur utilisation pour la chimiothérapie de cancers, seuls ou en en association.

De tels médicaments destinés à la chimiothérapie de cancers peuvent être utilisés seuls ou en en association.

Les produits de la présente demande peuvent notamment être administrés seuls ou en association avec de la chimiothérapie ou de la radiothérapie ou encore en association par exemple avec d'autres agents thérapeutiques.

De tels agents thérapeutiques peuvent être des agents anti-tumoraux couramment utilisés.

On peut notamment attendre un bénéfice thérapeutique en administrant les produits de la présente demande en combinaisons avec des thérapies ciblées variées. Ces thérapies ciblées sont notamment les suivantes : i) les thérapies inhibant la voie de signalisation MAP Kinase comme les thérapies inhibant RAS, RAF, MEK ou ERK ; ii) les thérapies ciblées inhibant les kinases ou pseudo-kinases de la voie PI3K/AKT/mTOR comme EGFR, HER2, HER3, ALK, MET, PI3K, PDK1, AKT, mTOR et S6K.

La présente invention a notamment pour objet l'utilisation d'un produit de formule (I) tel que défini ci-dessus pour la préparation d'un médicament destiné à la prévention ou au traitement de maladies lysosomales telles que la glycogénose de type II ou maladie de Pompe. De tels médicaments destinés au traitement des maladies lysosomales peuvent être utilisés seuls ou en en association par exemple avec d'autres agents thérapeutiques.
La présente invention a ainsi pour objet les produits de formule (I) tels que définis ci-dessus pour la prévention ou le traitement de maladies lysosomales telles que la glycogénose de type II ou maladie de Pompe.
La présente invention a ainsi pour objet l'utilisation des produits de formule (I) tel que défini ci-dessus pour la préparation d'un médicament destiné à la prévention ou au traitement de maladies lysosomales telles que la glycogénose de type II ou maladie de Pompe.
La présente invention a ainsi pour objet l'utilisation telle que définie ci-dessus dans laquelle lesdits produits de formule (I) sont seuls ou en association.
La présente invention a également pour objet l'utilisation d'un produit de formule (I) tel que défini ci-dessus pour la préparation d'un médicament destiné au traitement de maladies parasitaires telles que la malaria, la maladie du sommeil, la maladie de Chagas, les leishmanioses. De tels médicaments destinés au traitement des infections parasitaires peuvent être utilisés seuls ou en en association par exemple avec d'autres agents thérapeutiques.
La présente invention a ainsi pour objet les produits de formule (I) tels que définis ci-dessus pour le traitement de maladies parasitaires telles que la malaria, la maladie du sommeil, la maladie de Chagas, les leishmanioses.
La présente invention a ainsi pour objet l'utilisation des produits de formule (I) tels que définis ci-dessus pour la préparation d'un médicament pour le traitement de maladies parasitaires telles que la malaria, la maladie du sommeil, la maladie de Chagas, les leishmanioses.
La présente invention a encore pour objet à titre de produits industriels nouveaux, les intermédiaires de synthèse de formules D, E, F et J tels que définis ci-dessus et rappelés ci-après : 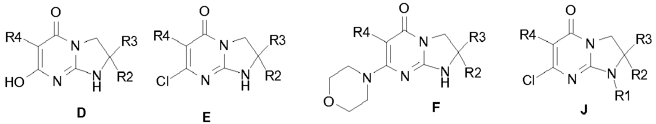 dans lesquels R1, R2, R3 et R4 ont les définitions indiquées à l'une quelconque des revendications 1 à 2.

### Partie expérimentale

La nomenclature des composés de cette présente invention a été effectuée avec le logiciel ACDLABS version 10.0.

Le four à microondes utilisé est un appareil Biotage, Initiator™ 2.0, 400W max, 2450 MHz.

Les spectres de RMN ¹H à 400 MHz et ¹H à 500 MHz ont été effectués sur spectromètre BRUKER AVANCE DRX-400 ou BRUKER AVANCE DPX-500 avec les déplacements chimiques (δ en ppm) dans le solvant diméthylsulfoxide-d₆ (DMSO-d₆) référencé à 2,5 ppm à la température de 303K.

Les spectres de masse (SM) ont été obtenus soit par la méthode A, soit par la méthode B, soit par la méthode E :
Méthode A :
   Appareil WATERS UPLC-SQD ; Ionisation : électrospray en mode positif et/ou négatif (ES+/-) ; Conditions chromatographiques : Colonne : ACQUITY BEH C18 1,7 µm - 2,1 x 50 mm ; Solvants : A : H₂O (0,1 % acide formique) B : CH₃CN (0,1 % acide formique) ; Température de colonne : 50 °C ; Débit : 1 ml/min ; Gradient (2 min) : de 5 à 50 % de B en 0,8 min ; 1,2 min : 100 % de B ; 1,85 min : 100 % de B ; 1,95 : 5 % de B ; Temps de rétention = Tr (min).
Méthode B :
   Appareil WATERS ZQ ; Ionisation : électrospray en mode positif et/ou négatif (ES+/-) ; Conditions chromatographiques : Colonne : XBridge C₁₈ 2,5 µm - 3 x 50 mm ; Solvants : A : H₂O (0,1 % acide formique) B : CH₃CN (0,1 % acide formique) ; Température de colonne : 70°C ; Débit : 0,9 ml/min ; Gradient (7 min) : de 5 à 100 % de B en 5,3 min ; 5,5 min : 100 % de B ; 6,3 min : 5 % de B ; Temps de rétention = Tr (min).
Méthode E :
   Appareil WATERS UPLC-SQD ; Ionisation : électrospray en mode positif et/ou négatif (ES+/-) ; Conditions chromatographiques : Colonne : Ascentis express C18 2,7 µm - 2,1 x 50 mm ; Solvants : A : H2O (0,02 % acide trifluorocétique) B: CH3CN (0,014 % acide trifluoroacétique) ; Température de colonne : 55 °C ; Débit : 1 ml/min ; Gradient: T0min 2%B, T1min 98%B, T1.3min 98%B, T1.33min 2%B, T1.5 min autre injection; Temps de rétention = Tr (min).

Les pouvoirs rotatoires (PR) ont été mesurés sur un polarimètre modèle 341 de chez Perkin Elmer. Longueur d'onde: raie α du sodium (589 nanomètres).

Purifications par HPLC / MS préparative :
• Méthode C
Colonne de phase inverse C18 SunFire (Waters) 30 x 100, 5 µ. Gradient d'acétonitrile (+ 0.07 % TFA) dans l'eau (+ 0.07 % TFA)

| | |
|---|---|
| T0 : | 20 % acétonitrile (+ 0.07 % TFA) |
| T1 : | 20 % acétonitrile (+ 0.07 % TFA) |
| T11.5 : | 95 % acétonitrile (+ 0.07 % TFA) |
| T15 : | 95 % acétonitrile (+ 0.07 % TFA) |
| T15.5: | 20% acétonitrile (+ 0.07 % TFA) |
| Débit : | 30 ml/min |
| Masse : | 130_800 UMA= ; ESP+, ESP |

• Méthode D
Colonne de phase inverse C18 SunFire (Waters) 30 x 100, 5 µ. Gradient d'acétonitrile (+ 0.07 % TFA) dans l'eau (+ 0.07 % TFA)

| | |
|---|---|
| T0 : | 15 % acétonitrile (+ 0.07 % TFA) |
| T1 : | 15 % acétonitrile (+ 0.07 % TFA) |
| T11 : | 90 % acétonitrile (+ 0.07 % TFA) |
| T11.5 : | 95 % acétonitrile (+ 0.07 % TFA) |
| T14 : | 95 % acétonitrile (+ 0.07 % TFA) |
| T15 : | 10% acétonitrile (+ 0.07 % TFA) |
| Débit : | 30 ml/min |
| Masse : | 130_800 UMA= ; ESP+, ESP |

### Exemple 1 : (S)-1-[2-(4-méthoxy-phényl)-éthyl]-2-méthyl-7-morpholin-4-yl-2-trifluorométhyl-2,3-dihydro-1H-imidazo[1,2-a]pyrimidin-5-one

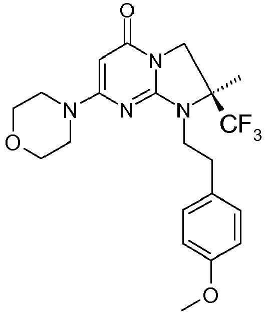

### Stade k : (S)-1-[2-(4-méthoxy-phényl)-éthyl]-2-méthyl-7-morpholin-4-yl-2-trifluorométhyl-2,3-dihydro-1H-imidazo[1,2-a]pyrimidin-5-one

A une solution de 60 mg de (S)-2-méthyl-7-morpholin-4-yl-2-trifluorométhyl-2,3-dihydro-1H-imidazo[1,2-a]pyrimidin-5-one dans 3 mL de N,N-diméthylformamide anhydre sont ajoutés, à température ambiante, sous atmosphère d'argon, 20 mg d'hydrure de sodium. Le mélange réactionnel obtenu est alors chauffé à 60°C. On ajoute ensuite 0.04 mL de bromure de 4-méthoxy phénéthyle. Après une heure de chauffage et après contrôle par CCM (CH2Cl2/MeOH : 95/05), la réaction est partielle. On additionne alors 10 mg d'hydrure de sodium et 0.04 mL de bromure de 4-méthoxy phénéthyle et le chauffage est maintenu à 60°C. Après deux heures supplémentaires de chauffage et après contrôle par CCM (CH2Cl2/MeOH : 95/05), la réaction est terminée.

Après refroidissement, on ajoute au mélange obtenu 10 mL d'eau froide et 20 mL d'acétate d'éthyle. La phase organique est alors séparée puis séchée sur sulfate de magnésium, filtrée et concentrée sous pression réduite. Le résidu obtenu est purifié par chromatographie sur silice (éluant: CH2Cl2/MeOH: 98/02) pour donner 54 mg de (S)-1-[2-(4-méthoxy-phényl)-éthyl]-2-méthyl-7-morpholin-4-yl-2-trifluorométhyl-2,3-dihydro-1H-imidazo[1,2-a]pyrimidin-5-one, sous forme d'une meringue blanc cassé, dont les caractéristiques sont les suivantes :
Spectre RM N 1 H
1.52 (s, 3 H) ; 2.79 (m, 1 H) ; 2.95 (m, 1 H) ; 3.30 à 3.60 (m, 6 H) ; 3.65 (t, J=4.9 Hz, 4 H) ; 3.72 (s, 3 H) ; 3.84 (d, J=12.6 Hz, 1 H) ; 4.11 (d, J=12.6 Hz, 1 H) ;4.88 (s, 1 H) ; 6.87 (d, J=8.6 Hz, 2 H) ; 7.14 (d, J=8.6 Hz, 2 H)
Spectrométrie de Masse : méthode B
Temps de rétention Tr (min) = 4,07
[M+H]+ : m/z 439
Pouvoir rotatoire : PR= +89 ; C=0.710mg/0.5ML DMSO.

### Stade j :(S)-2-méthyl-7-morpholin-4-yl-2-trifluorométhyl-2,3-dihydro-1H-imidazo[1,2-a]pyrimidin-5-one

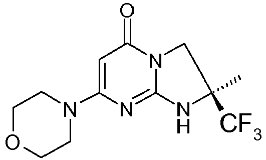

Un mélange de 2.2 g de (S)-7-chloro-2-méthyl-2-trifluorométhyl-2,3-dihydro-1H-imidazo[1,2-a]pyrimidin-5-one dans 60 mL de morpholine est chauffé à 120°C. Après une heure de chauffage et après contrôle par LC/MS, la réaction est terminée.

Après refroidissement, le mélange réactionnel est concentré sous pression réduite. Sur le résidu obtenu, on ajoute 30 mL d'eau froide et 150 mL d'acétate d'éthyle. La phase organique est alors séparée, séchée sur sulfate de magnésium, filtrée puis concentrée sous pression réduite pour donner 2.6 g de (S)-2-méthyl-7-morpholin-4-yl-2-trifluorométhyl-2,3-dihydro-1H-imidazo[1,2-a]pyrimidin-5-one dont les caractéristiques sont les suivantes:
Spectrométrie de Masse : méthode B
Temps de rétention Tr (min) = 2,53
[M+H]+ : m/z 305 ; [M-H]- : m/z 303
Pouvoir rotatoire : PR= -9.0+/-0.6 ; C=1.996710mg/0.5ML DMSO.

### Stade i : (S)-7-chloro-2-méthyl-2-trifluorométhyl-2,3-dihydro-1H-imidazo[1,2-a]pyrimidin-5-one

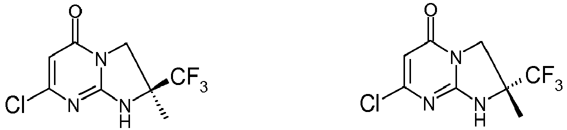

La séparation des deux énantiomères de la 7-chloro-2-méthyl-2-trifluorométhyl-2,3-dihydro-1H-imidazo[1,2-a]pyrimidin-5-one (9.22 g) a été réalisée par chromatographie chirale :
phase stationnaire : Chiralpak AD ; phase mobile : EtOH (05%) / MeOH (05%) / Heptane (90%).

L'énantiomère dextrogyre est concentré pour obtenir 4.56 g de la (R)-7-chloro-2-méthyl-2-trifluorométhyl-2,3-dihydro-1H-imidazo[1,2-a]pyrimidin-5-one.

L'énantiomère lévogyre est concentré pour obtenir 4.47 g de la (S)-7-chloro-2-méthyl-2-trifluorométhyl-2,3-dihydro-1H-imidazo[1,2-a]pyrimidin-5-one, sous forme d'une poudre blanche, dont les caractéristiques sont les suivantes :
Pouvoir rotatoire : PR= -70.9+/-1.1; C=2.623mg/0.5ML DMSO.
Spectrométrie de Masse : méthode A
Temps de rétention Tr (min) = 0,51
[M+H]+ : m/z 254 ; [M-H]- : m/z 252

### Stade h : (R,S)-7-chloro-2-méthyl-2-trifluorométhyl-2,3-dihydro-1H-imidazo[1,2-a]pyrimidin-5-one

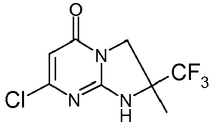

A une suspension de 20 g de (R,S)-7-hydroxy-2-méthyl-2-trifluorométhyl-2,3-dihydro-1H-imidazo[1,2-a]pyrimidin-5-one dans 400 mL de 1,2-dichloroéthane sont ajoutés, à température ambiante et sous atmosphère d'argon, 20 mL d'oxychlorure de phosphore. Le mélange réactionnel est alors chauffé à 65°C. Après deux heures d'agitation et après contrôle par LC/MS, la réaction est terminée.

Après refroidissement, le solide jaune pâle formé est filtré pour donner 4.05 g d'un premier lot de produit chloré S1. Le filtrat résultant est évaporé à sec sous pression réduite et le résidu obtenu repris par 20 mL d'eau froide et 200 mL d'acétate d'éthyle. Sur le mélange obtenu, on additionne de la soude 32% jusqu'à pH = 5-6. La phase organique est alors séparée, séchée sur sulfate de magnésium, filtrée puis concentrée sous pression réduite pour donner une meringue jaune. Celle-ci est purifiée par chromatographie sur silice (éluant : CH2Cl2/MeOH : 98/02) pour donner 12.24 g d'un solide S2.

Les deux lots S1 et S2 qui sont identiques en CCM sont réunis pour donner 16.29 g de (R,S)-7-chloro-2-méthyl-2-trifluorométhyl-2,3-dihydro-1H-imidazo[1,2-a]pyrimidin-5-one dont les caractéristiques sont les suivantes :
Spectrométrie de Masse : méthode A
Temps de rétention Tr (min) = 0,51
[M+H]+ : m/z 254 ; [M-H]- : m/z 252

### Stade g : (R,S)-7-hydroxy-2-méthyl-2-trifluorométhyl-2,3-dihydro-1H-imidazo[1,2-a]pyrimidin-5-one

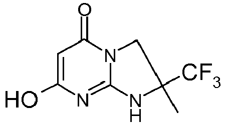

Sur un mélange de 20.4 g de malonate de diéthyle dans 320 mL de méthanol, sont ajoutés 31.6 g de bromhydrate de 4-méthyl-4-trifluorométhyl-imidazolidin-2-ylidène amine et 13.7 g de méthylate de sodium. Le mélange résultant est porté au reflux pendant 18 heures.

Après refroidissement, le mélange réactionnel est concentré à sec sous pression réduite. Sur le résidu obtenu, on ajoute 100 ml d'eau froide. A la suspension épaisse résultante, on additionne de l'acide chlorhydrique 25% jusqu'à pH=5. La suspension obtenue est agitée dans un bain de glace pendant deux heures puis filtrée sur verre frité. L'insoluble est rincé avec de l'eau (2 fois 15 ml) puis séché pour donner 30 g de (R,S)-7-hydroxy-2-méthyl-2-trifluorométhyl-2,3-dihydro-1H-imidazo[1,2-a]pyrimidin-5-one, sous forme d'un solide blanc, dont les caractéristiques sont les suivantes :
Spectrométrie de Masse : méthode B
Temps de rétention Tr (min) = 1,29
[M+H]+ : m/z 236 ; [M-H]- : m/z 234

### Stade f : Bromhydrate de (R,S)-4-méthyl-4-trifluorométhyl-imidazolidin-2-ylidène amine

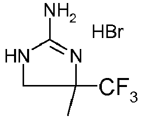

Sur une solution refroidie à 5°C de 5 g de (R,S)-3,3,3-trifluoro-2-méthyl-propane-1,2-diamine dans 30 mL d'eau, sont additionnés, par petit morceaux, 3.72 g de bromure de cyanogène tout en maintenant la température entre 5 et 10°C. A la fin de l'addition, le mélange réactionnel est laissé à 5°C pendant 30 minutes. Le bain de glace est ensuite retiré et le mélange réactionnel agité à température ambiante pendant 3 heures.

Le mélange réactionnel est alors concentré sous pression réduite. Le résidu obtenu est repris 2 fois avec 200 ml d'EtOH, puis 2 fois avec 200 ml de toluène, avec à chaque fois évaporation à sec. Le solide résultant est trituré avec de l'éther éthylique puis filtré pour donner 7 g du bromhydrate de (R,S)-4-méthyl-4-trifluorométhyl-imidazolidin-2-ylidène amine, sous forme d'un solide blanc, dont les caractéristiques sont les suivantes :
Spectrométrie de Masse : méthode A
[M+H]+: m/z = 168.

### Stade e : (R,S)-3,3,3-trifluoro-2-méthyl-propane-1,2-diamine

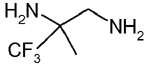

Dans un ballon, on introduit 27 g du chlorhydrate de la (R,S)-3,3,3-trifluoro-2-méthyl-propane-1,2-diamine, 15 mL d'eau et 400 mL d'éther éthylique. Sous agitation magnétique, on additionne, goutte à goutte, sur le mélange obtenu, 25 mL de soude 32% jusqu'à pH=12. La phase aqueuse est alors décantée puis extraite par 4 fois 200 ml d'éther éthylique.

Les phases organiques sont réunies, séchées sur sulfate de magnésium, filtrées puis concentrées sous pression réduite (300 mbar / température du bain = 25°C) pour donner 21.9 g de (R,S)-3,3,3-trifluoro-2-méthyl-propane-1,2-diamine, sous forme d'une huile jaune clair, dont les caractéristiques sont les suivantes :
Spectrométrie de Masse : méthode A
[M+H]+: m/z = 143.

### Stade d : Dichlorhydrate de la (R,S)-3,3,3-trifluoro-2-méthyl-propane-1,2-diamine, 2HCl

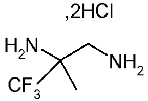

Dans un autoclave, on introduit 8 g d'hydroxyde de palladium à 20%, 58 g de la (R,S)-N-benzyl-3,3,3-trifluoro-2-méthyl-propane-1,2-diamine dans 200 ml de méthanol et 183 ml d'acide chlorhydrique 3N. Le mélange résultant est hydrogéné à une pression d'hydrogène de 5 bars, à 22 °C, pendant 48 heures.

Le mélange résultant est alors filtré puis le filtrat est concentré sous pression réduite. Le résidu obtenu est repris 2 fois avec 300 ml d'EtOH, puis 2 fois avec 300 ml de toluène, avec évaporation à sec à chaque fois. On obtient ainsi 50 g du dichlorhydrate de la (R,S)-3,3,3-trifluoro-2-méthyl-propane-1,2-diamine, sous forme d'une meringue blanc cassé, dont les caractéristiques sont les suivantes :
Spectrométrie de Masse : méthode A
[M+H]+: m/z = 143

### Stade c : (R,S)-N-benzyl-3,3,3-trifluoro-2-méthyl-propane-1,2-diamine

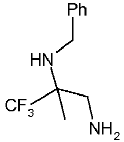

Dans un tricol sous argon, sont additionnés par petites portions, sur une solution de 23 g de (R,S)-N-benzylamino-3,3,3-trifluoro-2-méthyl-propionitrile dans 1000 mL d'éther éthylique anhydre refroidie à 4 °C, 15.5 g d'hydrure de lithium aluminium Un fort dégagement gazeux avec élévation de la température à 8°C est observé.

A la fin de l'addition, on laisse la température remonter à l'ambiante et on laisse le mélange sous agitation à température ambiante, pendant 18h. Le mélange réactionnel résultant est refroidi à 4°C avant ajout de 20 ml d'eau, goutte à goutte et très lentement. On observe un fort dégagement gazeux avec élévation de la température jusqu'à 12°C.

Toujours à 4°C, on additionne, goutte à goutte et très lentement, sur le mélange obtenu, 20 ml de potasse à 15% puis, toujours goutte à goutte et très lentement, 40 ml d'eau.

Le précipité blanc résultant est filtré et le filtrat séché sur sulfate de magnésium puis concentré sous pression réduite pour donner 22.5 g de la (R,S)-N-benzyl-3,3,3-trifluoro-2-méthyl-propane-1,2-diamine, sous forme d'une huile incolore, dont les caractéristiques sont les suivantes :
Spectrométrie de Masse : méthode A
[M+H]+: m/z = 233

### Stade b : 2-benzylamino-3,3,3-trifluoro-2-méthyl-propionitrile

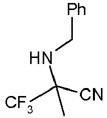

Dans un tricol sous argon, on additionne, goutte à goutte, à une solution de 80 g de (R,S)-N-benzyl-[2,2,2-trifluoro-1-méthyl-éth-(E)-ylidène]-amine dans 800 mL de dichlorométhane, refroidie à -70 °C, 59.17 g de cyanure de triméthysilyle puis, goutte à goutte, 84.65 g de trifluoro éthérate de bore. La température monte à -63°C et la solution devient orange. Aprsè addition, le mélange réactionnel est agité à -63°C pendant 30 mn.

Le bain de carboglace est alors retiré pour laisser la température remonter à l'ambiante. Le mélange réactionnel est ensuite laissé sous agitation à température ambiante pendant une nuit.

On additionne alors sur le mélange résultant une solution saturée de bicarbonate de sodium jusqu'à pH = 8. La phase organique est alors séparée puis séchée sur sulfate de magnésium, filtrée et concentrée sous pression réduite. Le résidu obtenu est purifié par filtration sur silice (éluant : dichlorométhane /cyclohexane : 25/75) pour donner 48 g du (R,S)-2-benzylamino-3,3,3-trifluoro-2-méthyl-propionitrile, sous forme d'une huile incolore, dont les caractéristiques sont les suivantes :
Spectrométrie de Masse :
   les spectres ont été réalisés par impact électronique sur appareil WATERS GCTof (introduction directe sans LC).
   EI : [M] +. : m/z 228 ; m/z 91 (pic de base)

### Stade a : benzyl-[2,2,2-trifluoro-1-méthyl-éth-(E)-ylidène]-amine

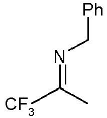

Dans un tricol, on additionne, goutte à goutte, sur une solution de 157 g de trifluoroacétone dans 600 mL de toluène, refroidie à 5°C, 100 g de benzylamine. La température monte à 25°C.

On ajoute alors en une seule fois 9.4 g de para-toluène sulfonate de pyridinium. Le mélange réactionnel résultant est agité à température ambiante pendant 30 minutes. Un réfrigérant surmonté d'un Dean-Stark est alors installé et le mélange réactionnel est chauffé au reflux pendant 4 heures, durant lesquelles on recueille 25 ml d'eau.

Après refroidissement, le solide formé est filtré et le filtrat est concentré sous pression réduite pour donner 150 g de la [2,2,2-trifluoro-1-méthyl-éth-(E)-ylidène]-amine, sous forme d'un liquide incolore, dont les caractéristiques sont les suivantes :
Spectrométrie de Masse :
   Les spectres ont été réalisés par impact électronique sur appareil WATERS GCTof (introduction directe sans LC).
   EI : [M] +. : m/z 201 ; m/z 91 (pic de base)

Alternativement, la (S)-7-chloro-2-méthyl-2-trifluorométhyl-2,3-dihydro-1H-imidazo[1,2-a]pyrimidin-5-one peut être préparée de la façon suivante :

### Stade h' : (S)- 7-Chloro-2-méthyl-2-trifluorométhyl-2,3-dihydro-1H-imidazo[1,2-a]pyrimidin-5-one

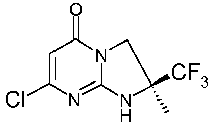

A une suspension de 5.6 g de (S)-7-hydroxy-2-méthyl-2-trifluorométhyl-2,3-dihydro-1H-imidazo[1,2-a]pyrimidin-5-one dans 100 mL de 1,2-dichloroéthane, sont ajoutés, à température ambiante et sous atmosphère d'argon, 11 mL d'oxychlorure de phosphore. Le mélange résultant est alors chauffé à 70°C. Après deux heures d'agitation et après contrôle par LC/MS, la réaction est terminée.

Après refroidissement, le mélange réactionnel est évaporé à sec sous pression réduite. Le résidu obtenu est repris par 5 mL d'eau froide et 200 mL d'acétate d'éthyle. Sur le mélange obtenu, on additionne de la soude 32% jusqu'à pH = 6. La phase organique est alors séparée puis séchée sur sulfate de magnésium, filtrée et concentrée sous pression réduite pour donner 6 g de la (S)-7-chloro-2-méthyl-2-trifluorométhyl-2,3-dihydro-1H-imidazo[1,2-a]pyrimidin-5-one dont les caractéristiques sont les suivantes :
Spectrométrie de Masse :
   Méthode A
   Temps de rétention Tr (min) = 0,51
   [M+H]+ : m/z 254 ; [M-H]- : m/z 252
   Pouvoir rotatoire : PR= -64.8 +/-1.1; C=2.2mg/0.5ML DMSO.

### Stade g' : (S)-7-Hydroxy-2-méthyl-2-trifluorométhyl-2,3-dihydro-1H-imidazo[1,2-a]pyrimidin-5-one

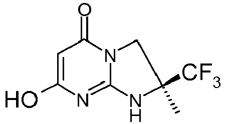

Sur un mélange de 5.4 g de malonate de diéthyle dans 50 mL de méthanol, sont ajoutés 8.4 g du bromhydrate de la (S)-4-méthyl-4-trifluorométhyl-imidazolidin-2-ylidène amine et 2.16 g de méthylate de sodium.

Le mélange résultant est porté au reflux pendant 18 heures. Après refroidissement, le mélange obtenu est concentré à sec sous pression réduite. Sur le résidu obtenu, on ajoute 20 ml d'eau froide pour obtenir une suspension épaisse sur laquelle on additionne de l'acide chlorhydrique 25% jusqu'à pH=5.

La suspension résultante est agitée dans un bain de glace pendant deux heures puis filtrée sur verre frité. L'insoluble obtenu est rincé avec de l'eau (2 fois 4 ml) puis séché pour donner 5.6 g de la (S)-7-hydroxy-2-méthyl-2-trifluorométhyl-2,3-dihydro-1H-imidazo[1,2-a]pyrimidin-5-one, sous forme d'un solide blanc, dont les caractéristiques sont les suivantes :
Spectrométrie de Masse : méthode A
Temps de rétention Tr (min) = 0,32
[M+H]+ : m/z 236 ; [M-H]- : m/z 234
Pouvoir rotatoire : PR= -5.6+/-0.6; C=1.789mg/0.5ML MeOH.

### Stade f' : Bromhydrate de la (S)-4-méthyl-4-trifluorométhyl-imidazolidin-2-ylidène amine

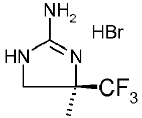

Sur une solution refroidie à 5°C de 2.3 g de la (S)-3,3,3-trifluoro-2-méthyl-propane-1,2-diamine dans 10 mL d'eau, on additionne, par petits morceaux, 1.7 g de bromure de cyanogène tout en maintenant la température entre 5 et 10°C. A la fin de l'addition, le mélange réactionnel est laissé à 5°C pendant 30 minutes. Le bain de glace est alors retiré et le mélange obtenu est agité à température ambiante pendant 3 heures.

Le mélange résultant est alors concentré sous pression réduite. Le résidu obtenu est repris 2 fois avec 100 ml d'éthanol, puis 2 fois avec 100 ml de toluène, avec à chaque fois évaporation à sec. Le solide obtenu est trituré avec de l'éther éthylique puis filtré pour donner 4.5 g du bromhydrate de la (S)-4-méthyl-4-trifluorométhyl-imidazolidin-2-ylidène amine, sous forme d'un solide blanc, dont les caractéristiques sont les suivantes :
Spectrométrie de Masse : méthode A
Temps de rétention Tr (min) = 0,14
[M+H]+ : m/z 168
Pouvoir rotatoire : PR= -5.2+/-0.3; C=4.909mg/0.5ML DMSO.

### Stade e' : (S)-3,3,3-Trifluoro-2-méthyl-propane-1,2-diamine

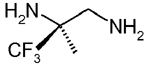

Dans un ballon, on introduit 4.8 g du chlrohydrate de la (S)-3,3,3-trifluoro-2-méthyl-propane-1,2-diamine, 2.5 mL d'eau et 100 mL d'éther éthylique. On additionne, goutte à goutte, sur le mélange résultant, 4.5 mL de soude à 32% jusqu'à pH=12. La phase aqueuse est ensuite décantée puis extraite par 4 fois 200 ml d'éther éthylique.

Les phases organiques sont réunies, séchées sur sulfate de magnésium, filtrées puis concentrées sous pression réduite (300 mbar / température du bain = 25°C) pour donner 2.3 g de la 3,3,3-trifluoro-2-méthyl-propane-1,2-diamine, sous forme d'une huile jaune clair, dont les caractéristiques sont les suivantes :
Spectrométrie de Masse : méthode B
Temps de rétention Tr (min) = 0,34
[M+H]+ : m/z 143; pic de base : m/z 126
Pouvoir rotatoire : PR= -4.3+/-0.6; C=1.778mg/0.5ML DMSO.

### Stade d' : Dichlorhydrate de la (S)-3,3,3-trifluoro-2-méthyl-propane-1,2-diamine

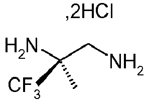

Dans un autoclave, on hydrogène à 22°C, sous une pression d'hydrogène de 5 bars et pendant 18 heures, un mélange de 7 g de (R)-2-((S)-1-aminométhyl-2,2,2-trifluoro-1-méthyl-éthylamino)-2-phényl-éthanol dans 40.5 mL de méthanol, 23.5 mL d'acide chlorhydrique 3 N et 0.94 g du Pd(OH)2/C (20% w/w). Le mélange obtenu est ensuite filtré et le filtrat évaporé à sec. L'huile obtenue est reprise par une solution d'acide chlorhydrique 3 N (50 mL). Le mélange obtenu est extrait avec de l'éther diéthylique (3 x 50 mL). La phase aqueuse est ensuite évaporée à sec, repris avec du méthanol puis évaporé de nouveau à sec. Le solide jaunâtre obtenu est séché sous vide pour conduire à 5.54 g (79%) du dichlorhydrate de la (S)-3,3,3-trifluoro-2-méthyl-propane-1,2-diamine, sous forme d'un solide blanc cassé, dont les caractéristiques sont les suivantes :
Spectre RMN 1 H (400 MHz, D2O) : 1.55 (s, 3 H), 3.40 (d, J = 14.6 Hz, 1 H), 3.51 (d, J = 14.6 Hz, 1 H).
RMN 19F (400 MHz, D2O) : - 81.08 (non calibré avec C6F6)
[ ]D : + 4.65 (C 2.2, CH3OH)

### Stade c' : (R)-2-((S)-1-Aminométhyl-2,2,2-trifluoro-1-méthyl-éthylamino)-2-phényl-éthanol

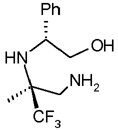

Dans un tricol sous argon, on additionne, par petites portions, 1.6 g d'hydrure de lithium aluminium, sur une solution refroidie à 4 °C, de 2.5 g de (S)-3,3,3-trifluoro-2-((R)-2-hydroxy-1-phényl-éthylamino)-2-méthyl-propionitrile dans 250 mL d'éther éthylique anhydre. On observe un fort dégagement gazeux avec élévation de la température à 8°C.

A la fin de l'addition, on laisse la température remonter à l'ambiante puis on laisse le mélange réactionnel sous agitation pendant 18h. Le mélange obtenu est refroidi à 4°C avant ajout, goutte à goutte et très lentement, de 2 ml d'eau. On observe un fort dégagement gazeux avec élévation de la température jusqu'à 12°C.

Au mélange résultant maintenu à 4°C, on additionne, goutte à goutte et très lentement, 2 ml de potasse à 15% puis, toujours goutte à goutte et très lentement, 4 ml d'eau.

Le précipité blanc formé est filtré et le filtrat obtenu séché sur sulfate de magnésium puis concentré sous pression réduite pour donner 2.2 g de la (R)-2-((S)-1-aminométhyl-2,2,2-trifluoro-1-méthyl-éthylamino)-2-phényl-éthanol dont les caractéristiques sont les suivantes :
Spectrométrie de Masse : méthode A
Temps de rétention Tr (min) = 0,43
[M+H]+ : m/z 263
Pouvoir rotatoire : PR= -51.2+/-1.3; C=1.576 mg/0.5ML DMSO.

### Stade b' :(S)-3,3,3-Trifluoro-2-((R)-2-hydroxy-1-phényl-éthylamino)-2-méthyl-propionitrile

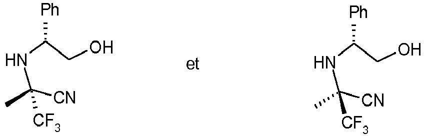

Dans un tricol sous argon, on additionne, goutte à goutte, sur une solution refroidie à 0 °C de 5.3 g de (R)-2-méthyl-4-phényl-2-trifluorométhyl-oxazolidine dans 100 mL de dichlorométhane, 3.4 g de cyanure de trimethysilyle, puis, goutte à goutte, 4.9 g de trifluoro étherate de bore. Le bain froid est ensuite retiré pour laisser la température remonter à l'ambiante. Le mélange résultant est laissé sous agitation à température ambiante pendant 18 heures avant addition d'une solution saturée de bicarbonate de sodium jusqu'à pH = 8. La phase organique est séparée puis séchée sur sulfate de magnésium, filtrée et concentrée sous pression réduite.

Le résidu obtenu est purifié par chromatographie sur silice (éluant : cyclohexane/AcOEt : 80/20) pour donner 3 g du (R)-3,3,3-trifluoro-2-((R)-2-hydroxy-1-phényl-éthylamino)-2-méthyl-propionitrile, sous forme d'une huile incolore et 2.5 g du (S)-3,3,3-trifluoro-2-((R)-2-hydroxy-1-phényl-éthylamino)-2-méthyl-propionitrile, sous forme d'un solide blanc, dont les caractéristiques sont :
Spectrométrie de Masse : méthode A
Temps de rétention Tr (min) = 0,86
[M+H]+ : m/z 259 ; [M-H+HCO2H]- : m/z 303
Pouvoir rotatoire : PR= -89.0+/-1.4; C=2.440mg/0.5ML CHCl3 et PR = -77.6+/-1.4; C=1.818mg/0.5ML DMSO.

### Stade a' : (R,S)-2-Méthyl-4-(R)-phényl-2-trifluorométhyl-oxazolidine

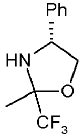

Dans un tricol surmonté d'un Dean-Stark, on additionne sur une solution de 5 g de trifluoroacétone dans 180 mL de toluène, 4.8 g de (R)-phénylglycinol puis, en une seule fois, 0.8 g d'acide para-toluène sulfonate de pyridinium. Le mélange obtenu est ensuite chauffé au reflux pendant 18 heures durant lesquelles on recueille 0.3 mL d'eau.

Après refroidissement, le mélange réactionnel est concentré sous pression réduite. Le résidu obtenu est purifié par filtration sur silice (éluant : dichlorométhane) pour donner 5.3 g de la (R,S)-2-méthyl-4-(R)-phényl-2-trifluorométhyl-oxazolidine, sous forme d'un liquide incolore, dont les caractéristiques sont les suivantes :
Spectrométrie de Masse : méthode A
Temps de rétention Tr (min) = 0,96
[M+H]+ : m/z 232
Pouvoir rotatoire : PR= -23.4+/-0.8; C=1.794mg/0.5ML CH3OH.

### Exemple 2 : (R,S)-1-[2-(4-méthoxyphényl)éthyl]-2-méthyl-7-(morpholin-4-yl)-2-(trifluorométhyl)-2,3-dihydroimidazo[1,2-a]pyrimidin-5(1H)-one

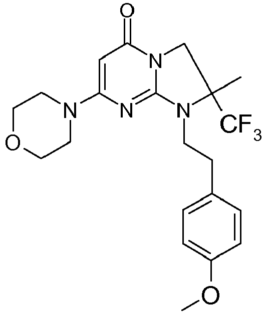

Le produit est préparé en suivant le mode opératoire décrit à l'exemple 1, à partir de 120 mg de la (R,S)-2-méthyl-7-morpholin-4-yl-2-trifluorométhyl-2,3-dihydro-1H-imidazo[1,2-a]pyrimidin-5-one (préparée en suivant le protocole de l'exemple 1j mais à partir de la (R,S)-7-chloro-2-méthyl-2-trifluorométhyl-2,3-dihydro-1H-imidazo[1,2-a]pyrimidin-5-one décrite dans l'exemple 1h) et de 420 mg de bromure de 4-méthoxy phénéthyle. Après purification par chromatographie sur silice (éluant : gradient de 0% à 20% de l'éluant CH2Cl2/MeOH/NH4OH 28% 38/17/2 dans le dichlorométhane), on obtient 80 mg de la (R,S)-1-[2-(4-méthoxy-phényl)-éthyl]-2,6-diméthyl-7-morpholin-4-yl-2-trifluorométhyl-2,3-dihydro-1H-imidazo[1,2-a]pyrimidin-5-one dont les caractéristiques sont les suivantes :
Spectre RM N 1 H:
   1,52 (s large, 3 H) ; 2,73 à 2,84 (m, 1 H) ; 2,90 à 3,01 (m, 1 H) ; 3,36 à 3,59 (m, 6 H) ; 3,61 à 3,68 (m, 4 H) ; 3,72 (s, 3 H) ; 3,84 (d large, J=12,5 Hz, 1 H) ; 4,11 (d, J=12,5 Hz, 1 H) ; 4,88 (s, 1 H) ; 6,87 (d, J=8,6 Hz, 2 H) ; 7,14 (d, J=8,6 Hz, 2 H)
   Spectrométrie de Masse : méthode A
   Temps de rétention Tr (min) = 0,96
   [M+H]+ : m/z 439

### Exemple 3 : (S)-1-Benzyl-2-méthyl-7-morpholin-4-yl-2-trifluorométhyl-2,3-dihydro-1H-imidazo[1,2-a]pyrimidin-5-one

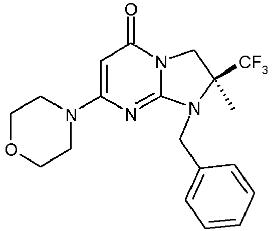

Le produit est préparé en suivant le mode opératoire décrit à l'exemple 1 à partir de 100 mg de la (S)-2-méthyl-7-morpholin-4-yl-2-trifluorométhyl-2,3-dihydro-1H-imidazo[1,2-a]pyrimidin-5-one (exemple 1j) et de 281 mg de bromure de benzyle, en remplaçant l'hydrure de sodium par le carbonate de césium et en ajoutant 10 mg de chlorure de benzyl triéthylammonim (BTEAC). Après purification par chromatographie sur colonne de silice (éluant : dichlorométhane/méthanol 98/02), on obtient 70 mg de la (S)-1-benzyl-2-méthyl-7-morpholin-4-yl-2-trifluorométhyl-2,3-dihydro-1H-imidazo[1,2-a]pyrimidin-5-one dont les caractéristiques sont les suivantes :
Spectre RM N 1 H:
   1.60 (s, 3 H) ; 3.34 (m partiellement masqué, 4 H) ; 3.54 (m, 4 H) ; 3.97 (d,J=12.5 Hz, 1 H) ; 4.16 (d,J=12.5 Hz, 1 H) ; 4.57 (d,J=16.4 Hz, 1 H) ; 4.77 (d,J=16.4Hz, 1 H) ; 4.89 (s, 1 H) ; 7.20 à 7.45 (m, 5 H)
   Spectrométrie de Masse : méthode B
   Temps de rétention Tr (min) = 3,89
   [M+H]+ : m/z 395
   Pouvoir rotatoire : PR= -20.9+/-0.8; C=1.829mg/0.5ML DMSO.

### Exemple 4 : (S)-2-méthyl-7-morpholin-4-yl-1-phénéthyl-2-trifluorométhyl-2,3-dihydro-1H-imidazo[1,2-a]pyrimidin-5-one

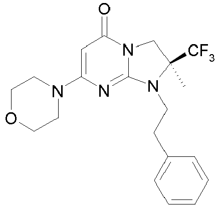

Le produit est préparé en suivant le mode opératoire décrit à l'exemple 1, à partir de 100 mg de la (S)-2-méthyl-7-morpholin-4-yl-2-trifluorométhyl-2,3-dihydro-1H-imidazo[1,2-a]pyrimidin-5-one (exemple 1j) et de 304 mg du (2-bromoéthyl)benzène, en remplaçant l'hydrure de sodium par le carbonate de césium et, en ajoutant 10 mg de chlorure de benzyl triéthylammonim (BTEAC). Après purification par chromatographie sur colonne de silice (éluant : dichlorométhane/méthanol 98/02) on obtient 120 mg de la (S)-2-méthyl-7-morpholin-4-yl-1-phénéthyl-2-trifluorométhyl-2,3-dihydro-1H-imidazo[1,2-a]pyrimidin-5-one dont les caractéristiques sont les suivantes :
Spectre RMN 1H (400MHz, en ppm , DMSO-d6) : 1.52 (s, 3 H) ;
   2.79 à 2.91 (m, 1H) ; 2.97 à 3.08 (m, 1 H) ; 3.40 à 3.51 (m, 5 H) ; 3.54 à 3.68 (m, 1 H) ; 3.64 (t, J=4.8 Hz, 4 H) ; 3.84 (d, J=12.5 Hz, 1 H) ; 4.11 (d, J=12.5 Hz, 1 H) ; 4.88 (s, 1 H) ; 7.20 à 7.26 (m, 3 H) ; 7.27 à 7.37 (m, 2 H)
   Spectrométrie de Masse : méthode B
   Temps de rétention Tr (min) = 4,14
   [M+H]+ : m/z 409
   Pouvoir rotatoire : PR= -34.8+/-0.8; C=2.558mg/0.5ML DMSO.

### Exemple 5 : (S)-2-méthyl-7-morpholin-4-yl-1-(3-phényl-propyl)-2-trifluorométhyl-2,3-dihydro-1H-imidazo[1,2-a]pyrimidin-5-one

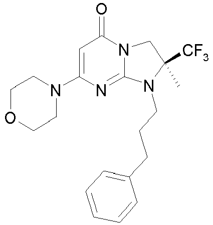

Le produit est préparé en suivant le mode opératoire décrit à l'exemple 1, à partir de 100 mg de la (S)-2-méthyl-7-morpholin-4-yl-2-trifluorométhyl-2,3-dihydro-1H-imidazo[1,2-a]pyrimidin-5-one (exemple 1j) et de 131 mg du 1-bromo-3-phényl propane, en remplaçant l'hydrure de sodium par le carbonate de césium. Après purification par chromatographie sur colonne de silice (éluant : dichlorométhane/méthanol 97/03), on obtient 100 mg de la (S)-2-méthyl-7-morpholin-4-yl-1-(3-phényl-propyl)-2-trifluorométhyl-2,3-dihydro-1H-imidazo[1,2-a]pyrimidin-5-one dont les caractéristiques sont les suivantes :
Spectre RMN 1H :
   1.61 (s, 3 H) ; 1.83 à 2.02 (m, 2 H) ; 2.62 (t, J=7.3 Hz, 2 H) ; 3.26 à 3.42 (m partiellement masqué, 6 H) ; 3.56 à 3.62 (m, 4 H) ; 3.86 (d, J=12.5 Hz, 1 H) ; 4.08(d, J=12.5 Hz, 1 H) ; 4.82 (s, 1 H) ; 7.14 à 7.23 (m, 3 H) ; 7.24 à 7.33 (m, 2 H).
   Spectrométrie de Masse : méthode B
   Temps de rétention Tr (min) = 4,27
   [M+H]+ : m/z 423
   Pouvoir rotatoire : PR= -1.5+/-0.4; C=2.576mg/0.5ML DMSO.

### Exemple 6 : (S)-2-méthyl-7-morpholin-4-yl-1-(2-phénoxy-éthyl)-2-trifluorométhyl-2,3-dihydro-1H-imidazo[1,2-a]pyrimidin-5-one.

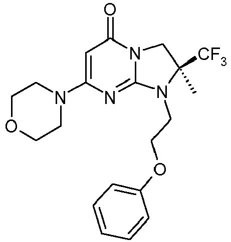

Le produit est préparé en suivant le mode opératoire décrit à l'exemple 1, à partir de 100 mg de la (S)-2-méthyl-7-morpholin-4-yl-2-trifluorométhyl-2,3-dihydro-1H-imidazo[1,2-a]pyrimidin-5-one (exemple 1j) et de 143 mg de l'éther de (2-bromoéthyl)phényl, en remplaçant l'hydrure de sodium par le carbonate de césium. Après purification par chromatographie sur colonne de silice (éluant : dichlorométhane/méthanol 97/03), on obtient 124 mg du (S)-2-méthyl-7-morpholin-4-yl-1-(2-phénoxy-éthyl)-2-trifluorométhyl-2,3-dihydro-1H-imidazo[1,2-a]pyrimidin-5-one dont les caractéristiques sont les suivantes :
Spectre RMN 1 H :
   1.69 (s, 3 H) ; 3.35 à 3.44 (m, 4 H) ; 3.59 (t, J=4.7 Hz, 4 H) ; 3.63 à 3.73 (m, 1 H) ; 3.74 à 3.86 (m, 1 H) ; 3.92 (d, J=12.5 Hz, 1 H) ; 4.10 à 4.30 (m, 3 H) ; 4.88 (s, 1 H) ; 6.83 à 7.00 (m, 3 H) ; 7.24 à 7.32 (m, 2 H).
   Spectrométrie de Masse : méthode A
   Temps de rétention Tr (min) = 0,95
   [M+H]+ : m/z 425

### Exemple 7 : (S)-2-méthyl-7-morpholin-4-yl-1-(2-phénylsulfanyl-éthyl)-2-trifluorométhyl-2,3-dihydro-1H-imidazo[1,2-a]pyrimidin-5-one

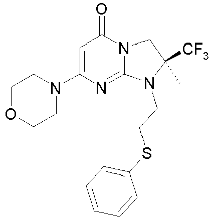

Le produit est préparé en suivant le mode opératoire décrit à l'exemple 1, à partir de 100 mg de la (S)-2-méthyl-7-morpholin-4-yl-2-trifluorométhyl-2,3-dihydro-1H-imidazo[1,2-a]pyrimidin-5-one (exemple 1j) et de 143 mg du sulfure de 2-bromoéthyl phényl, en remplaçant l'hydrure de sodium par le carbonate de césium. Après purification par chromatographie sur colonne de silice (éluant : dichlorométhane/méthanol 97/03), on obtient 96 mg de la (S)-2-méthyl-7-morpholin-4-yl-1-(2-phénylsulfanyl-éthyl)-2-trifluorométhyl-2,3-dihydro-1H-imidazo[1,2-a]pyrimidin-5-one dont les caractéristiques sont les suivantes :
Spectre RMN 1H :
   1.62 (s, 3 H) ; 3.05 à 3.17 (m, 1 H) ; 3.20 à 3.34 (m, 5 H) ; 3.40 à 3.51 (m, 1 H) ; 3.55 à 3.64 (s, 5 H) ; 3.83 (d, J=12.5 Hz, 1 H) ; 4.10 (d, J=12.5 Hz, 1 H) ; 4.86 (s, 1 H) ; 7.26 (t, J=7.5 Hz, 1 H) ; 7.34 (t, J=7.5 Hz, 2 H) ; 7.44 (d, J=7.5 Hz, 2 H).
   Spectrométrie de Masse : méthode A
   Temps de rétention Tr (min) = 1,01
   [M+H]+ : m/z 441

### Exemple 8 : (S)-2-méthyl-7-morpholin-4-yl-1-((R)-2-phényl-propyl)-2-trifluorométhyl-2,3-dihydro-1H-imidazo[1,2-a]pyrimidin-5-one

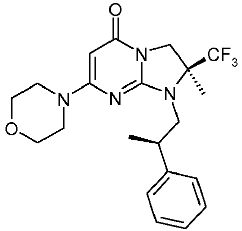

Le produit est préparé en suivant le mode opératoire décrit à l'exemple 1, à partir de 100 mg de la (S)-2-méthyl-7-morpholin-4-yl-2-trifluorométhyl-2,3-dihydro-1H-imidazo[1,2-a]pyrimidin-5-one (exemple 1j) et de 143 mg du 1-bromo-2-phényl propane , en remplaçant l'hydrure de sodium par le carbonate de césium. Après purification par chromatographie sur colonne de silice (éluant : dichlorométhane/méthanol 97/03), on obtient 100 mg de (2S)-2-méthyl-7-morpholin-4-yl-1-((R et S)-2-phényl-propyl)-2-trifluorométhyl-2,3-dihydro-1H-imidazo[1,2-a]pyrimidin-5-one.

La séparation des deux diastéréoisomères de la (2S)-2-méthyl-7-morpholin-4-yl-1-(2-phényl-propyl)-2-trifluorométhyl-2,3-dihydro-1H-imidazo[1,2-a]pyrimidin-5-one a été réalisée par chromatographie chirale :
phase stationnaire : Chiralpak AD, phase mobile: EtOH (04%) / MeOH (01 %) / Heptane (95%).

Le premier diastéréoisomère est concentré pour donner 17 mg de la (S)-2-méthyl-7-morpholin-4-yl-1-((R)-2-phényl-propyl)-2-trifluorométhyl-2,3-dihydro-1H-imidazo[1,2-a]pyrimidin-5-one dont les caractéristiques sont les suivantes :
Spectre RMN 1H :
   1.25 (d, J=6.4 Hz, 3 H) ; 1.68 (s, 3 H) ; 3.32 à 3.51 (m, 7 H) ; 3.60 à 3.67 (m, 4 H) ; 3.91 (d, J=12.4 Hz, 1 H) ; 4.12 (d, J=12.4 Hz, 1 H) ; 4.87 (s, 1 H) ; 7.20 à 7.24 (m, 1 H) ; 7.25 à 7.36 (m, 4 H).
   Spectrométrie de Masse : méthode A
   Temps de rétention Tr (min) = 1,01
   [M+H]+ : m/z 423

### Exemple 9 : (S)-2-méthyl-7-morpholin-4-yl-1-((S)-2-phényl-propyl)-2-trifluorométhyl-2,3-dihydro-1H-imidazo[1,2-a]pyrimidin-5-one

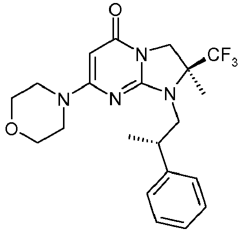

Le deuxième diastéréoisomère obtenu à l'exemple 8 est concentré pour donner 19 mg de la (S)-2-méthyl-7-morpholin-4-yl-1-((S)-2-phényl-propyl)-2-trifluorométhyl-2,3-dihydro-1H-imidazo[1,2-a]pyrimidin-5-one dont les caractéristiques sont les suivantes :
Spectre RMN 1 H :
   1.08 (s, 3 H) ; 1.26 (d, J=6.8 Hz, 3 H) ; 3.35 à 3.70 (m, 12 H) ; 4.05 (d, J=12.7 Hz, 1 H) ; 4.88 (s, 1 H) ; 7.18 à 7.25 (m, 3 H) ; 7.27 à 7.34 (m, 2 H).
   Spectrométrie de Masse : méthode A
   Temps de rétention Tr (min) = 1,01
   [M+H]+ : m/z 423

### Exemple 10 : (S)-1-((S)-2-hydroxy-2-phényl-éthyl)-2-méthyl-7-morpholin-4-yl-2-trifluorométhyl-2,3-dihydro-1H-imidazo[1,2-a]pyrimidin-5-one

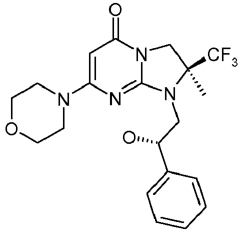

Le produit est préparé en suivant le mode opératoire décrit à l'exemple 1, à partir de 200 mg de la (R,S)-2-méthyl-7-morpholin-4-yl-2-trifluorométhyl-2,3-dihydro-1H-imidazo[1,2-a]pyrimidin-5-one (préparée en suivant le protocole de l'exemple 1j mais à partir de la (R,S)-7-chloro-2-méthyl-2-trifluorométhyl-2,3-dihydro-1H-imidazo[1,2-a]pyrimidin-5-one décrite dans l'exemple 1h) et de 0.4 mL de (S)-2-chloro-1-phényl-éthanol, en remplaçant l'hydrure de sodium par le carbonate de césium et en ajoutant 10 mg de chlorure de benzyl triéthylammonim (BTEAC). Après purification par LC/MS préparative puis retour à la base par passage sur colonne de silice (éluant : dichlorométhane/méthanol/triéthylamine 98/02/0.5), on obtient 100 mg de la (S)-1-((S)-2-hydroxy-2-phényl-éthyl)-2-méthyl-7-morpholin-4-yl-2-trifluorométhyl-2,3-dihydro-1H-imidazo[1,2-a]pyrimidin-5-one dont les caractéristiques sont les suivantes :
Spectre RMN 1 H :
   1.71 (s, 3 H) ; 3.17 (dd, J=9.7 et 14.4 Hz, 1 H) ; 3.40 à 3.52 (m, 4 H) ; 3.56 (dd, J=2.9 et 14.4 Hz, 1 H) ; 3.65 (t, J=4.9 Hz, 4 H) ; 3.85 (d, J=12.5 Hz, 1 H) ; 4.18 (d, J=12.5 Hz, 1 H) ; 4.90 (s, 1 H) ; 5.06 à 5.15 (m, 1 H) ; 5.60 (d, J=4.4 Hz, 1 H) ; 7.23 à 7.43 (m, 5 H).
   Spectrométrie de Masse : méthode A
   Temps de rétention Tr (min) = 0,85
   [M+H]+ : m/z 425 ; [MHCO2H-H]- : m/z 469
   Pouvoir rotatoire : PR= -45.1+/-1.0; C=2.151mg/0.5ML DMSO.

Les purifications ci-dessus conduisent également à 36 mg du second diastéréoisomère, la (R)-1-((S)-2-hydroxy-2-phényl-éthyl)-2-méthyl-7-morpholin-4-yl-2-trifluorométhyl-2,3-dihydro-1H-imidazo[1,2-a]pyrimidin-5-one.

### Exemple 11 : (S)-1-((R)-2-hydroxy-2-phényl-éthyl)-2-méthyl-7-morpholin-4-yl-2-trifluorométhyl-2,3-dihydro-1H-imidazo[1,2-a]pyrimidin-5-one

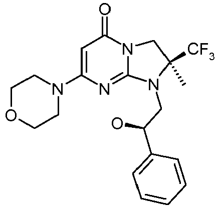

Le produit est préparé en suivant le mode opératoire décrit à l'exemple 1, à partir de 275 mg de la (R,S)-2-méthyl-7-morpholin-4-yl-2-trifluorométhyl-2,3-dihydro-1H-imidazo[1,2-a]pyrimidin-5-one (préparée en suivant le protocole de l'exemple 1j mais à partir de la (R,S)-7-chloro-2-méthyl-2-trifluorométhyl-2,3-dihydro-1H-imidazo[1,2-a]pyrimidin-5-one décrite dans l'exemple 1h) et de 0.155 mL de (R)-2-chloro-1-phényl-éthanol. Après purification par chromatographie sur colonne de silice, (éluant : dichlorométhane /méthanol 97/03) puis purification par LC/MS préparative et retour à la base par passage sur colonne de silice (éluant : dichlorométhane/méthanol/triéthylamine 98/02/0.5), on obtient 40 mg de la (S)-1-((R)-2-hydroxy-2-phényl-éthyl)-2-méthyl-7-morpholin-4-yl-2-trifluorométhyl-2,3-dihydro-1H-imidazo[1,2-a]pyrimidin-5-one dont les caractéristiques sont les suivantes :
Spectre RMN 1 H :
   1.10 (s, 3 H) ; 3.25 (dd, J=6.8 et 13.5 Hz, 1 H) ; 3.38 à 3.49 (m, 4 H) ; 3.64 (m, 6 H) ; 4.07 (d, J=12.5 Hz, 1 H) ; 4.88 (s, 1 H) ; 5.05 à 5.13 (m, 1 H) ; 5.55 (d, =4.2 Hz, 1 H) ; 7.20 à 7.45 (m, 5 H).
   Spectrométrie de Masse : méthode B
   Temps de rétention Tr (min) = 3,48
   [M+H]+ : m/z 425 ; [M+HCO2H-H]- : m/z 469
   Pouvoir rotatoire : PR= +75.0+/-1.4; C=1.794mg/0.5ML DMSO.

Les purifications ci-dessus conduisent également à un second diastéréoisomère, la (R)-1-((R)-2-hydroxy-2-phényl-éthyl)-2-méthyl-7-morpholin-4-yl-2-trifluorométhyl-2,3-dihydro-1H-imidazo[1,2-a]pyrimidin-5-one.

### Exemple 12 : (2S)-2-méthyl-1-((R) ou (S)-1-méthyl-2-phényl-éthyl)-7-morpholin-4-yl-2-trifluorométhyl-2,3-dihydro-1H-imidazo[1,2-a]pyrimidin-5-one

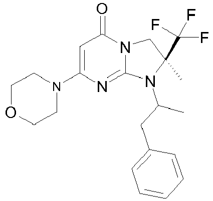

A une solution de 400 mg de la (S)-2-méthyl-7-morpholin-4-yl-2-trifluorométhyl-2,3-dihydro-1H-imidazo[1,2-a]pyrimidin-5-one (exemple 1j) dans 5 mL de toluène, est ajoutée, à température ambiante et sous atmosphère d'argon, une solution de 800 mg de soude dans 5 mL d'eau puis 90 mg d'hydrogéno sulfate de tétrabutylammonium et 524 mg de (R,S)-2-bromo-1-phénylpropane dans 5 mL de tétrahydrofuranne. Le mélange obtenu est alors chauffé à 60°C pendant dix-huit heures. Après refroidissement, on ajoute au mélange résultant 50 mL d'acétate d'éthyle et une solution aqueuse saturée en chlorure de sodium. La phase organique est séparée puis séchée sur sulfate de magnésium, filtrée et concentrée sous pression réduite. Le résidu obtenu est purifié par chromatographie sur silice (éluant : CH2Cl2/MeOH : 97/03) pour donner 110 mg d'un résidu qui est purifié sur colonne chirale :
conditions : phase stationnaire : Chiralpak IA; phase mobile: EtOH (05%) / Heptane (95%) puis, deuxième phase stationnaire: Hypersil C18 Elite, phase mobile: ACN (40%) / H2O (60%).

On obtient ainsi 8.2 mg de la (S)-2-méthyl-1-(1-méthyl-2-phényl-éthyl)-7-morpholin-4-yl-2-trifluorométhyl-2,3-dihydro-1H-imidazo[1,2-a]pyrimidin-5-one, sous forme d'un seul diastéréoisomère de configuration indéterminée sur la chaine phénéthyle et dont les caractéristiques sont les suivantes :
Spectre RMN 1 H :
   1,31 (d, J=6,8 Hz, 3 H) ; 1,66 (s, 3 H) ; 3,01 à 3,13 (m, 1 H) ; 3,35 à 3,43 (m, 1 H) ; 3,45 à 3,49 (m, 4 H) ; 3,65 à 3,71 (m, 4 H) ; 3,74 (s, 1 H) ; 3,87 (d, J=12,2 Hz, 1 H) ; 4,06 (d, J=12,2 Hz, 1 H) ; 4,86 à 4,92 (m, 1 H) ; 7,15 à 7,25 (m, 3 H) ; 7,28 à 7,35 (m, 2 H)
   Spectrométrie de Masse : méthode B
   Temps de rétention Tr (min) = 4,30
   [M+H]+ : m/z 423

### Exemple 13 : (S)-2-méthyl-7-morpholin-4-yl-1-((R) ou (S) -1-phényl-propyl)-2-trifluorométhyl-2,3-dihydro-1H-imidazo[1,2-a]pyrimidin-5-one

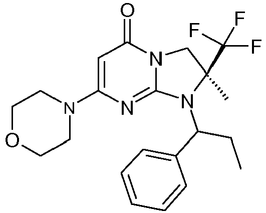

La séparation chromatographique décrite ci-dessus, à l'exemple 12, a également donné 11.2 mg d'un premier diastéréosiomère de la (S)-2-méthyl-7-morpholin-4-yl-1-(1-phényl-propyl)-2-trifluorométhyl-2,3-dihydro-1H-imidazo[1,2-a]pyrimidin-5-one, de configuration indéterminée sur la chaine benzyle et dont les caractéristiques sont les suivantes :
Spectre RM N 1 H:
   0,90 (t, J=7,5 Hz, 3 H) ; 1,57 (s, 3 H) ; 2,34 à 2,47 (m, 2 H) ; 3,39 (m, 4 H) ; 3,62 (m, 4 H) ; 3,86 (d, J=12,7 Hz, 1 H) ; 4,13 (d, J=12,7 Hz, 1 H) ; 4,48 (t, J=7,5 Hz, 1 H) ; 4,88 (s, 1 H) ; 7,25 (t, J=7,5 Hz, 1 H) ; 7,30 à 7,36 (t, J=7,5 Hz, 2 H) ; 7,55 (d, J=7,5 Hz, 2 H)
   Spectrométrie de Masse : méthode B
   Temps de rétention Tr (min) = 4,25
   [M+H]+ : m/z 423

### Exemple 14 : (S)-2-méthyl-7-morpholin-4-yl-1-((S) ou (R)-1-phényl-propyl)-2-trifluorométhyl-2,3-dihydro-1H-imidazo[1,2-a]pyrimidin-5-one

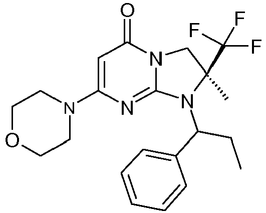

La séparation chirale décrite ci-dessus, à l'exemple 12, a aussi donné 40.5 mg du second diastéréoisomère de la (S)-2-méthyl-7-morpholin-4-yl-1-(1-phényl-propyl)-2-trifluorométhyl-2,3-dihydro-1H-imidazo[1,2-a]pyrimidin-5-one, de configuration indéterminée sur la chaine benzyle et dont les caractéristiques sont les suivantes :
Spectre RM N 1 H:
   0,89 (t, J=7,5 Hz, 3 H) ; 1,71 (s, 3 H) ; 1,94 à 2,08 (m, 1 H) ; 2,52 à 2,59 (m, 1 H) ; 3,38 (m, 4 H) ; 3,61 (m, 4 H) ; 3,98 (d, J=12,7 Hz, 1 H) ; 4,12 (d, J=12,7 Hz, 1 H) ; 4,50 (dd, J=7,1 et 8,6 Hz, 1 H) ; 4,92 (s, 1 H) ; 7,21 (t, J=7,5 Hz, 1 H) ; 7,29 (t, J=7,5 Hz, 2 H) ; 7,55 (d, J=7,5 Hz, 2 H)
   Spectrométrie de Masse : méthode B
   Temps de rétention Tr (min) = 4,14
   [M+H]+ : m/z 423

### Exemple 15 : (S)-2-méthyl-7-morpholin-4-yl-1-[2-(4-morpholin-4-yl-phényl)-éthyl]-2-trifluorométhyl-2,3-dihydro-1H-imidazo[1,2-a]pyrimidin-5-one

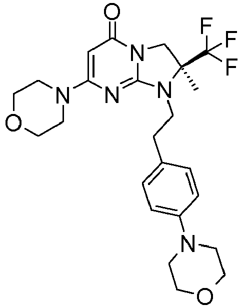

A une solution de 100 mg de la (S)-2-méthyl-7-morpholin-4-yl-2-trifluorométhyl-2,3-dihydro-1H-imidazo[1,2-a]pyrimidin-5-one (exemple 1j) dans 5 mL de tétrahydrofuranne, sont ajoutés 135 mg de 2-(4-morpholino-phényl)éthanol et 223 mg de triphénylphosphine supportée sur polymère (3 mmol/g). Après agitation pendant cinq minutes à température ambiante, on ajoute 0.12 mL d'azodicarboxylate de diéthyle. Le mélange réactionnel résultant est alors agité pendant une nuit à température ambiante. Après filtration, on évapore le filtrat sous pression réduite. Le résidu obtenu est purifié par chromatographie sur colonne de silice (éluant : dichlorométhane/méthanol 97/03), pour donner 40 mg de la (S)-2-méthyl-7-morpholin-4-yl-1-[2-(4-morpholin-4-yl-phényl)-éthyl]-2-trifluorométhyl-2,3-dihydro-1H-imidazo[1,2-a]pyrimidin-5-one dont les caractéristiques sont les suivantes :
Spectre RM N 1 H:
   1,53 (s, 3 H) ; 2,70 à 2,81 (m, 1 H) ; 2,86 à 2,98 (m, 1 H) ; 3,02 à 3,08 (m, 4 H) ; 3,35 à 3,58 (m, 6 H) ; 3,62 à 3,67 (m, 4 H) ; 3,70 à 3,75 (m, 4 H) ; 3,84 (d, J=12,5 Hz, 1 H) ; 4,11 (d, J=12,5 Hz, 1 H) ; 4,88 (s, 1 H) ; 6,88 (d, J=8,6 Hz, 2 H) ; 7,08 (d, J=8,6 Hz, 2 H)
   Spectrométrie de Masse : méthode A
   Temps de rétention Tr (min) = 0,87
   [M+H]+ : m/z 494 ; [M+2H]2+: m/z 247,5 (pic de base)

### Exemple 16 : (2S)-2-méthyl-7-(morpholin-4-yl)-1-((R) et (S)-1-phényléthyl)-2-(trifluorométhyl)-2,3-dihydroimidazo[1,2-a]pyrimidin-5(1H)-one

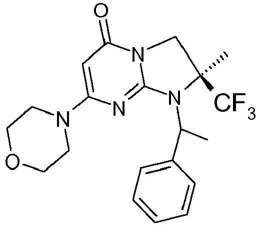

### Stade b :

Dans un ballon, on introduit 190 mg de la (S)-7-chloro-2-méthyl-1-(1-phényléthyl)-2-(trifluorométhyl)-2,3-dihydroimidazo[1,2-a]pyrimidin-5(1H)-one et 3 mL de morpholine. Le mélange résultant est chauffé à 80 °C pendant 30 minutes. Après refroidissement, le mélange réactionnel est concentré sous pression réduite. Le résidu est purifié par chromatographie sur silice (éluant : CH2Cl2/MeOH 97.5/2.5) pour donner 28 mg d'un mélange 1/2 des deux diastéréoisomères de la (2S)-2-méthyl-7-(morpholin-4-yl)-1-(1-phényléthyl)-2-(trifluorométhyl)-2,3-dihydroimidazo[1,2-a]pyrimidin-5(1H)-one dont les caractéristiques sont les suivantes :
Spectre RMN 1 H :
   Mélange 2/3 - 1/3 de diastéréoisomères avec : 1,74 à 1,79 (m, 5 H) ; 1,82 (d, J=7,0 Hz, 1 H) ; 3,16 à 3,26 (m, 4 H) ; 3,41 à 3,56 (m, 4 H) ; 3,91 à 4,02 (m, 1 H) ; 4,13 (d, J=12,5 Hz, 0,65 H) ; 4,17 (d, J=12,5 Hz, 0,35 H) ; 4,79 (s, 0,65 H) ; 4,85 (s, 0,35 H) ; 4,86 à 4,94 (m, 1 H) ; 7,16 à 7,26 (m, 1 H) ; 7,27 à 7,35 (m, 2 H) ; 7,42 (d, J=7,8 Hz, 1,3 H) ; 7,46 (d, J=7,8 Hz, 0,7 H)
   Spectrométrie de Masse : méthode A
   Temps de rétention Tr (min) = 0,93 et 0,94 avec mélange 2/3 - 1/3 de diastéréoisomères
   [M+H]+ : m/z 409

### Stade a :

Dans un ballon, on introduit 200 mg de (S)-7-chloro-2-méthyl-2-trifluorométhyl-2,3-dihydro-1H-imidazo[1,2-a]pyrimidin-5-one préparé (exemple 1i) dans 5 mL de tétrahydrofuranne, 192 mg de (R,S)-phényl éthanol et 537 mg de triphénylphosphine supportée sur polymère (3mmol/g). Après avoir agité 5 minutes à température ambiante, on ajoute 275 mg de (E)-diazène-1,2-dicarboxylate de diéthyle (DIAD). Le mélange réactionnel est ensuite agité 4 heures à température ambiante avant filtration. Le filtrat est alors concentré sous pression réduite et le résidu est purifié par chromatographie sur silice (éluant : CH2Cl2/AcOEt : 96/04) pour donner 200 mg d'un mélange 90/10 des deux diastéréoisomères de la (S)-7-chloro-2-méthyl-1-(1-phényléthyl)-2-(trifluorométhyl)-2,3-dihydroimidazo[1,2-a]pyrimidin-5(1H)-one dont les caractéristiques sont les suivantes :
Spectrométrie de Masse : méthode B
Temps de rétention Tr (min) = 4,56 et 4,47 (mélange de diastéréoisomères 90% -10%)
[M+H]+ : m/z 358

### Exemple 17 : 1-[2-(4-Methoxy-phenyl)-ethyl]-2,2-dimethyl-7-morpholin-4-yl-2,3-dihydro-1H-imidazo[1,2-a]pyrimidin-5-one

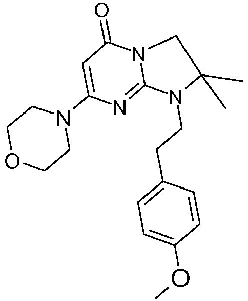

### Stade e : 1-[2-(4-méthoxy-phényl)-éthyl]-2,2-diméthyl-7-morpholin-4-yl-2,3-dihydro-1H-imidazo[1,2-a]pyrimidin-5-one

Le produit est préparé en suivant le mode opératoire décrit à l'exemple 1, à partir de 100 mg de la 2,2-diméthyl-7-morpholin-4-yl-2,3-dihydro-1H-imidazo[1,2-a]pyrimidin-5-one et de 0.94 mL de bromure de 4-méthoxyphénéthyle, en remplaçant l'hydrure de sodium par le carbonate de césium. Après purification par chromatographie sur colonne de silice (éluant : dichlorométhane/méthanol : 98/02), on obtient 39 mg de la 1-[2-(4-méthoxy-phényl)-éthyl]-2,2-diméthyl-7-morpholin-4-yl-2,3-dihydro-1H-imidazo[1,2-a]pyrimidin-5-one dont les caractéristiques sont les suivantes :
Spectre RM N 1 H:
   1,21 (s, 6 H) ; 2,83 (t, J=7,7 Hz, 2 H) ; 3,32 à 3,38 (m, 2 H) ; 3,40 à 3,45 (m, 4 H) ; 3,59 (s, 2 H) ; 3,61 à 3,65 (m, 4 H) ; 3,72 (s, 3 H) ; 4,78 (s, 1 H) ; 6,86 (d, J=8,6 Hz, 2 H) ; 7,14 (d, J=8,6 Hz, 2 H)
   Spectrométrie de Masse : Methode A
   Temps de rétention Tr (min) = 0,84
   [M+H]+ : m/z 385

### Stade d : 2,2-Diméthyl-7-morpholin-4-yl-2,3-dihydro-1H-imidazo[1,2-a]pyrimidin-5-one

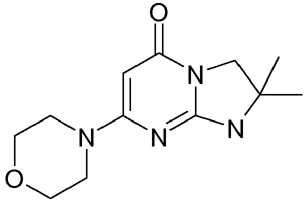

Un mélange de 1 g de la 7-chloro-2,2-diméthyl-2,3-dihydro-1H-imidazo[1,2-a]pyrimidin-5-one et de 10 mL de morpholine est chauffé à 120°C pendant 1 heure. Après refroidissement, le mélange réactionnel est concentré sous pression réduite. Le résidu obtenu est purifié par chromatographie sur silice (éluant : CH2Cl2/MeOH 97/03) pour donner 650 mg de la 2,2-diméthyl-7-morpholin-4-yl-2,3-dihydro-1H-imidazo[1,2-a]pyrimidin-5-one dont les caractéristiques sont les suivantes :
Spectrométrie de Masse : méthode A
   [M+H]+ : m/z 251

### Stade c : 7-Chloro-2,2-diméthyl-2,3-dihydro-1H-imidazo[1,2-a]pyrimidin-5-one

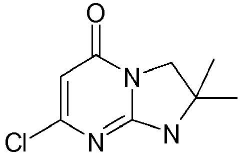

Dans un ballon, on introduit 4,5 g de la 7-hydroxy-2,2-diméthyl-2,3-dihydro-1H-imidazo[1,2-a]pyrimidin-5-one et 35 mL d'oxychlorure de phosphore. Le mélange résultant est alors chauffé à 120°C pendant trois heures. Après refroidissement, le mélange réactionnel est concentré à sec sous pression réduite. Sur le résidu obtenu, on additionne de la glace, puis on ajoute de la soude concentrée jusqu'à l'obtention d'un pH proche de 5-6. Le solide formé est filtré pour donner 1 g de la 7-chloro-2,2-diméthyl-2,3-dihydro-1H-imidazo[1,2-a]pyrimidin-5-one, sous forme de solide marron, dont les caractéristiques sont les suivantes :
Spectrométrie de Masse : méthode B
[M+H]+ : m/z 200 ; [M-H]- : m/z 198

### Stade b : 7-hydroxy-2,2-diméthyl-2,3-dihydro-1H-imidazo[1,2-a]pyrimidin-5-one

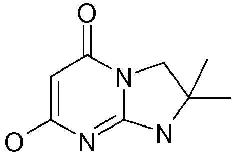

On procède suivant le mode opératoire décrit au stade g de l'exemple 1, à partir de 5 g du bromhydrate de la 4,4-diméthyl-imidazolidin-2-ylidèneamine, de 4 mL de malonate de diéthyle et de 2.8 g de méthylate de sodium. On obtient ainsi 4.5 g de 7-hydroxy-2,2-diméthyl-2,3-dihydro-1H-imidazo[1,2-a]pyrimidin-5-one, sous forme d'un solide blanc.

### Stade a : Bromhydrate de la 4,4-diméthyl-imidazolidin-2-ylidèneamine

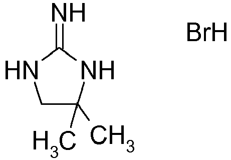

On procède suivant le mode opératoire décrit au stade f de l'exemple 1 à partir de 21 g de 1,2-diamino-2-méthylpropane et de 25.3 g de bromure de cyanogène. On obtient ainsi 46 g de bromhydrate de la 4,4-diméthyl-imidazolidin-2-ylidèneamine, sous forme d'un solide blanc, dont les caractéristiques sont les suivantes :
Spectrométrie de Masse : méthode B
[M+H]+ : m/z 114

### Exemple 18 : (2S)-6-fluoro-1-[2-(4-méthoxyphényl)éthyl]-2-méthyl-7-(morpholin-4-yl)-2-(trifluorométhyl)-2,3-dihydroimidazo[1,2-a]pyrimidin-5(1H)-one

### Stade e :

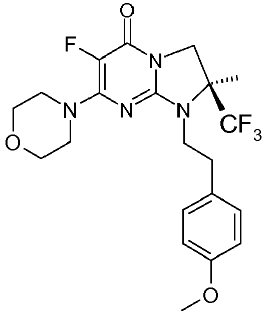

La séparation des deux énantiomères de la (2R, 2S)-6-fluoro-1-[2-(4-méthoxyphényl)éthyl]-2-méthyl-7-(morpholin-4-yl)-2-(trifluorométhyl)-2,3-dihydroimidazo[1,2-a]pyrimidin-5(1H)-one a été réalisée par chromatographie chirale à partir de 130 mg du mélange racémique :
Phase stationnaire : Chiralcel OJ 20µm ; phase mobile: EtOH (100%)

On obtient ainsi 62 mg de la (2S)-6-fluoro-1-[2-(4-méthoxyphényl)éthyl]-2-méthyl-7-(morpholin-4-yl)-2-(trifluorométhyl)-2,3-dihydroimidazo[1,2-a]pyrimidin-5(1H)-one dont les caractéristiques sont les suivantes:
Spectre RMN 1 H (500MHz) :
   1,55 (s, 3 H) ; 2,79 (m, 1 H) ; 2,93 (m, 1 H) ; 3,41 (m, 1 H) ; 3,52 (m, 1 H) ; 3,59 (m, 4 H) ; 3,69 (m, 4 H) ; 3,74 (s, 3 H) ; 3,94 (d, J=12,3 Hz, 1 H) ; 4,17 (d, J=12,3 Hz, 1 H) ; 6,89 (d, J=8,2 Hz, 2 H) ; 7,15 (d, J=8,2 Hz, 2 H)
   Spectrométrie de Masse : méthode A
   Temps de rétention Tr (min) = 1,01
   [M+H]+ : m/z 457

### Stade d : (2R, 2S)-6-fluoro-1-[2-(4-méthoxyphényl)éthyl]-2-méthyl-7-(morpholin-4-yl)-2-(trifluorométhyl)-2,3-dihydroimidazo[1,2-a]pyrimidin-5(1H)-one

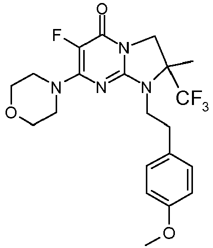

A une solution de 120 mg de la (2R, 2S)-6-fluoro-2-méthyl-7-(morpholin-4-yl)-2-(trifluorométhyl)-2,3-dihydroimidazo[1,2-a]pyrimidin-5(1H)-one dans 5 mL d'acétonitrile sont ajoutés 243 mg de carbonate de césium et 120 mg de bromure de 4-méthoxyphénéthyle. Le mélange réactionnel est alors chauffé à 60°C pendant sept heures. Après refroidissement, le mélange réactionnel est concentré sous pression réduite. Sur le résidu obtenu, on ajoute 5 mL d'eau froide et 20 mL d'acétate d'éthyle. La phase organique est séparée, séchée sur sulfate de magnésium, filtrée puis concentrée sous pression réduite. Le résidu obtenu est purifié par chromatographie sur silice (éluant: dichlorométhane/méthanol: 97.5/2.5) pour donner 130 mg de la (2R, 2S)-6-fluoro-1-[2-(4-méthoxyphényl)éthyl]-2-méthyl-7-(morpholin-4-yl)-2-(trifluorométhyl)-2,3-dihydroimidazo[1,2-a]pyrimidin-5(1H)-one dont les caractéristiques sont les suivantes :
Spectrométrie de Masse : Méthode B
Temps de rétention Tr (min) = 4,27
[M+H]+ : m/z 457

### Stade c : (2R, 2S)-6-fluoro-2-méthyl-7-(morpholin-4-yl)-2-(trifluorométhyl)-2,3-dihydroimidazo[1,2-a]pyrimidin-5(1H)-one

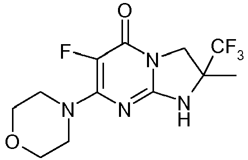

Un mélange de 220 mg de la (2R, 2S)-7-chloro-6-fluoro-2-méthyl-2-(trifluorométhyl)-2,3-dihydroimidazo[1,2-a]pyrimidin-5(1H)-one dans 3 mL de morpholine est chauffé à 60°C pendant trois heures. Après refroidissement, le mélange réactionnel est concentré sous pression réduite. Sur le résidu obtenu, on ajoute 5 mL d'eau froide et 20 mL d'acétate d'éthyle. La phase organique est séparée, séchée sur sulfate de magnésium, filtrée puis concentrée sous pression réduite pour donner 220 mg de la (2R, 2S)-6-fluoro-2-méthyl-7-(morpholin-4-yl)-2-(trifluorométhyl)-2,3-dihydroimidazo[1,2-a]pyrimidin-5(1H)-one dont les caractéristiques sont les suivantes:
Spectrométrie de Masse : méthode A
Temps de rétention Tr (min) = 0,55
[M+H]+ : m/z 323 ; [M-H]- : m/z 321

### Stade b: (2R, 2S)-7-chloro-6-fluoro-2-méthyl-2-(trifluorométhyl)-2,3-dihydroimidazo[1,2-a]pyrimidin-5(1H)-one

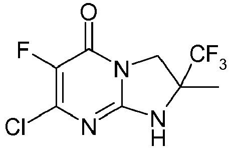

Le produit est préparé en suivant le mode opératoire décrit au stade h de l'exemple 1 à partir de 430 mg de la (2R, 2S)-6-fluoro-7-hydroxy-2-méthyl-2-(trifluorométhyl)-2,3-dihydroimidazo[1,2-a]pyrimidin-5(1H)-one (tel qu'isolée à l'étape (a) ci-dessous) au lieu du (2R, 2S)-7-hydroxy-2-méthyl-2-trifluorométhyl-2,3-dihydro-1H-imidazo[1,2-a]pyrimidin-5-one et 0.800 mL d'oxychlorure de phosphore. Après purification par chromatographie sur colonne de silice (éluant : dichlorométhane/méthanol 97/03), on obtient 220 mg de la (2R, 2S)-7-chloro-6-fluoro-2-méthyl-2-(trifluorométhyl)-2,3-dihydroimidazo[1,2-a]pyrimidin-5(1H)-one dont les caractéristiques sont les suivantes :
Spectrométrie de Masse : Méthode B
Temps de rétention Tr (min) = 2,92
[M+H]+ : m/z 272 ; [M-H]- : m/z 270

### Stade a : (2R, 2S)-6-fluoro-7-hydroxy-2-méthyl-2-(trifluorométhyl)-2,3-dihydroimidazo[1,2-a]pyrimidin-5(1H)-one

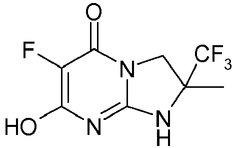

Le produit est préparé en suivant le mode opératoire décrit au stade g de l'exemple 1 à partir de 1 g de 4-méthyl-4-trifluorométhyl-imidazolidin-2-ylideneamine, 605 mg de fluoro-propanedioate de diméthyle au lieu du malonate de diéthyle et 440 mg de méthylate de sodium. On obtient ainsi 490 mg d'un mélange contenant 50% de la (2R, 2S)-6-fluoro-7-hydroxy-2-méthyl-2-(trifluorométhyl)-2,3-dihydroimidazo[1,2-a]pyrimidin-5(1H)-one qui est utilisé tel que dans l'étape suivante.

### Exemple 19 : (2S)-1-benzyl-6-fluoro-2-méthyl-7-(morpholin-4-yl)-2-(trifluorométhyl)-2,3-dihydroimidazo[1,2-a]pyrimidin-5(1H)-one

### Stade b :

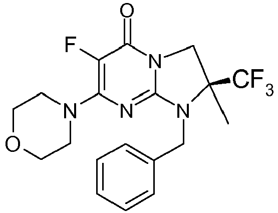

La séparation des deux énantiomères de la (R,S)-1-benzyl-6-fluoro-2-méthyl-7-(morpholin-4-yl)-2-(trifluorométhyl)-2,3-dihydroimidazo[1,2-a]pyrimidin-5(1 H)-one a été réalisée par chromatographie chirale à partir de 75 mg du mélange racémique :
Phase stationnaire : Whelk 01 RR
Phase mobile : Heptane 80% EtOH 20%

On obtient ainsi 35 mg de la (2S)-1-benzyl-6-fluoro-2-méthyl-7-(morpholin-4-yl)-2-(trifluorométhyl)-2,3-dihydroimidazo[1,2-a]pyrimidin-5(1H)-one dont les caractéristiques sont les suivantes :
Spectre RMN 1 H (400MHz) :
   1,63 (s, 3 H) ; 3,45 (m, 4 H) ; 3,53 (m, 4 H) ; 4,06 (d, J=12,2 Hz, 1 H) ; 4,21 (d, J=12,2 Hz, 1 H) ; 4,55 (d, J=16,5 Hz, 1 H) ; 4,61 (d, J=16,5 Hz, 1 H) ; 7,22 à 7,28 (m, 1 H) ; 7,29 à 7,38 (m, 4 H)
   Spectrométrie de Masse : méthode A
   Temps de rétention Tr (min) = 0,95 ;
   [M+H]+ : m/z 413

### Stade a : (R,S)-1-benzyl-6-fluoro-2-méthyl-7-(morpholin-4-yl)-2-(trifluorométhyl)-2,3-dihydroimidazo[1,2-a]pyrimidin-5(1H)-one

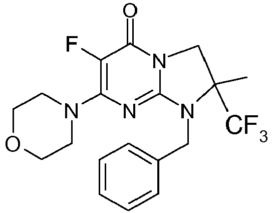

A une solution de 100 mg de la (R,S)-6-fluoro-2-méthyl-7-(morpholin-4-yl)-2-(trifluorométhyl)-2,3-dihydroimidazo[1,2-a]pyrimidin-5(1H)-one (préparée en suivant le protocole de l'exemple 18c) dans 5 mL d'acétonitrile sont ajoutés 121 mg de carbonate de césium et 0.074 mL de bromure de benzyle. Le mélange réactionnel est alors agité à température ambiante pendant une heure. Le mélange réactionnel résultant est concentré sous pression réduite. Sur le résidu obtenu, on ajoute 5 mL d'eau froide et 20 mL d'acétate d'éthyle. La phase organique est séparée, séchée sur sulfate de magnésium, filtrée puis concentrée sous pression réduite. Le résidu obtenu est purifié par chromatographie sur silice (éluant: CH₂Cl₂/MeOH: 97.5/2.5) pour donner 75 mg de la (R,S)-1-benzyl-6-fluoro-2-méthyl-7-(morpholin-4-yl)-2-(trifluorométhyl)-2,3-dihydroimidazo[1,2-a]pyrimidin-5(1H)-one dont les caractéristiques sont les suivantes :
Spectrométrie de Masse : Méthode B
Temps de rétention Tr (min) = 4,10
[M+H]+ : m/z 413

### Exemple 20 : (2S)-1-[(5-chloro-1-benzothiophén-3-yl)méthyl]-2-méthyl-7-(morpholin-4-yl)-2-(trifluorométhyl)-2,3-dihydroimidazo[1,2-a]pyrimidin-5(1H)-one

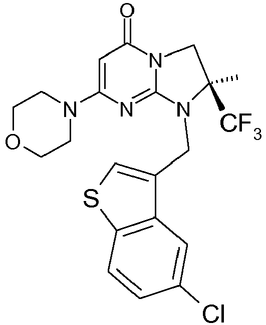

Le produit est préparé en suivant le mode opératoire décrit au stade k de l'exemple 1 à partir de 100 mg de la (2S)-2-méthyl-7-morpholin-4-yl-2-trifluorométhyl-2,3-dihydro-1H-imidazo[1,2-a]pyrimidin-5-one (exemple 1j) et de 103 mg de 3-(bromométhyl)-5-chloro-1-benzothiophène, en remplaçant l'hydrure de sodium par le carbonate de césium. Après purification par HPLC / MS préparative (méthode C), on obtient 49 mg de la (2S)-1-[(5-chloro-1-benzothiophén-3-yl)méthyl]-2-méthyl-7-(morpholin-4-yl)-2-(trifluorométhyl)-2,3-dihydroimidazo[1,2-a]pyrimidin-5(1H)-one, sous forme d'un solide marron dont les caractéristiques sont les suivantes :
Spectre RMN 1 H (400MHz) :
   1,65 (s, 3 H) ; 3,31 à 3,36 (m, 4 H) ; 3,53 (m, 4 H) ; 3,98 (d, J=12,5 Hz, 1 H) ; 4,17 (d, J=12,5 Hz, 1 H) ; 4,82 (d, J=16,5 Hz, 1 H) ; 4,90 à 4,98 (m, 2 H) ; 7,41 (dd, J=2,0 et 8,6 Hz, 1 H) ; 7,79 (s, 1 H) ; 8,03 (d, J=8,6 Hz, 1 H) ; 8,16 (d, J=2,0 Hz, 1 H)
   Spectrométrie de Masse : méthode A
   Temps de rétention Tr (min) = 1,06
   [M+H]+ : m/z 485

### Exemple 21 : (2S)-2-méthyl-7-(morpholin-4-yl)-1-(phénylcarbonyl)-2-(trifluorométhyl)-2,3-dihydroimidazo[1,2-a]pyrimidin-5(1H)-one

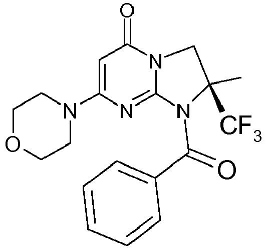

A une solution de 150 mg de la (2S)-2-méthyl-7-morpholin-4-yl-2-trifluorométhyl-2,3-dihydro-1H-imidazo[1,2-a]pyrimidin-5-one (exemple 1j), dans 3 mL de tétrahydrofuranne, sont ajoutés 14.2 mg d'hydrure de sodium. Après 25 minutes d'agitation à une température voisine de 20°C, on ajoute 0.092 mL de chlorure de benzoyle. Le mélange réactionnel est agité pendant 1 heure à température ambiante avant ajout de 1.5 mL d'une solution saturée en bicarbonate de sodium et de l'acétate d'éthyle. La phase organique est successivement séparée, lavée par une solution aqueuse saturée en chlorure de sodium, séchée sur sulfate de magnésium, filtrée, puis concentrée sous pression réduite. Après purification par chromatographie sur colonne de silice (éluant: dichlorométhane/méthanol: 98/02), on obtient 49 mg de la (2S)-2-méthyl-7-(morpholin-4-yl)-1-(phénylcarbonyl)-2-(trifluorométhyl)-2,3-dihydroimidazo[1,2-a]pyrimidin-5(1H)-one, sous forme d'un solide marron, dont les caractéristiques sont les suivantes :
Spectre RMN 1 H :
   1,96 (s, 3 H) ; 2,72 à 2,92 (m, 4 H) ; 3,24 à 3,36 (m
   partiellement masqué, 4 H) ; 4,12 (d, J=12,5 Hz, 1 H) ; 4,34 (d, J=12,5 Hz, 1 H) ; 5,01 (s, 1 H) ; 7,45 (t, J=7,6 Hz, 2 H) ; 7,53 (t, J=7,6 Hz, 1 H) ; 7,63 (d, J=7,6 Hz, 2 H)
   Spectrométrie de Masse : méthode B
   Temps de rétention Tr (min) = 3,76
   [M+H]+ : m/z 409

### Exemple 22: (2S)-1-[(1R ou 1S)-1-(3-fluorophényl)éthyl]-2-méthyl-7-(morpholin-4-yl)-2-(trifluorométhyl)-2,3-dihydroimidazo[1,2-a]pyrimidin-5(1H)-one

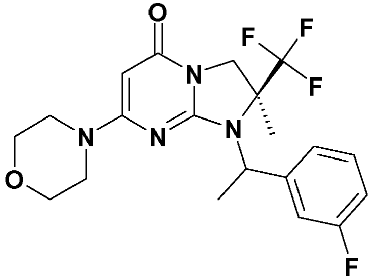

### Stade b :

La séparation des deux diastéréoisomères de la (2S)-1-[1-(3-fluorophényl)éthyl]-2-méthyl-7-(morpholin-4-yl)-2-(trifluorométhyl)-2,3-dihydroimidazo[1,2-a]pyrimidin-5(1H)-one a été réalisée par chromatographie chirale à partir de 66 mg d'un mélange 65/35 des deux diastéréosiomères : Phase stationnaire : Chiralpak AD 20µm 8*35cm ; phase mobile: Heptane 85% EtOH 15%

On obtient ainsi 21 mg de la (2S)-1-[(1R ou 1S)-1-(3-fluorophényl)éthyl]-2-méthyl-7-(morpholin-4-yl)-2-(trifluorométhyl)-2,3-dihydroimidazo[1,2-a]pyrimidin-5(1H)-one dont les caractéristiques sont les suivantes :
Spectre RMN 1 H (500MHz) :
   1,76 (s, 3 H) ; 1,80 (d, J=6,8 Hz, 3 H) ; 3,16 à 3,28 (m, 4 H) ; 3,41 à 3,55 (m, 4 H) ; 4,03 (d, J=12,7 Hz, 1 H) ; 4,17 (d, J=12,7 Hz, 1 H) ; 4,82 (s, 1 H) ; 4,90 (q, J=6,8 Hz, 1 H) ; 7,07 (dt, J=2,0 et 8,3 Hz, 1 H) ; 7,27 à 7,40 (m, 3 H)
   Spectrométrie de Masse : méthode B
   Temps de rétention Tr (min) = 4,07
   [M+H]+ : m/z 427

### Stade a : (2S)-1-[(1R et 1S)-1-(3-fluorophényl)éthyl]-2-méthyl-7-(morpholin-4-yl)-2-(trifluorométhyl)-2,3-dihydroimidazo[1,2-a]pyrimidin-5(1H)-one

Le produit peut être préparé en suivant le mode opératoire décrit au stade d de l'exemple 18 mais à partir de 300 mg de la (2S)-2-méthyl-7-(morpholin-4-yl)-2-(trifluorométhyl)-2,3-dihydroimidazo[1,2-a]pyrimidin-5(1H)-one, de 643 mg de carbonate de césium et de 234 mg de 1-(1-chloroéthyl)-3-fluorobenzène dans 13 mL d'acétonitrile. Après purification par chromatographie sur colonne de silice (éluant: dichlorométhane/méthanol: 97/03), on obtient 66 mg d'un mélange 65/35 des deux diastéréosimomères de la (2S)-1-[(1R et 1S)-1-(3-fluorophényl)éthyl]-2-méthyl-7-(morpholin-4-yl)-2-(trifluorométhyl)-2,3-dihydroimidazo[1,2-a]pyrimidin-5(1H)-one, sous la forme d'un résidu collant jaune pâle, dont les caractéristiques sont les suivantes :
Spectrométrie de Masse : méthode A
Temps de rétention Tr (min) = 0,59 et 0,67; mélange des distéréoisomères
[M+H]+ : m/z 427 ; [M-H]- : m/z 425

### Exemple 23 : -trifluoro acétate de (2S)-1-{[4-chloro-2-(trifluorométhyl)quinoléin-6-yl]méthyl}-2-méthyl-7-(morpholin-4-yl)-2-(trifluorométhyl)-2,3-dihydroimidazo[1,2-a]pyrimidin-5(1H)-one

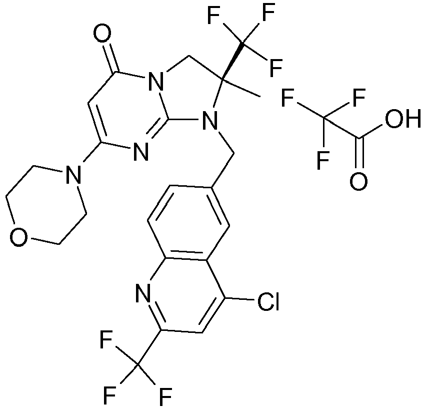

Le produit est préparé en suivant le mode opératoire décrit au stade k de l'exemple 1 à partir de 95 mg de la (2S)-2-méthyl-7-(morpholin-4-yl)-2-(trifluorométhyl)-2,3-dihydroimidazo[1,2-a]pyrimidin-5(1H)-one (exemple 1j) et de 101 mg de 6-(bromométhyl)-4-chloro-2-(trifluorométhyl)quinoléine, en remplaçant l'hydrure de sodium par 203 mg de carbonate de césium. Après purification par HPLC / MS préparative (méthode C), on obtient 40 mg de la (2S)-1-{[4-chloro-2-(trifluorométhyl)quinoléin-6-yl]méthyl}-2-méthyl-7-(morpholin-4-yl)-2-(trifluorométhyl)-2,3-dihydroimidazo[1,2-a]pyrimidin-5(1H)-one, sous forme d'un sel de l'acide trifluoroacétique et sous forme d'une poudre beige, dont les caractéristiques ont les suivantes :
Spectre RMN 1 H (400MHz):
   1,74 (s, 3 H) ; 3,26 à 3,31 (m, 4 H) ; 3,45 à 3,50 (m, 4 H) ; 4,03 (d, J=12,7 Hz, 1 H) ; 4,21 (d, J=12,7 Hz, 1 H) ; 4,90 (s, 1 H) ; 4,93 (s, 2 H) ; 8,03 (dd, J=2,0 et 8,8 Hz, 1 H) ; 8,25 (d, J=8,8 Hz, 1 H) ; 8,28 (s, 1 H) ; 8,36 (d, J=2,0 Hz, 1 H)
   Spectrométrie de Masse : méthode A
   Temps de rétention Tr (min) = 1,08
   [M+H]+ : m/z 548

### Exemple 24: (2S)-1-(3-bromo-4-fluorobenzyl)-2-méthyl-7-(morpholin-4-yl)-2-(trifluorométhyl)-2,3-dihydroimidazo[1,2-a]pyrimidin-5(1H)-one

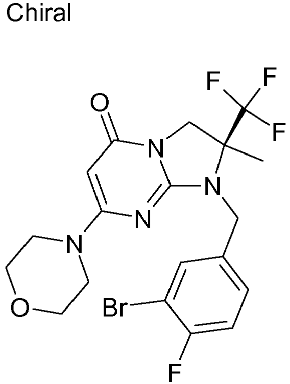

Le produit est préparé en suivant le mode opératoire décrit au stade k de l'exemple 1 à partir de 100 mg de la (2S)-2-méthyl-7-(morpholin-4-yl)-2-(trifluorométhyl)-2,3-dihydroimidazo[1,2-a]pyrimidin-5(1H)-one (exemple 1j) et de 106 mg de 2-bromo-4-(bromométhyl)-1-fluorobenzène, en remplaçant l'hydrure de sodium par 214 mg de carbonate de césium. Après purification par HPLC / MS préparative (méthode C), on obtient 50 mg de la (2S)-1-(3-bromo-4-fluorobenzyl)-2-méthyl-7-(morpholin-4-yl)-2-(trifluorométhyl)-2,3-dihydroimidazo[1,2-a]pyrimidin-5(1H)-one sous forme d'un semi-solide blanc cassé dont les caractéristiques ont les suivantes:
Spectre RMN 1 H (400MHz) :
   1,66 (s, 3 H) ; 3,29 à 3,41 (m, 4 H) ; 3,49 à 3,60 (m, 4 H) ; 3,98 (d, J=12,7 Hz, 1 H) ; 4,16 (d, J=12,7 Hz, 1 H) ; 4,60 (s, 2 H) ; 4,89 (s, 1 H) ; 7,33 (t, J=8,8 Hz, 1 H) ; 7,38 à 7,46 (m, 1 H) ; 7,76 (dd, J=2,0 et 6,8 Hz, 1 H)
   Spectrométrie de Masse : méthode A
   Temps de rétention Tr (min) = 1,00
   [M+H]+ : m/z 491

### Exemple 25 : (2S)-1-(2,3-difluorobenzyl)-2-méthyl-7-(morpholin-4-yl)-2-(trifluorométhyl)-2,3-dihydroimidazo[1,2-a]pyrimidin-5(1H)-one

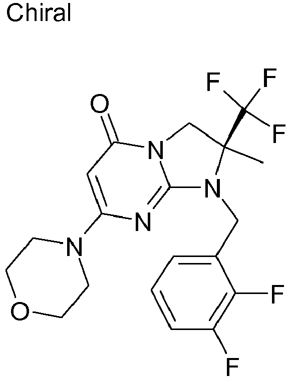

Le produit est préparé en suivant le mode opératoire décrit au stade k de l'exemple 1 à partir de 100 mg de la (2S)-2-méthyl-7-(morpholin-4-yl)-2-(trifluorométhyl)-2,3-dihydroimidazo[1,2-a]pyrimidin-5(1H)-one (exemple 1j) et de 82 mg de 1-(bromométhyl)-2,3-difluorobenzène, en remplaçant l'hydrure de sodium par 214 mg de carbonate de césium. Après purification par HPLC / MS préparative (Méthode C), on obtient 90 mg de la (2S)-1-(2,3-difluorobenzyl)-2-méthyl-7-(morpholin-4-yl)-2-(trifluorométhyl)-2,3-dihydroimidazo[1,2-a]pyrimidin-5(1H)-one, sous forme d'un semi-solide blanc, dont les caractéristiques ont les suivantes :
Spectre RMN 1 H (400MHz):
   1,67 (s, 3 H) ; 3,26 à 3,35 (m, 4 H) ; 3,49 à 3,58 (m, 4 H) ; 3,99 (d, J=12,7 Hz, 1 H) ; 4,18 (d, J=12,7 Hz, 1 H) ; 4,69 (s, 2 H) ; 4,89 (s, 1 H) ; 7,18 (m, 1 H) ; 7,25 (m, 1 H) ; 7,34 (m, 1 H)
   Spectrométrie de Masse : méthode A
   Temps de rétention Tr (min) = 0,94
   [M+H]+ : m/z 431

### Exemple 26 : (2S)-1-[2-(3-méthoxyphényl)éthyl]-2-méthyl-7-(morpholin-4-yl)-2-(trifluorométhyl)-2,3-dihydroimidazo[1,2-a]pyrimidin-5(1H)-one

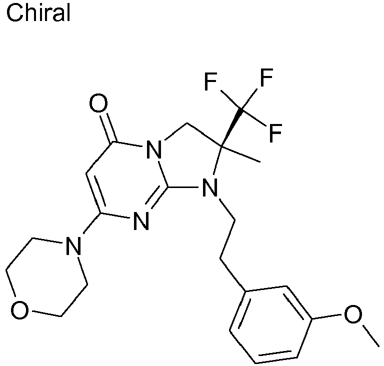

Le produit est préparé en suivant le mode opératoire décrit au stade k de l'exemple 1 à partir de 100 mg de la (2S)-2-méthyl-7-(morpholin-4-yl)-2-(trifluorométhyl)-2,3-dihydroimidazo[1,2-a]pyrimidin-5(1H)-one (exemple 1j) et de 85 mg de 1-(2-bromoéthyl)-3-méthoxybenzène, en remplaçant l'hydrure de sodium par 214 mg de carbonate de césium. Après purification par HPLC / MS préparative (Méthode C), on obtient 65 mg de la (2S)-1-[2-(3-méthoxyphényl)éthyl]-2-méthyl-7-(morpholin-4-yl)-2-(trifluorométhyl)-2,3-dihydroimidazo[1,2-a]pyrimidin-5(1H)-one, sous forme d'une huile, dont les caractéristiques ont les suivantes :
Spectre RMN 1 H (400MHz) :
   1,55 (s, 3 H) ; 2,82 (m, 1 H) ; 2,98 (m, 1 H) ; 3,39 à 3,52 (m, 5 H) ; 3,54 à 3,67 (m, 5 H) ; 3,73 (s, 3 H) ; 3,84 (d, J=12,7 Hz, 1 H) ; 4,12 (d, J=12,7 Hz, 1 H) ; 4,88 (s, 1 H) ; 6,79 (m, 3 H) ; 7,22 (m, 1 H)
   Spectrométrie de Masse : méthode A
   Temps de rétention Tr (min) = 0,96
   [M+H]+ : m/z 439

### Exemple 27 : (2S)-1-[2-(2-chlorophényl)éthyl]-2-méthyl-7-(morpholin-4-yl)-2-(trifluorométhyl)-2,3-dihydroimidazo[1,2-a]pyrimidin-5(1H)-one

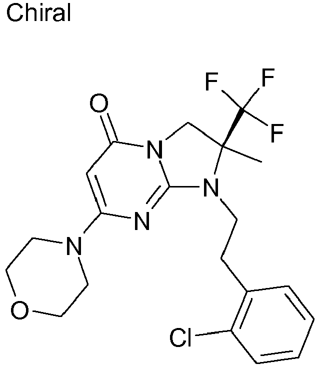

Le produit est préparé en suivant le mode opératoire décrit au stade k de l'exemple 1 à partir de 100 mg de la (2S)-2-méthyl-7-(morpholin-4-yl)-2-(trifluorométhyl)-2,3-dihydroimidazo[1,2-a]pyrimidin-5(1H)-one (exemple 1j) et de 87 mg de 1-(2-bromoéthyl)-2-chlorobenzène, en remplaçant l'hydrure de sodium par 214 mg de carbonate de césium. Après purification par HPLC / MS préparative (Méthode C), on obtient 40 mg de la (2S)-1-[2-(2-chlorophényl)éthyl]-2-méthyl-7-(morpholin-4-yl)-2-(trifluorométhyl)-2,3-dihydroimidazo[1,2-a]pyrimidin-5(1H)-one, sous forme d'un solide blanc cassé, dont les caractéristiques ont les suivantes :
Spectre RMN 1 H (400MHz):
   1,56 (s, 3 H) ; 3,02 (m, 1 H) ; 3,16 (m, 1 H) ; 3,31 à 3,53 (m partiellement masqué, 5 H) ; 3,57 à 3,67 (m, 5 H) ; 3,86 (d, J=12,5 Hz, 1 H) ; 4,12 (d, J=12,5 Hz, 1H) ; 4,87 (s, 1 H) ; 7,24 à 7,36 (m, 3 H) ; 7,41 à 7,47 (m, 1 H)
   Spectrométrie de Masse : méthode A
   Temps de rétention Tr (min) = 1,04
   [M+H]+ : m/z 443

### Exemple 28 : (2S)-1-[2-(4-chlorophényl)éthyl]-2-méthyl-7-(morpholin-4-yl)-2-(trifluorométhyl)-2,3-dihydroimidazo[1,2-a]pyrimidin-5(1H)-one

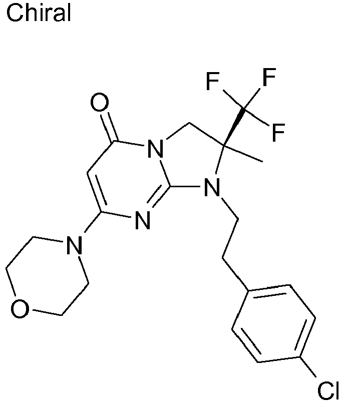

Le produit est préparé en suivant le mode opératoire décrit au stade k de l'exemple 1 à partir de 100 mg de la (2S)-2-méthyl-7-(morpholin-4-yl)-2-(trifluorométhyl)-2,3-dihydroimidazo[1,2-a]pyrimidin-5(1H)-one (exemple 1j) et de 87 mg de 1-(2-bromoéthyl)-4-chlorobenzène, en remplaçant l'hydrure de sodium par 214 mg de carbonate de césium. Après purification par HPLC / MS préparative (Méthode C) on obtient 65 mg de la (2S)-1-[2-(4-chlorophényl)éthyl]-2-méthyl-7-(morpholin-4-yl)-2-(trifluorométhyl)-2,3-dihydroimidazo[1,2-a]pyrimidin-5(1H)-one, sous forme d'une poudre blanche, dont les caractéristiques ont les suivantes :
Spectre RM N 1 H :
   1,53 (s, 3 H) ; 2,81 à 2,91 (m, 1 H) ; 2,95 à 3,06 (m, 1 H) ; 3,32 à 3,51 (m partiellement masqué, 5 H) ; 3,55 à 3,67 (m, 5 H) ; 3,85 (d, J=12,5 Hz, 1 H) ; 4,11 (d,J=12,5 Hz, 1 H) ; 4,87 (s, 1 H) ; 7,26 (d, J=8,3 Hz, 2 H) ; 7,36 (d, J=8,3 Hz, 2 H)
   Spectrométrie de Masse : méthode A
   Temps de rétention Tr (min) = 1,05
   [M+H]+ : m/z 443

### Exemple 29 : (2S)-1-[2-(3-chlorophényl)éthyl]-2-méthyl-7-(morpholin-4-yl)-2-(trifluorométhyl)-2,3-dihydroimidazo[1,2-a]pyrimidin-5(1H)-one

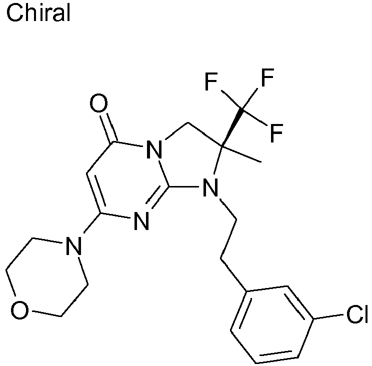

Le produit est préparé en suivant le mode opératoire décrit au stade k de l'exemple 1 à partir de 100 mg de la (2S)-2-méthyl-7-(morpholin-4-yl)-2-(trifluorométhyl)-2,3-dihydroimidazo[1,2-a]pyrimidin-5(1H)-one (exemple 1j) et de 87 mg de 1-(2-bromoéthyl)-3-chlorobenzène, en remplaçant l'hydrure de sodium par 214 mg de carbonate de césium. Après purification par HPLC / MS préparative (Méthode C), on obtient 38 mg de la (2S)-1-[2-(3-chlorophényl)éthyl]-2-méthyl-7-(morpholin-4-yl)-2-(trifluorométhyl)-2,3-dihydroimidazo[1,2-a]pyrimidin-5(1H)-one, sous forme d'une huile, dont les caractéristiques ont les suivantes :
Spectre RMN 1 H :
   1,56 (s, 3 H) ; 2,83 à 2,93 (m, 1 H) ; 2,96 à 3,05 (m, 1 H) ; 3,31 à 3,55 (m partiellement masqué, 5 H) ; 3,58 à 3,68 (m, 5 H) ; 3,85 (d, J=12,5 Hz, 1 H) ; 4,12 (d, J=12,5 Hz, 1 H) ; 4,88 (s, 1 H) ; 7,19 (d large, J=7,5 Hz, 1 H) ; 7,26 à 7,39 (m, 3 H)
   Spectrométrie de Masse : méthode A
   Temps de rétention Tr (min) = 1,04
   [M+H]+ : m/z 443

### Exemple 30 : (2S)-1-(1,3-benzoxazol-2-ylméthyl)-2-méthyl-7-(morpholin-4-yl)-2-(trifluorométhyl)-2,3-dihydroimidazo[1,2-a]pyrimidin-5(1H)-one

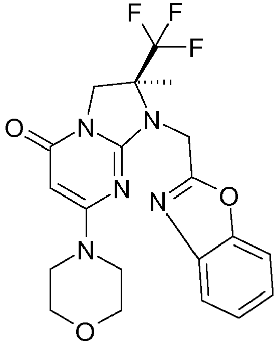

### Stade b :

Dans un four micro-ondes sont placés 210 mg de la (2S)-1-(1,3-benzoxazol-2-ylméthyl)-7-chloro-2-méthyl-2-(trifluorométhyl)-2,3-dihydroimidazo[1,2-a]pyrimidin-5(1H)-one dans 2 mL de morpholine. Après 18 min d'irradiation micro-ondes à une température de 85°C, le mélange réactionnel est dilué à l'acétate d'éthyle. Le mélange obtenu est lavé par de l'eau puis par une solution aqueuse saturée en chlorure de sodium avant d'être séché sur sulfate de magnésium anhydre, filtré et concentré à sec sous pression réduite. Après purification par chromatographie sur colonne de silice (éluant: dichlorométhane/méthanol: gradient de 0 à 50% de MeOH), on obtient 112 mg de la (2S)-1-(1,3-benzoxazol-2-ylméthyl)-2-méthyl-7-(morpholin-4-yl)-2-(trifluorométhyl)-2,3-dihydroimidazo[1,2-a]pyrimidin-5(1H)-one, sous forme d'une meringue jaune, dont les caractéristiques sont les suivantes :
Spectre RMN 1 H (400MHz):
   1,76 (s, 3 H) ; 3,17 à 3,24 (m, 4 H) ; 3,32 à 3,41 (m, 4 H) ; 4,02 (d, J=12,5 Hz, 1 H) ; 4,24 (d, J=12,5 Hz, 1 H) ; 4,85 (s, 1 H) ; 4,95 (s, 2 H) ; 7,32 à 7,42 (m, 2H) ; 7,66 à 7,75 (m, 2 H)
   Spectrométrie de Masse : méthode A
   Temps de rétention Tr (min) = 0,81
   [M+H]+ : m/z 436 ; [M-H]- : m/z 434

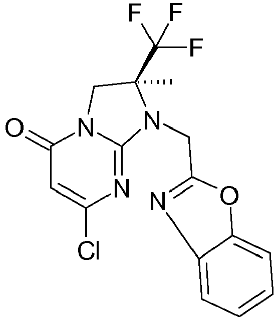

### Stade a : (2S)-1-(1,3-benzoxazol-2-ylméthyl)-2-méthyl-7-chloro-2-(trifluorométhyl)-2,3-dihydroimidazo[1,2-a]pyrimidin-5(1H)-one

Le produit est préparé en suivant le mode opératoire décrit au stade k de l'exemple 1 à partir de 160 mg de la (2S)-7-chloro-2-méthyl-2-trifluorométhyl-2,3-dihydro-1H-imidazo[1,2-a]pyrimidin-5-one (exemple 1 stade h'), 106 mg de 2-(chlorométhyl)-1,3-benzoxazole, en remplaçant l'hydrure de sodium par 360 mg de carbonate de césium. Après 15 heures de réaction à une température voisine de 20°C et traitement comme décrit à l'exemple 1k, on obtient 211 mg de (2S)-1-(1,3-benzoxazol-2-ylméthyl)-7-chloro-2-méthyl-2-(trifluorométhyl)-2,3-dihydroimidazo[1,2-a]pyrimidin-5(1H)-one, sous forme d'une meringue marron, dont les caractéristiques sont les suivantes :
Spectrométrie de Masse : méthode A ;
[M+H]+ : m/z 385
Temps de rétention Tr (min) = 1.30mn

### Exemple 31 : (2S)-2-méthyl-7-(morpholin-4-yl)-1-[(1R ou 1S)-1-phényléthyl]-2-(trifluorométhyl)-2,3-dihydroimidazo[1,2-a]pyrimidin-5(1H)-one

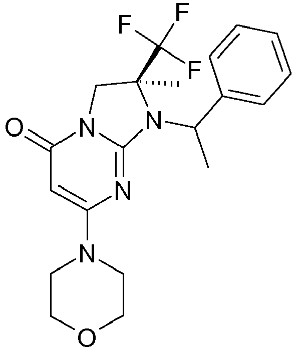

### Stade b :

La séparation des deux diastéréoisomères de la (2S)-2-méthyl-7-(morpholin-4-yl)-1-[(1R et 1S)-1-phényléthyl)-2-(trifluorométhyl)-2,3-dihydroimidazo[1,2-a]pyrimidin-5(1H)-one a été réalisée par chromatographie chirale à partir de 60 mg d'un mélange 65/35 des deux diastéréosiomères :
Phase stationnaire : Chiralpak AD 20µm 8*35cm
Phase mobile: Heptane (90%) EtOH (5%) MeOH (5%)

On obtient ainsi 16.4 mg de la (2S)-2-méthyl-7-(morpholin-4-yl)-1-[(1 R ou 1S)-1-phényléthyl]-2-(trifluorométhyl)-2,3-dihydroimidazo[1,2-a]pyrimidin-5(1 H)-one dont les caractéristiques sont les suivantes:
Spectre RMN 1 H (400MHz):
   1,76 (s, 3 H) ; 1,82 (d, J=6,8 Hz, 3 H) ; 3,12 à 3,25 (m, 4 H) ; 3,37 à 3,55 (m, 4 H) ; 3,99 (d, J=12,5 Hz, 1 H) ; 4,17 (d, J=12,5 Hz, 1 H) ; 4,79 (s, 1 H) ; 4,87 (q, J=6,8 Hz, 1 H) ; 7,17 à 7,27 (t, J=7,5 Hz, 1 H) ; 7,32 (t, J=7,5 Hz, 2 H) ; 7,46 (d, J=7,5 Hz, 2 H)
   Spectrométrie de Masse : Méthode B
   Temps de rétention Tr (min) = 4,04
   [M+H]+ : m/z 409
   Pouvoir rotatoire : PR= +16.6+/-0.7; c = 2.08mg/0.5ML DMSO.

### Stade a : (2S)-2-méthyl-7-(morpholin-4-yl)-1-((1R et 1S)-1-phényléthyl)-2-(trifluorométhyl)-2,3-dihydroimidazo[1,2-a]pyrimidin-5(1H)-one

Le produit peut être préparé comme décrit au stade d de l'exemple 18 mais à partir de 500 mg de la (2S)-2-méthyl-7-(morpholin-4-yl)-2-(trifluorométhyl)-2,3-dihydroimidazo[1,2-a]pyrimidin-5(1H)-one, de 1 g de carbonate de césium et de 346 mg de (1-chloroéthyl)benzène dans 25 mL d'acétonitrile. Après purification par chromatographie sur colonne de silice (éluant: CH₂Cl₂/MeOH: 97/03), on obtient 60 mg d'un mélange 65/35 des deux diastéréosiomères de la (2S)-2-méthyl-7-(morpholin-4-yl)-1-((1R et 1S)-1-phényléthyl)-2-(trifluorométhyl)-2,3-dihydroimidazo[1,2-a]pyrimidin-5(1H)-one, sous la forme d'une poudre orange, dont les caractéristiques sont les suivantes :
Spectrométrie de Masse : méthode B
Temps de rétention Tr (min) = 4,00 et 4,04; mélange d'isomères 2/3-1/3
[M+H]+ : m/z 409

### Exemple 32 : (2S)-2-méthyl-7-(morpholin-4-yl)-1-[(1R ou 1S)-1-phényléthyl]-2-(trifluorométhyl)-2,3-dihydroimidazo[1,2-a]pyrimidin-5(1H)-one

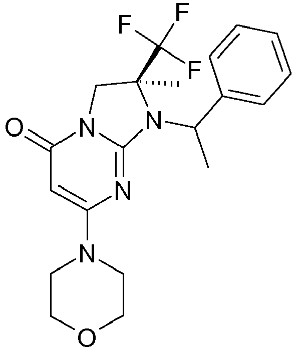

La séparation chirale décrite ci-dessus, au stade b de l'exemple 31, a aussi donné 27.9 mg du second diastéréoisomère de la (2S)-2-méthyl-7-(morpholin-4-yl)-1-[(1R et 1S)-1-phényléthyl)-2-(trifluorométhyl)-2,3-dihydroimidazo[1,2-a]pyrimidin-5(1H)-one, dont les caractéristiques sont les suivantes :
Spectre RMN 1 H (400MHz):
   1,76 (d, J=7,0 Hz, 3 H) ; 1,77 (s, 3 H) ; 3,17 à 3,25 (m, 4 H) ; 3,49 (m, 4 H) ; 3,96 (d, J=12,7 Hz, 1 H) ; 4,13 (d, J=12,7 Hz, 1 H) ; 4,85 (s, 1 H) ; 4,90 (q, J=7,0 Hz, 1 H) ; 7,20 (t, J=7,6 Hz, 1 H) ; 7,30 (t, J=7,6 Hz, 2 H) ; 7,42 (t, J=7,6 Hz, 2 H)
   Spectrométrie de Masse : méthode B
   Temps de rétention Tr (min) = 4,00
   [M+H]+ : m/z 409
   Pouvoir rotatoire : PR= -95.7+/-1.6; c = 951mg/0.5ML DMSO.

### Exemple 33 : (2S)-1-(1H-indol-3-ylméthyl)-2-méthyl-7-(morpholin-4-yl)-2-(trifluorométhyl)-2,3-dihydroimidazo[1,2-a]pyrimidin-5(1H)-one

### Stade c :

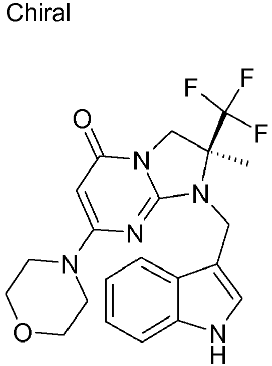

A une solution de 200 mg de 3-{[(2S)-2-méthyl-7-(morpholin-4-yl)-5-oxo-2-(trifluorométhyl)-2,3-dihydroimidazo[1,2-a]pyrimidin-1(5H)-yl]méthyl}-1 H-indole-1-carboxylate de 2-méthylpropan-2-yle dans 3 mL de chlorure de méthylène est ajouté 1 mL d'acide trifluoroacétique. Après une nuit à une température voisine de 20°C, le mélange réactionnel est concentré à sec sous pression réduite. Après purification par LC MS préparative (Méthode D), on concentre l'acétonitrile puis on extrait la phase aqueuse par de l'acétate d'éthyle. La phase organique est successivement lavée par une solution aqueuse saturée en bicarbonate de sodium, par deux fois de l'eau et une fois par une solution aqueuse saturée en chlorure de sodium. La phase organique résultante est séchée sur sulfate de magnésium anhydre, filtrée puis concentrée à sec, sous pression réduite. Le résidu est repris par de l'eau puis lyophilisé. On obtient ainsi 75 mg de (2S)-1-(1 H-indol-3-ylméthyl)-2-méthyl-7-(morpholin-4-yl)-2-(trifluorométhyl)-2,3-dihydroimidazo[1,2-a]pyrimidin-5(1H)-one, sous forme d'un lyophilisat de couleur rosée, dont les caractéristiques sont les suivantes :
Spectre RMN 1 H (400MHz):
   1,54 (s, 3 H) ; 3,41 à 3,48 (m, 4 H) ; 3,57 à 3,64 (m, 4 H) ; 3,87 (d, J=12,5 Hz, 1 H) ; 4,11 (d, J=12,5 Hz, 1 H) ; 4,65 (d, J=16,1 Hz, 1 H) ; 4,90 (s, 1 H) ; 4,96 (d, J=16,1 Hz, 1 H) ; 6,97 (dt, J=1,0 et 8,1 Hz, 1 H) ; 7,08 (dt, J=1,0 et 8,1 Hz, 1 H) ; 7,32 à 7,39 (m, 2 H) ; 7,65 (d large, J=8,1 Hz, 1 H) ; 10,97 (s large, 1 H)
   Spectrométrie de masse : Méthode A
   Temps de rétention Tr (min) = 0,91
   [M+H]+ : m/z 434 ;

### Stade b : 3-{[(2S)-2-méthyl-7-(morpholin-4-yl)-5-oxo-2-(trifluorométhyl)-2,3-dihydroimidazo[1,2-a]pyrimidin-1(5H)-yl]méthyl}-1H-indole-1-carboxylate de 2-méthylpropan-2-yle

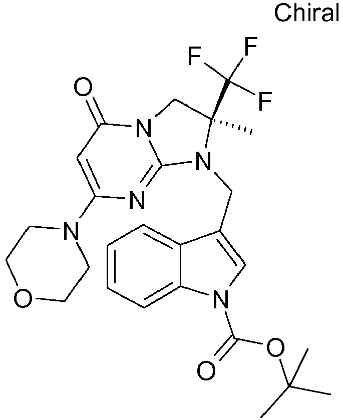

Le produit est préparé en suivant le mode opératoire décrit au stade b de l'exemple 30, mais à partir de 371 mg de 3-{[7-chloro-(2S)-2-méthyl-5-oxo-2-(trifluorométhyl)-2,3-dihydroimidazo[1,2-a]pyrimidin-1(5H)-yl]méthyl}-1H-indole-1-carboxylate de 2-méthylpropan-2-yle, dans 5 mL de morpholine. Après 20mn d'irradiation micro-ondes à une température de 90°C, et purification par chromatographie sur colonne de silice du mélange réactionnel (éluant: CH2Cl2/MeOH: gradient de 100/0 à 90/10), on obtient 200 mg de 3-{[(2S)-2-méthyl-7-(morpholin-4-yl)-5-oxo-2-(trifluorométhyl)-2,3-dihydroimidazo[1,2-a]pyrimidin-1(5H)-yl]méthyl}-1H-indole-1-carboxylate de 2-méthylpropan-2-yle qui est utilisé tel quel dans l'étape suivante.

### Stade a : 3-{[7-chloro-(2S)-2-méthyl-5-oxo-2-(trifluorométhyl)-2,3-dihydroimidazo[1,2-a]pyrimidin-1(5H)-yl]méthyl}-1H-indole-1-carboxylate de 2-méthylpropan-2-yle

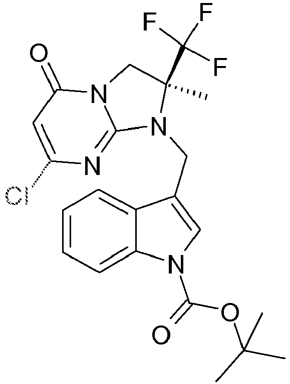

Le produit peut être préparé comme décrit au stade a de l'exemple 30 mais à partir de 204 mg 7-chloro-(2S)-2-méthyl-2-(trifluorométhyl)-2,3-dihydroimidazo[1,2-a]pyrimidin-5(1H)-one, de 250 mg de 3-(bromométhyl)-1 H-indole-1-carboxylate de 2-méthylpropan-2-yle et de 525 mg de carbonate de césium en remplaçant l'acétonitrile par la diméthylformamide. Après quatre jours d'agitation à une température voisine de 20°C, on obtient 370 mg d'un mélange contenant 3-{[7-chloro-(2S)-2-méthyl-5-oxo-2-(trifluorométhyl)-2,3-dihydroimidazo[1,2-a]pyrimidin-1(5H)-yl]méthyl}-1H-indole-1-carboxylate de 2-méthylpropan-2-yle qui est utilisé tel quel dans l'étape suivante.

### Exemple 34 : Synthèse de (2S)-1-[(2-chlorophényl)carbonyl]-2-méthyl-7-(morpholin-4-yl)-2-(trifluorométhyl)-2,3-dihydroimidazo[1,2-a]pyrimidin-5(1H)-one

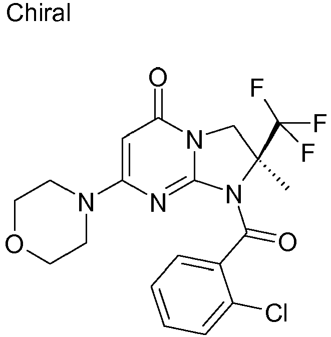

Le produit peut être préparé comme décrit à l'exemple 21 mais à partir de 200 mg de la (2S)-2-méthyl-7-(morpholin-4-yl)-2-(trifluorométhyl)-2,3-dihydroimidazo[1,2-a]pyrimidin-5(1H)-one, de 31 mg d'hydrure de sodium à 60% dans l'huile et de 115 mg de chlorure de 2-chlorobenzoyle dans 4 mL de tétrahydrofurane. Après purification par chromatographie sur colonne de silice (éluant : dichlorométhane/méthanol ; gradient de 100/0 à 97/03), on obtient 74 mg de la (2S)-1-[(2-chlorophényl)carbonyl]-2-méthyl-7-(morpholin-4-yl)-2-(trifluorométhyl)-2,3-dihydroimidazo[1,2-a]pyrimidin-5(1H)-one, sous forme d'une meringue ivoire ,dont les caractéristiques sont les suivantes :
Spectre RMN 1 H (400MHz):
   2,03 (s, 3 H) ; 2,70 à 2,93 (m, 4 H) ; 3,33 à 3,41 (m, 4 H) ; 4,07 (m étalé, 1 H) ; 4,35 (d, J=12,7 Hz, 1 H) ; 5,04 (s, 1 H) ; 7,40 à 7,58 (m, 4 H)
   Spectrométrie de masse : méthode A
   Temps de rétention Tr (min) = 0,89
   [M+H]+ : m/z 443

### Exemple 35 : (2S)-2-méthyl-1-[(2-méthylphényl)carbonyl]-7-(morpholin-4-yl)-2-(trifluorométhyl)-2,3-dihydroimidazo[1,2-a]pyrimidin-5(1H)-one

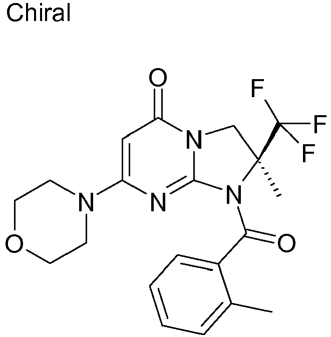

Le produit peut être préparé comme décrit à l'exemple 21 mais à partir de 200 mg de la (2S)-2-méthyl-7-(morpholin-4-yl)-2-(trifluorométhyl)-2,3-dihydroimidazo[1,2-a]pyrimidin-5(1H)-one, de 31 mg d'hydrure de sodium à 60% dans l'huile et de 101 mg de chlorure de 2-méthylbenzoyle dans 4 mL de tétrahydrofuranne. Après purification par chromatographie sur colonne de silice (éluant : CH2Cl2/MeOH ; gradient de 100/0 à 97/03), on obtient 44 mg de la (2S)-2-méthyl-1-[(2-méthylphényl)carbonyl]-7-(morpholin-4-yl)-2-(trifluorométhyl)-2,3-dihydroimidazo[1,2-a]pyrimidin-5(1H)-one, sous forme d'une meringue ivoire, dont les caractéristiques sont les suivantes :
Spectre RMN 1 H (400MHz):
   2,03 (s, 3 H) ; 2,24 (s, 3 H) ; 2,70 à 2,90 (m, 4 H) ; 3,23 à 3,41 (m partielllement masqué, 4 H) ; 4,04 (d, J=12,5 Hz, 1 H) ; 4,32 (d, J=12,5 Hz, 1 H) ; 5,00 (s, 1 H) ; 7,16 à 7,42 (m, 4 H)
   Spectrométrie de masse : Méthode B
   Temps de rétention Tr (min) = 3,92
   [M+H]+ : m/z 423

### Exemple 36: (2S)-1-[(1R ou 1S)-1-(2-fluorophényl)éthyl]-2-méthyl-7-(morpholin-4-yl)-2-(trifluorométhyl)-2,3-dihydroimidazo[1,2-a]pyrimidin-5(1H)-one

### Stade b :

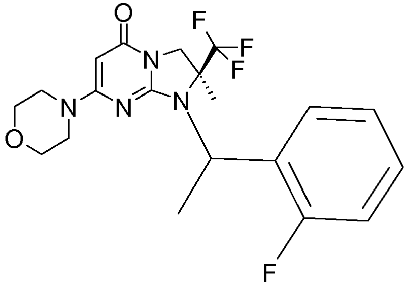

La séparation des deux diastéréoisomères de la (2S)-1-[(1R et 1S)-1-(2-fluorophényl)éthyl]-2-méthyl-7-(morpholin-4-yl)-2-(trifluorométhyl)-2,3-dihydroimidazo[1,2-a]pyrimidin-5(1H)-one a été réalisée par chromatographie chirale à partir de 89 mg d'un mélange des deux diastéréoisomères : Phase stationnaire : Chiralpak AD 20µm 8*35cm ; phase mobile: Heptane (85%) EtOH (15%).

On obtient ainsi 51.2 mg de la (2S)-1-[(1R ou 1S)-1-(2-fluorophényl)éthyl]-2-méthyl-7-(morpholin-4-yl)-2-(trifluorométhyl)-2,3-dihydroimidazo[1,2-a]pyrimidin-5(1H)-one, sous forme d'une meringue blanche, dont les caractéristiques sont les suivantes :
Spectre RMN 1 H (500MHz):
   1,73 (s, 3 H) ; 1,81 (d, J=7,1 Hz, 3 H) ; 3,33 à 3,37 (m, 4 H) ; 3,58 (t, J=4,9 Hz, 4 H) ; 3,96 (d, J=12,7 Hz, 1 H) ; 4,13 (d, J=12,7 Hz, 1 H) ; 4,95 (s, 1 H) ; 5,03 (q, J=7,1 Hz, 1 H) ; 7,11 à 7,19 (m, 2 H) ; 7,23 à 7,34 (m, 1 H) ; 7,67 à 7,76 (m, 1 H)
   Spectrométrie de Masse : méthode A
   Temps de rétention Tr (min) = 0,96
   [M+H]+ : m/z 427

### Stade a : (2S)-1-[(1R et 1S)-1-(2-fluorophényl)éthyl]-2-méthyl-7-(morpholin-4-yl)-2-(trifluorométhyl)-2,3-dihydroimidazo[1,2-a]pyrimidin-5(1H)-one

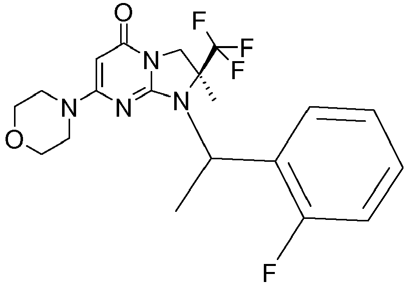

Le produit peut être préparé comme décrit au stade d de l'exemple 18 mais à partir de 300 mg de la (2S)-2-méthyl-7-(morpholin-4-yl)-2-(trifluorométhyl)-2,3-dihydroimidazo[1,2-a]pyrimidin-5(1H)-one, de 643 mg de carbonate de césium et de 234 mg de 1-(1-chloroéthyl)-2-fluorobenzène dans 13 mL d'acétonitrile. Après purification par chromatographie sur colonne de silice (éluant : dichlorométhane/méthanol : 97/03), on obtient 89 mg d'un mélange 35/65 des deux diastérosiomères de la (2S)-1-[(1R et 1S)-1-(2-fluorophényl)éthyl]-2-méthyl-7-(morpholin-4-yl)-2-(trifluorométhyl)-2,3-dihydroimidazo[1,2-a]pyrimidin-5(1H)-one, sous forme d'une poudre blanc-jaune, dont les caractéristiques sont les suivantes :
Spectrométrie de masse : Méthode A
Temps de rétention Tr (min) = 0,98 et 0,96 : mélange 35/65 des deux diastéréoisomères
[M+H]+ : m/z 427

### Exemple 37: (2S)-1-[(1R ou 1S)-1-(2-fluorophényl)éthyl]-2-méthyl-7-(morpholin-4-yl)-2-(trifluorométhyl)-2,3-dihydroimidazo[1,2-a]pyrimidin-5(1H)-one

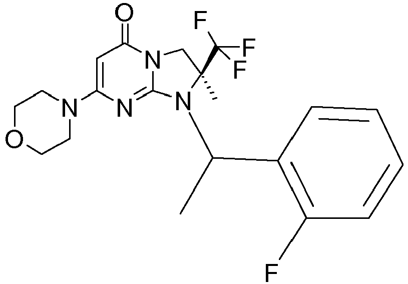

La séparation chirale décrite ci-dessus, au stade b de l'exemple 36, a aussi donné 26.8 mg du second diastéréoisomère de la (2S)-1-[(1 R et 1 S)-1-(2-fluorophényl)éthyl]-2-méthyl-7-(morpholin-4-yl)-2-(trifluorométhyl)-2,3-dihydroimidazo[1,2-a]pyrimidin-5(1H)-one, dont les caractéristiques sont les suivantes :
Spectre RMN 1 H (500MHz) :
   1,64 (s, 3 H) ; 1,84 (d, J=7,0 Hz, 3 H) ; 3,25 à 3,38 (m partiellement masqué, 4 H) ; 3,50 à 3,63 (m, 4 H) ; 3,91 (d, J=12,7 Hz, 1 H) ; 4,16 (d, J=12,7 Hz, 1 H) ; 4,86 (s, 1 H) ; 5,06 (q, J=7,0 Hz, 1 H) ; 7,14 à 7,22 (m, 2 H) ; 7,30 à 7,38 (m, 1 H) ; 7,71 (m, 1 H)
   Spectrométrie de masse : Méthode A
   Temps de rétention Tr (min) = 0,98
   [M+H]+ : m/z 427

### Exemple 38: Composition pharmaceutique

On a préparé des comprimés répondant à la formule suivante :
Produit de l'exemple 1....................... 0,2 g
Excipient pour un comprimé terminé à .......... 1 g
(détail de l'excipient : lactose, talc, amidon, stéarate de magnésium).

L'exemple 1 est pris à titre d'exemple de préparation pharmaceutique, cette préparation pouvant être réalisée si désiré avec d'autres produits de formule (I) selon la présente invention et notamment en exemples dans la présente demande, parmi les exemples 2 à 37 et 39 à 43

Les produits du tableau ci-dessous qui sont des produits de formule (I) tels que définis ci-dessus constituent les exemples 39 à 43 de la présente invention. Ces produits des exemples 39 à 43 sont préparés comme indiqué ci-dessus dans la partie expérimentale et sont caractérisés par les résultats physico-chimiques donnés dans ce tableau.

| Exemple | Nom | Spectrométrie de masse : Méthode E | |
|---|---|---|---|
| | | Tr (min) | [M+H]+: m/z |
| Exemple 39 | (S)-1-[2-(2-Fluoro-4,5-dimethoxy-phenyl)-2-hydroxy-ethyl]-2-methyl-7-morpholin-4-yl-2-trifluoromethyl-2,3-dihydro-1H-imidazo[1,2-a]pyrimidin-5-one | 0.69 | m/z 503 |
| Exemple 40 | (S)-1-[(S)-2-Hydroxy-2-(2-methoxy-phenyl)-ethyl]-2-methyl-7-morpholin-4-yl-2-trifluoromethyl-2,3-dihydro-1H-imidazo[1,2-a]pyrimidin-5-one | 0.74 | m/z 455 |
| Exemple 41 | (S)-1-[(S)-2-(4-Chloro-2-methoxy-phenyl)-2-hydroxy-ethyl]-2-methyl-7-morpholin-4-yl-2-trifluoromethyl-2,3-dihydro-1H-imidazo[1,2-a]pyrimidin-5-one | 0.80 | m/z 489 |
| Exemple 42 | (S)-1-[(S)-2-(4-Fluoro-2-methoxy-phenyl)-2-hydroxy-ethyl]-2-methyl-7-morpholin-4-yl-2-trifluoromethyl-2,3-dihydro-1H-imidazo[1,2-a]pyrimidin-5-one | 0.76 | m/z 473 |
| Exemple 43 | (S)-1-[(S)-2-(2-Chloro-4-methoxy-phenyl)-2-hydroxy-ethyl]-2-methyl-7-morpholin-4-yl-2-trifluoromethyl-2,3-dihydro-1H-imidazo[1,2-a]pyrimidin-5-one | 0.77 | m/z 489 |

### Partie pharmacologique :

### Protocoles expérimentaux

### Procédures expérimentales in vitro

L'activité inhibitrice des molécules sur la phosphorylation d'AKT est mesurée soit par western blotting par la technique décrite ci-dessous, soit par la technique MSD Multi-spot Biomarker detection de Meso Scale Discovery également décrite ci-dessous. Il a été démontré sur un set de molécules que les 2 techniques donnent des résultats compatibles.

### Etude de l'expression de pAKT dans les cellules humaines PC3 de carcinome de prostate mesurée par western blotting (Test A):

Cet essai est basé sur la mesure de l'expression de la protéine AKT phosphorylée sur la serine 473. La phosphorylation d'AKT (pAKT) est mesurée par western blotting dans la lignée de carcinome de prostate humaine PC3 (ATCC CRL-1435), en utilisant un anticorps reconnaissant spécifiquement pAKT-S473.

Le jour 1, les cellules PC3 sont ensemencées en plaques 6 puits (TPP, # 92006) à la concentration de 0.8x106 cellules/puits dans 1800 µl de milieu DMEM (DMEM Gibco #11960-044) contenant 10% de sérum de veau foetal (SVF Gibco, #10500-056) et 1% Glutamine (L-Glu Gibco #25030-024), et incubées à 37°C, 5% CO2, pendant une nuit.

Le jour 2, les cellules sont incubées en présence ou pas des produits à tester pendant 1 à 2 heures à 37°C en présence de 5% CO2. Les molécules diluées dans du diméthylsulfoxyde (DMSO Sigma #D2650), sont ajoutées à partir d'une solution mère concentrée 10 fois, le pourcentage final de DMSO étant de 0.1%. Les molécules sont testées soit à une seule concentration inférieure ou égale à 10µM, soit à des concentrations croissantes dans une gamme pouvant s'étendre de moins de 1 nM à 10µM.

Après cette incubation, les cellules sont lysées pour la préparation des protéines. Après aspiration du milieu de culture, les cellules sont rincées par 1 ml de PBS (DPBS Gibco, #14190-094), récupérées par grattage dans 200µl de tampon HNTG complet et transfert en plaque 96 puits (Greiner #651201), et lysées pendant 1 H sur glace. Le tampon HNTG est composé du mélange suivant :Hepes 50 mM, NaCl 150 mM, Triton 1%, Glycerol 10%, avec ajoût extemporané d'une pastille de Protease Inhibitor Cocktail Mini (Roche 1836153) et d'une pastille de Phosphatase Inhibitor Cocktail (Roche104906837001) pour 10ml de tampon.

Le lysat est centrifugé 10min à 6000RPM. 155µl de surnageant sont récupérés. 150 µl sont incubés pour dénaturation pendant 5min à 95°C en présence de tampon NuPAGE LDS Sample Buffer 4X dilué 4 fois (Ref InVitrogen NP0007) et de NuPAGE Sample Reducing Agent 10X dilué 10 fois (Ref InVitrogen NP0009). Ces échantillons sont ensuite congelés à -20°C. 5 µl sont dosées par la technique microBCA selon la fiche technique du MicroBCA Proteine Assay Kit (Pierce #23235).

Pour la séparation des protéines, 20µg de protéines sont déposées sur gel NU-PAGE 4-12% Bis Tris Gel 12 puits (Ref InVitrogen NP0322BOX) et la migration est effectuée pendant 1h30 en tampon de migration NU-PAGE MOPS SDS Running Buffer 20X dilué 20 fois (Ref InVitrogen NP0001), à 150 Volts.

Le gel est ensuite transféré sur une membrane Invitrolon PVDF (Invitrogen #LC2007) préalablement perméabilisée quelques secondes dans de l'éthanol (Ethanol Fischer Scientific #E/0600DF/15).

Le transfert s'effectue dans une cuve Biorad à 30 Volts pendant la nuit ou à 60 volts pendant 3 heures, en présence de tampon de transfert NUPAGE Transfer Buffer 20X dilué 20 fois (Ref InVitrogen NP0006).

La membrane est ensuite saturée en solution de saturation composé de TBS (Tris Buffer Saline 10x, Sigma #T5912 Sigma, dilué 10 fois), Tween 20 0.1% (#P5927 Sigma) et BSA 3% (Bovine Albumin Serum Fraction V, Sigma #A4503) pendant 6h après un transfert d'une durée de une nuit ou bien pendant 1 h après un transfert d'une durée de 3h.

Les anticorps primaires sont dilués au 1/1000e pour l'anticorps anti-phospho AKT-Ser473 (193H2, monoclonal de lapin, cat#4058 de chez Cell Signaling Technology) Abcam), en solution de saturation composée de PBS, Tween 20 0.1%, BSA 3%, puis mis sous agitation pendant la nuit à 4°C.

Deux rinçages de 5 min en solution de lavage composée de TBS, Tween 20 0.1% sont effectués avant l'hybridation des anticorps secondaires.

Les anticorps secondaires sont dilués au 1/10000e pour l'anticorps Rabbit anti-Mouse IgG HRP (W402 Promega) et au 1/10000e pour l'anticorps Goat anti-Rabbit IgG HRP (W401 Promega) en solution de saturation puis mis sous agitation pendant 1 h à température ambiante.

Deux rinçages de 30 min en solution de lavage sont effectués puis un rinçage de 5 min à l'H2O est effectué pour éliminer le Tween 20 restant.

La solution de révélation est préparée volume à volume selon la fiche technique du Western Lightning Chemiluminescence Reagent Plus (Western Lightning Chemiluminescence Reagent Plus Perkin Elmer #NEL104).

La membrane est placée pendant 1 min dans la solution de révélation, égouttée, insérée entre deux transparents puis placée dans l'appareil de mesure pour la lecture de la luminescence et la quantification du signal. La lecture de la luminescence est effectuée avec l'appareil FujiFilm (Ray Test).

L'appareil FUJI mesure le signal total de luminescence obtenu (AU) pour chaque bande sélectionnée. Puis il soustrait le bruit de fond (BG) proportionnel à la taille de la bande sélectionnée (Area), bruit de fond calculé à partir d'une bande de bruit de fond spécifique, en vue d'obtenir le signal spécifique (AU-BG) pour chaque bande. La bande obtenue en absence de produit et en présence de 0.1% DMSO est considérée comme le 100 % de signal. Le logiciel calcule le % d'activité spécifique (Ratio) obtenu pour chaque bande sélectionnée en fonction de ce 100 % de signal. Le calcul du pourcentage d'inhibition est fait pour chaque concentration selon la formule (100% - Ratio).

2 expériences indépendantes permettent de calculer la moyenne des pourcentages d'inhibition obtenus à une concentration donnée pour les produits testés uniquement à une concentration.

Le cas échéant, l'activité des produits est traduite en Cl50 approchée, obtenue à partir d'une courbe dose-réponse de différentes concentrations testées et représentant la dose donnant 50 % d'inhibition spécifique (Cl50 absolue). 2 expériences indépendantes permettent de calculer la moyenne des Cl50s.

### Etude de l'expression de pAKT dans les cellules humaines PC3 de carcinome de prostate mesurée par la technique MSD Multi-spot Biomarker Detection de Meso Scale Discovery (Test B):

Cet essai est basé sur la mesure de l'expression de la protéine AKT phosphorylée sur la sérine 473 (P-AKT-S473), dans la lignée de carcinome de prostate humaine PC3, par la technique basée sur un immuno-essai sandwich utilisant le kit MSD Multi-spot Biomarker Detection de Meso Scale Discovery : kits phospho-Akt (Ser473) whole cell lysate (#K151CAD) ou phospho-Akt (Ser473)/Total Akt whole cell lysate (#K151OOD). L'anticorps primaire spécifique de P-AKT-S473 (Kit #K151CAD) est coaté sur une électrode dans chaque puits des plaques 96 puits du kit MSD : après ajoût d'un lysat de protéines dans chaque puits, la révélation du signal se fait par l'addition d'un anticorps secondaire de détection marqué avec un composé électrochimioluminescent. La procédure suivie est celle décrite dans le kit.

Le jour 1, les cellules PC3 sont ensemencées en plaques 96 puits (TPP, #92096) à la concentration de 35000 cellules/puits dans 200 µl de milieu DMEM (DMEM Gibco #11960-044) contenant 10% de sérum de veau foetal (SVF Gibco, #10500-056) et 1% Glutamine (L-Glu Gibco #25030-024), et incubées à 37°C, 5% CO2, pendant une nuit.

Le jour 2, les cellules sont incubées en présence ou pas des produits à tester pendant 1 à 2h à 37°C en présence de 5% CO2. Les molécules diluées dans du diméthylsulfoxyde (DMSO Sigma #D2650), sont ajoutées à partir d'une solution mère concentrée 20 fois, le pourcentage final de DMSO étant de 0.1%. Les molécules sont testées soit à une seule concentration inférieure ou égale à 10µM, soit à des concentrations croissantes dans une gamme pouvant s'étendre de moins de 1 nM à 10µM.

Après cette incubation, les cellules sont lysées pour la préparation des protéines. Pour cela, après aspiration du milieu de culture, 50µl de tampon de lyse Tris Lysis Buffer complet du kit MSD contenant les solutions d'inhibiteurs de protéases et phosphatases sont ajoutés dans les puits et les cellules sont lysées pendant 1 H à 4°C sous agitation. A ce stade les plaques contenant les lysats peuvent être congelées à -20°C ou à -80°C.

Les puits des plaques 96 puits du kit MSD sont saturés pendant 1 h à température ambiante avec la solution bloquante du kit MSD. Quatre lavages sont effectués avec 150µl de solution de lavage Tris Wash Buffer du kit MSD. Les lysats préparés précédemment sont transférés dans les plaques Multi-spot 96 puits du kit MSD et incubés pendant 1 h à température ambiante, sous agitation. Quatre lavages sont effectués avec 150µl de solution de lavage Tris Wash Buffer du kit MSD. 25µl de la solution MSD sulfo-tag detection antibody sont ajoutés dans les puits et incubés pendant 1 h à température ambiante, sous agitation. Quatre lavages sont effectués avec 150µl de solution de lavage Tris Wash Buffer du kit MSD. 150µl de tampon de révélation Read Buffer du kit MSD sont ajoutés dans les puits et les plaques sont lues immédiatement sur l'instrument S12400 de Meso Scale Discovery.

L'appareil mesure un signal pour chaque puits. Des puits sans cellules et contenant le tampon de lyse servent à déterminer le bruit de fond qui sera soustrait à toutes les mesures (min). Les puits contenant des cellules en absence de produit et en présence de 0.1% DMSO sont considérés comme le 100 % de signal (max). Le calcul du pourcentage d'inhibition est fait pour chaque concentration de produit testé selon la formule suivante : (1- ((essai-min)/(max-min)))x100.

L'activité du produit est traduite en Cl₅₀, obtenue à partir d'une courbe dose-réponse de différentes concentrations testées et représentant la dose donnant 50 % d'inhibition spécifique (Cl₅₀ absolue). 2 expériences indépendantes permettent de calculer la moyenne des Cl₅₀ₛ.

L'activité inhibitrice des molécules sur l'autophagie est mesurée par la translocation de la protéine LC3 du cytoplasme vers les autophagososmes. Pour cela les cellules Hela ont été transfectées avec un vecteur codant pour la protéine chimérique GFP-LC3. Un clone Hela exprimant la protéine GFP-LC3 de manière stable a été sélectionné. La translocation de la protéine LC3 est déterminée en mesurant le nombre de cellules présentant des granulations de LC3 après un stress métabolique, à l'aide d'un cytomètre d'analyse automatique d'images iCyte (Compucyte)

### Etude de la translocation de la protéine LC3 dans les cellules humaines Hela mesurée parcytomètre d'analyse d'images (Test C):

Le jour 1, les cellules Hela GFP-LC3 sont ensemencées en plaques 96 puits coatées poly D lysine (Greiner, #655946) à la concentration de 15000 cellules/puits dans 200 µl de milieu DMEM (DMEM Gibco #11960-044) contenant 10% de sérum de veau foetal (SVF Gibco, #10500-056) et 1% Glutamine (L-Glu Gibco #25030-024), et incubées à 37°C, 5% CO2, pendant une nuit.

Le jour 2, les cellules sont lavées deux fois avec de l'EBSS (Sigma#E3024).. Les cellules sont ensuite incubées dans de l'EBSS, 10µM d'hydroxychloroquine et des produits à tester pendant 2h à 37°C en présence de 5% CO2. Les molécules sont diluées dans du diméthylsulfoxyde (DMSO Sigma #D2650). Le pourcentage final de DMSO étant de 0.1%. Les molécules sont testées à des concentrations croissantes dans une gamme pouvant s'étendre de 10 nM à 1µM.

Après cette incubation, les cellules sont fixées avec 4% paraformaldéhyde (Sigma#HT501128 4L) pendant 10min. Les cellules sont ensuite lavées 2 fois avec du PBS puis les noyaux colorées avec 2µg/ml de Hoechst 33342 (Invitrogen#H3570) Les plaques 96 puits sont ensuite lues avec le cytomètre analyse d'image iCyte (Compucyte). L'analyseur quantifie le nombre de cellules présentant des granulations de LC3. Une cellule est considérée comme positive lorsqu'elle présente au moins 4 granulations de LC3. Le pourcentage de cellules présentant plus de 4 granulations est calculé par rapport au nombre total de cellules.

L'activité du produit est traduite en Cl50, obtenue à partir d'une courbe dose-réponse de différentes concentrations testées et représentant la dose donnant 50 % d'inhibition spécifique (Cl50 absolue). 2 expériences indépendantes permettent de calculer la moyenne des Cl50s.

Les résultats obtenus pour les produits en exemples dans la partie expérimentale sont donnés dans le tableau de résultats pharmacologiques ci-après:

**Tableau 1 de résultats pharmacologiques :**

| exemple | Test A* | Test B* | Test C* |
|---|---|---|---|
| Exemple 1 | 100 | 3 | 44 |
| Exemple 2 | 324 | | 145 |
| Exemple 3 | 634 | | 493 |
| Exemple 4 | 25 | | 107 |
| Exemple 5 | 30 | | 73 |
| Exemple 6 | 1644 | | 81 |
| Exemple 7 | | | 71 |
| Exemple 8 | 15 | | 13 |
| Exemple 9 | | | 191 |
| Exemple 10 | 16 | | 31 |
| Exemple 11 | | | 235 |
| Exemple 12 | 77 | | 194 |
| Exemple 13 | | | 822 |
| Exemple 14 | 174 | | 10000 |
| Exemple 15 | 121 | | 72 |
| Exemple 16 | 79 | | |
| Exemple 17 | | | 345 |
| Exemple 18 | | | 39 |
| Exemple 19 | | 26 | 425 |
| Exemple 20 | | 18 | 703 |
| Exemple 21 | | 7 | >1000 |
| Exemple 22 | | 59 | 872 |
| Exemple 23 | | | 2116 |
| Exemple 24 | | 13 | 314 |
| Exemple 25 | | 28 | 621 |
| Exemple 26 | | 16 | |
| Exemple 27 | | 24 | 51 |
| Exemple 28 | | 10 | 198 |
| Exemple 29 | | 6 | 261 |
| Exemple 30 | | 89 | >1000 |
| Exemple 31 | | 60 | 334 |
| Exemple 32 | | 22 | >1000 |
| Exemple 33 | | 42 | 461 |
| Exemple 34 | | 159 | 1000 |
| Exemple 35 | | 32 | >1000 |
| Exemple 36 | | 4 | 995 |
| Exemple 37 | | 19 | 737 |

| | | | |
|---|---|---|---|
| * Tests A ,B et C : Cl50 (nM) | | | |

### Test d'activité anti-paludique

Les tests d'activité antipaludique sont effectués selon la micro méthode radioactive de Desjardins (R.E. Desjardins, C.J. Canfield, J.D. Haynes, J.D. Chulay, Antimicrob. Agents Chemother., 1979, 16, 710-718). Les essais sont réalisés dans des microplaques de 96 puits (Test Plates Réf. 92696, Techno Plastic Products Ag, Zollstrasse 155, CH-8219 Trasadingen). Les souches de P. falciparum sont mises en culture dans des solutions de RPMI 1640 complémenté avec 5 % de sérum humain avec un hématocrite à 2 % et une parasitémie à 1,5 %. Pour chaque essai, les parasites sont incubés avec des concentrations choisies de drogues pendant 48 h à 37 °C en atmosphère humide et à 5 % de CO2. L'artémisinine, l'artésunate ainsi que la chloroquine di-phosphate sont utilisées comme molécules référence. La première dilution de la drogue est réalisée à 1 mg/mL dans du diméthylsulfoxyde. La gamme de dilutions des solutions filles successives est également réalisée dans du diméthylsulfoxyde. Chaque dilution fille est ensuite diluée au 1/50 ème dans du RPMI 1640 complémenté avec 5 % de sérum humain, l'ensemble des dilutions étant réalisé à 37 °C. Ces dilutions sont ensuite ajoutées aux parasites en culture dans les microplaques. Après ajout de la drogue, les parasites sont en culture dans du RPMI 1640 à 5 % de sérum humain et à 1 % de diméthylsulfoxyde. La croissance des parasites est mesurée par l'incorporation d'hypoxanthine tritiée (ajoutée 24 h après le début de l'exposition à la drogue) comparée à l'incorporation en l'absence de drogue.

L'activité du produit est traduite en % inhibition de la croissance de P. falciparum (hautement résistant à la chloroquine souche Fcm29-Cameroun) à 1 uM et 0,1 uM dans un test in vitro utilisant des érythrocytes humains infectés.

Les résultats obtenus pour les produits en exemples dans la partie expérimentale sont donnés dans le tableau 2 de résultats pharmacologiques ci-dessous :

**- Tableau 2 de résultats pharmacologiques :**

| Exemple | P. falciparum % Inhibition 1 µM | P. falciparum % Inhibition 0.1 µM |
|---|---|---|
| Exemple 1 | 93 | 67 |
| Exemple 3 | 95 | 15 |
| Exemple 4 | 97 | 54 |
| Exemple 10 | 97 | 38 |
| Exemple 11 | 13 | / |
| Exemple 15 | 101 | 39 |
| Exemple 26 | 75 | 14 |
| Exemple 27 | 53 | / |
| Exemple 28 | 53 | 14 |
| Exemple 29 | 52 | / |
| Exemple 39 | 85 | 39 |
| Exemple 40 | 80 | 20 |
| Exemple 41 | 78 | / |
| Exemple 42 | 78 | / |
| Exemple 43 | 74 | 22 |

## Revendications

1. Produits de formule (I): 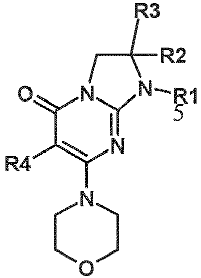
dans laquelle :
R1 représente un radical -L-aryle ou -L-hétéroaryle, tel que L représente :
soit un radical alkyle linéaire ou ramifié renfermant de 1 à 6 atomes de carbone et éventuellement substitué par un radical hydroxyle,
soit un groupe CO,
soit un groupe L'-X où L' représente un radical alkyle linéaire ou ramifié renfermant de 1 à 6 atomes de carbone et X un atome d'oxygène ou de soufre ;
les radicaux aryle et hétéroaryle étant éventuellement substitués par un ou plusieurs radicaux identiques ou différents choisis parmi les atomes d'halogène et les radicaux hydroxyle, CN, nitro, -COOH, -COOalk, -NRxRy, - CONRxRy, -NRxCORy, -NRxCO2Rz, -CORy, alcoxy, phénoxy, alkylthio, alkyle, cycloalkyle et hétérocycloalkyle ;
ces derniers radicaux alcoxy, phénoxy, alkylthio, alkyle et hétérocycloalkyle, étant eux-mêmes éventuellement substitués par un ou plusieurs radicaux identiques ou différents choisis parmi les atomes d'halogène et NRvRw ;
les radicaux hétérocycloalkyle et hétéroaryle pouvant de plus renfermer un radical oxo ;
R2 représente un atome d'hydrogène ou un radical alkyle ;
R3 représente un radical alkyle éventuellement substitué par un ou plusieurs atomes d'halogène ;
R4 représente un atome d'hydrogène ou un atome d'halogène ;
NRxRy étant tel que Rx représente un atome d'hydrogène ou un radical alkyle et Ry représente un atome d'hydrogène, un radical cycloalkyle ou un radical alkyle éventuellement substitué par un ou plusieurs radicaux identiques ou différents choisis parmi les radicaux hydroxyle, alcoxy, NRvRw et hétérocycloalkyle ; soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
NRvRw étant tel que Rv représente un atome d'hydrogène ou un radical alkyle et Rw représente un atome d'hydrogène, un radical cycloalkyle ou un radical alkyle éventuellement substitué par un ou plusieurs radicaux identiques ou différents choisis parmi les radicaux hydroxyle, alcoxy, hétérocycloalkyle ; soit Rv et Rw forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
les radicaux cycliques que peuvent former Rx et Ry ou Rv et Rw respectivement avec l'atome d'azote auquel ils sont liés, étant éventuellement substitués par un ou plusieurs radicaux identiques ou différents choisis parmi les atomes d'halogène, les radicaux alkyle, hydroxyle, oxo, alcoxy, NH2; NHalk et N(alk)2 ;
Rz représente les valeurs de Ry à l'exception de hydrogène ;
Rx, Ry et Rz dans les radicaux -NRxCORy, -CORy et NRxCO₂Rz étant choisis parmi les significations indiquées ci-dessus pour Rx, Ry, et Rz ;
tous les radicaux alkyle (alk), alcoxy et alkylthio ci-dessus étant linéaires ou ramifiés et renfermant de 1 à 6 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

2. Produits de formule (I) tels que définis à la revendication 1 dans laquelle :
R1 représente un radical -L-phényle ou -L-hétéroaryle, tel que L représente :
soit un radical alkyle linéaire ou ramifié renfermant de 1 à 6 atomes de carbone et éventuellement substitué par un radical hydroxyle,
soit un groupe CO,
soit un groupe L'-X où L' représente un radical alkyle linéaire ou ramifié renfermant de 1 à 6 atomes de carbone et X un atome d'oxygène ou de soufre ;
les radicaux phényle et hétéroaryle étant éventuellement substitués par un ou plusieurs radicaux identiques ou différents choisis parmi les atomes d'halogène et les radicaux -NRxRy, alcoxy et alkyle ;
ces derniers radicaux alcoxy et alkyle étant eux-mêmes éventuellement substitués par un ou plusieurs radicaux choisis parmi les atomes d'halogène ;
R2 représente un radical alkyle ;
R3 représente un radical alkyle éventuellement substitué par un ou plusieurs atomes d'halogène ;
R4 représente un atome d'hydrogène ou un atome de fluor ;
NRxRy étant tel que Rx représente un atome d'hydrogène ou un radical alkyle et Ry représente un atome d'hydrogène ou un radical alkyle; soit Rx et Ry forment avec l'atome d'azote auquel ils sont liés un radical morpholino ;
tous les radicaux alkyle (alk) ou alcoxy ci-dessus étant linéaires ou ramifiés et renfermant de 1 à 6 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

3. Produits de formule (I) tels que définis à l'une quelconque des revendications 1 ou 2, répondant aux formules suivantes :
- (2S)-1-[2-(4-méthoxyphényl)éthyl]-2-méthyl-7-(morpholin-4-yl)-2-(trifluorométhyl)-2,3-dihydroimidazo[1,2-a]pyrimidin-5(1H)-one
- 1-[2-(4-méthoxyphényl)éthyl]-2-méthyl-7-(morpholin-4-yl)-2-(trifluorométhyl)-2,3-dihydroimidazo[1,2-a]pyrimidin-5(1H)-one
- (2S)-1-benzyl-2-méthyl-7-(morpholin-4-yl)-2-(trifluorométhyl)-2,3-dihydroimidazo[1,2-a]pyrimidin-5(1H)-one
- (2S)-2-méthyl-7-(morpholin-4-yl)-1-(2-phényléthyl)-2-(trifluorométhyl)-2,3-dihydroimidazo[1,2-a]pyrimidin-5(1H)-one
- (2S)-2-méthyl-7-(morpholin-4-yl)-1-(3-phénylpropyl)-2-(trifluorométhyl)-2,3-dihydroimidazo[1,2-a]pyrimidin-5(1H)-one
- (2S)-2-méthyl-7-(morpholin-4-yl)-1-(2-phénoxyéthyl)-2-(trifluorométhyl)-2,3-dihydroimidazo[1,2-a]pyrimidin-5(1H)-one
- (2S)-2-méthyl-7-(morpholin-4-yl)-1-[2-(phénylsulfanyl)éthyl]-2-(trifluorométhyl)-2,3-dihydroimidazo[1,2-a]pyrimidin-5(1H)-one
- (2S)-2-méthyl-7-(morpholin-4-yl)-1-[(2R)-2-phénylpropyl]-2-(trifluorométhyl)-2,3-dihydroimidazo[1,2-a]pyrimidin-5(1H)-one
- (2S)-2-méthyl-7-(morpholin-4-yl)-1-[(2S)-2-phénylpropyl]-2-(trifluorométhyl)-2,3-dihydroimidazo[1,2-a]pyrimidin-5(1H)-one
- (2S)-1-[(2S)-2-hydroxy-2-phényléthyl]-2-méthyl-7-(morpholin-4-yl)-2-(trifluorométhyl)-2,3-dihydroimidazo[1,2-a]pyrimidin-5(1H)-one
- (2S)-1-[(2R)-2-hydroxy-2-phényléthyl]-2-méthyl-7-(morpholin-4-yl)-2-(trifluorométhyl)-2,3-dihydroimidazo[1,2-a]pyrimidin-5(1H)-one
- (2S)-2-méthyl-7-(morpholin-4-yl)-1-[(2S)-1-phénylpropan-2-yl]-2-(trifluorométhyl)-2,3-dihydroimidazo[1,2-a]pyrimidin-5(1H)-one
- (2S)-2-méthyl-7-(morpholin-4-yl)-1-[(1S)-1-phénylpropyl]-2-(trifluorométhyl)-2,3-dihydroimidazo[1,2-a]pyrimidin-5(1H)-one
- (2S)-2-méthyl-7-(morpholin-4-yl)-1-[(1R)-1-phénylpropyl]-2-(trifluorométhyl)-2,3-dihydroimidazo[1,2-a]pyrimidin-5(1H)-one
- (2S)-2-méthyl-7-(morpholin-4-yl)-1-{2-[4-(morpholin-4-yl)phényl]éthyl}-2-(trifluorométhyl)-2,3-dihydroimidazo[1,2-a]pyrimidin-5(1H)-one
- (2S)-2-méthyl-7-(morpholin-4-yl)-1-(1-phényléthyl)-2-(trifluorométhyl)-2,3-dihydroimidazo[1,2-a]pyrimidin-5(1H)-one
- 1-[2-(4-méthoxyphényl)éthyl]-2,2-diméthyl-7-(morpholin-4-yl)-2,3-dihydroimidazo[1,2-a]pyrimidin-5(1H)-one
- (2S)-6-fluoro-1-[2-(4-méthoxyphényl)éthyl]-2-méthyl-7-(morpholin-4-yl)-2-(trifluorométhyl)-2,3-dihydroimidazo[1,2-a]pyrimidin-5(1H)-one
- (2S)-1-benzyl-6-fluoro-2-méthyl-7-(morpholin-4-yl)-2-(trifluorométhyl)-2,3-dihydroimidazo[1,2-a]pyrimidin-5(1H)-one
- (2S)-1-[(5-chloro-1-benzothiophén-3-yl)méthyl]-2-méthyl-7-(morpholin-4-yl)-2-(trifluorométhyl)-2,3-dihydroimidazo[1,2-a]pyrimidin-5(1H)-one
-(2S)-2-méthyl-7-(morpholin-4-yl)-1-(phénylcarbonyl)-2-(trifluorométhyl)-2,3-dihydroimidazo[1,2-a]pyrimidin-5(1H)-one
- (2S)-1-[(1R ou 1S)-1-(3-fluorophényl)éthyl]-2-méthyl-7-(morpholin-4-yl)-2-(trifluorométhyl)-2,3-dihydroimidazo[1,2-a]pyrimidin-5(1H)-one
-trifluoro acétate de (2S)-1-{[4-chloro-2-(trifluorométhyl)quinoléin-6-yl]méthyl}-2-méthyl-7-(morpholin-4-yl)-2-(trifluorométhyl)-2,3-dihydroimidazo[1,2-a]pyrimidin-5(1H)-one
-(2S)-1-(3-bromo-4-fluorobenzyl)-2-méthyl-7-(morpholin-4-yl)-2-(trifluorométhyl)-2,3-dihydroimidazo[1,2-a]pyrimidin-5(1H)-one
- (2S)-1-(2,3-difluorobenzyl)-2-méthyl-7-(morpholin-4-yl)-2-(trifluorométhyl)-2,3-dihydroimidazo[1,2-a]pyrimidin-5(1H)-one
-(2S)-1-[2-(3-méthoxyphényl)éthyl]-2-méthyl-7-(morpholin-4-yl)-2-(trifluorométhyl)-2,3-dihydroimidazo[1,2-a]pyrimidin-5(1H)-one
- (2S)-1-[2-(2-chlorophényl)éthyl]-2-méthyl-7-(morpholin-4-yl)-2-(trifluorométhyl)-2,3-dihydroimidazo[1,2-a]pyrimidin-5(1H)-one
- (2S)-1-[2-(4-chlorophényl)éthyl]-2-méthyl-7-(morpholin-4-yl)-2-(trifluorométhyl)-2,3-dihydroimidazo[1,2-a]pyrimidin-5(1H)-one
- (2S)-1-[2-(3-chlorophényl)éthyl]-2-méthyl-7-(morpholin-4-yl)-2-(trifluorométhyl)-2,3-dihydroimidazo[1,2-a]pyrimidin-5(1H)-one
- (2S)-1-(1,3-benzoxazol-2-ylméthyl)-2-méthyl-7-(morpholin-4-yl)-2-(trifluorométhyl)-2,3-dihydroimidazo[1,2-a]pyrimidin-5(1H)-one
- (2S)-2-méthyl-7-(morpholin-4-yl)-1-[(1R ou 1S)-1-phényléthyl]-2-(trifluorométhyl)-2,3-dihydroimidazo[1,2-a]pyrimidin-5(1H)-one
- (2S)-2-méthyl-7-(morpholin-4-yl)-1-[(1R ou 1S)-1-phényléthyl]-2-(trifluorométhyl)-2,3-dihydroimidazo[1,2-a]pyrimidin-5(1H)-one
- (2S)-1-(1H-indol-3-ylméthyl)-2-méthyl-7-(morpholin-4-yl)-2-(trifluorométhyl)-2,3-dihydroimidazo[1,2-a]pyrimidin-5(1H)-one
- (2S)-1-[(2-chlorophényl)carbonyl]-2-méthyl-7-(morpholin-4-yl)-2-(trifluorométhyl)-2,3-dihydroimidazo[1,2-a]pyrimidin-5(1H)-one
- (2S)-2-méthyl-1-[(2-méthylphényl)carbonyl]-7-(morpholin-4-yl)-2-(trifluorométhyl)-2,3-dihydroimidazo[1,2-a]pyrimidin-5(1H)-one
- (2S)-1-[(1 R ou 1S)-1-(2-fluorophényl)éthyl]-2-méthyl-7-(morpholin-4-yl)-2-(trifluorométhyl)-2,3-dihydroimidazo[1,2-a]pyrimidin-5(1H)-one
- (2S)-1-[(1 R ou 1S)-1-(2-fluorophényl)éthyl]-2-méthyl-7-(morpholin-4-yl)-2-(trifluorométhyl)-2,3-dihydroimidazo[1,2-a]pyrimidin-5(1H)-one
- (S)-1-[2-(2-Fluoro-4,5-dimethoxy-phenyl)-2-hydroxy-ethyl]-2-methyl-7-morpholin-4-yl-2-trifluoromethyl-2,3-dihydro-1H-imidazo[1,2-a]pyrimidin-5-one
- (S)-1-[(S)-2-Hydroxy-2-(2-methoxy-phenyl)-ethyl]-2-methyl-7-morpholin-4-yl-2-trifluoromethyl-2,3-dihydro-1H-imidazo[1,2-a]pyrimidin-5-one
(S)-1-[(S)-2-(4-Chloro-2-methoxy-phenyl)-2-hydroxy-ethyl]-2-methyl-7-morpholin-4-yl-2-trifluoromethyl-2,3-dihydro-1H-imidazo[1,2-a]pyrimidin-5-one
(S)-1-[(S)-2-(4-Fluoro-2-methoxy-phenyl)-2-hyd roxy-ethyl]-2-methyl-7-morpholin-4-yl-2-trifluoromethyl-2,3-dihydro-1H-imidazo[1,2-a]pyrimidin-5-one
(S)-1-[(S)-2-(2-Chloro-4-methoxy-phenyl)-2-hydroxy-ethyl]-2-methyl-7-morpholin-4-yl-2-trifluoromethyl-2,3-dihydro-1H-imidazo[1,2-a]pyrimidin-5-one
ainsi que les sels d'addition avec les acides minéraux et organiques ou avec les bases minérales et organiques desdits produits de formule (I).

4. Procédé de préparation des produits de formule (I) tels que définis à l'une quelconque des revendications 1 à 3 selon le schéma 1 tel que défini ci-après. 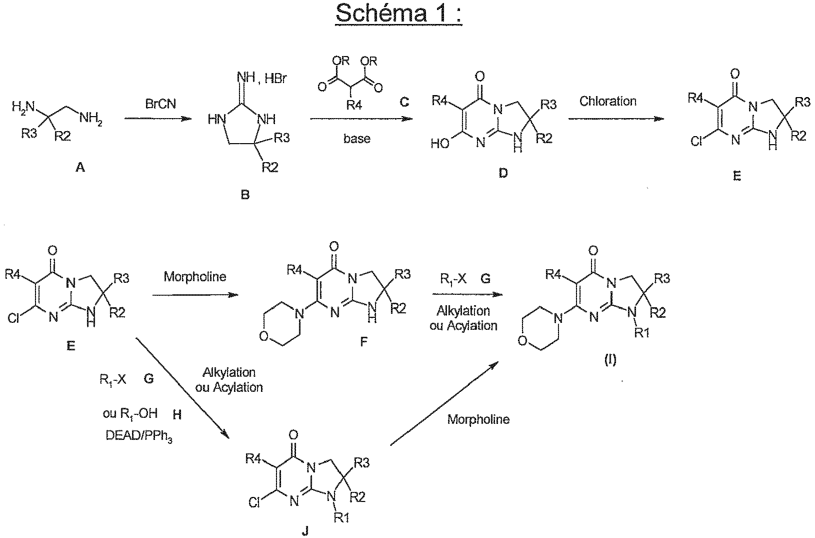 dans lequel les substituants R1, R2, R3 et R4 ont les significations indiquées à l'une quelconque des revendications 1 ou 2 et dans lequel R représente alkyle. X représente un atome de Chlore, brome ou iode ou un groupe sulfonyloxy tel que trifluorométhylsulfonyloxy.

5. A titre de médicaments, les produits de formule (I) telle que définie à l'une quelconque des revendications 1 à 3, ainsi que les sels d'addition avec les acides minéraux et organiques ou avec les bases minérales et organiques pharmaceutiquement acceptables desdits produits de formule (I).

6. A titre de médicaments, les produits de formule (I) telle que définie à la revendication 3, ainsi que les sels d'addition avec les acides minéraux et organiques ou avec les bases minérales et organiques pharmaceutiquement acceptables desdits produits de formule (I).

7. Compositions pharmaceutiques contenant à titre de principe actif l'un au moins des produits de formule (I) tel que défini à l'une quelconque des revendications 1 à 3, ou un sel pharmaceutiquement acceptable de ce produit et un support pharmaceutiquement acceptable.

8. Produits de formule (I) tels que définis à l'une quelconque des revendications 1 à 3 comme inhibiteurs de phosphorylation de AKT.

9. A titre de produits industriels, les intermédiaires de synthèse de formules D, E, F et J tels que définis à la revendication 4 ci-dessus et rappelés ci-après : 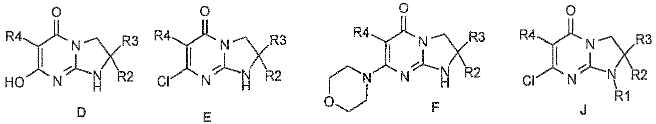 dans lesquels R1, R2, R3 et R4 ont les définitions indiquées à l'une quelconque des revendications 1 à 2.

10. Produits de formule (I) tels que définis à l'une quelconque des revendications 1 à 3 pour leur utilisation pour le traitement de cancers.

11. Produits de formule (I) tels que définis à l'une quelconque des revendications 1 à 3 pour leur utilisation pour le traitement de tumeurs solides ou liquides.

12. Produits de formule (I) tels que définis à l'une quelconque des revendications 1 à 3 pour leur utilisation pour le traitement de cancers résistant à des agents cytotoxiques.

13. Produits de formule (I) tels que définis à l'une quelconque des revendications 1 à 3 pour leur utilisation pour le traitement de tumeurs primaires et/ou de métastases.

14. Produits selon la revendication précédente pour leur utilisation pour le traitement de tumeurs primaires et/ou de métastases dans les cancers gastriques, hépatiques, rénaux, ovariens, du colon, de la prostate, de l'endomètre, du poumon , les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas, dans les hamartomes.

15. Produits de formule (I) tels que définis à l'une quelconque des revendications 1 à 3 pour leur utilisation pour la chimiothérapie de cancers.

16. Produits de formule (1) tels que définis à l'une quelconque des revendications 1 à 3 pour leur utilisation pour la chimiothérapie de cancers, seuls ou en association.

17. Produits de formule (I) tels que définis à l'une quelconque des revendications 1 à 3 pour la prévention ou le traitement de maladies lysosomales.

18. Produits selon la revendication précédente pour la prévention ou le traitement de la glycogénose de type II ou maladie de Pompe.

19. Produits tels que définis à la revendication 17 **caractérisés en ce qu'**ils sont utilisés seuls ou en association.

20. Produits de formule (I) tels que définis à l'une quelconque des revendications 1 à 3 pour le traitement de maladies parasitaires.

21. Produits selon la revendication précédente pour la prévention ou le traitement de la malaria, la maladie du sommeil, la maladie de Chagas, les leishmanioses.

## Patentansprüche

1. Produkte der Formel (I): 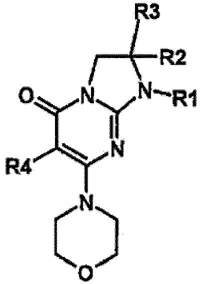 worin:
R1 für einen L-Aryl- oder L-Heteroarylrest steht, wobei L für:
einen linearen oder verzweigten Alkylrest, der 1 bis 6 Kohlenstoffatome enthält und gegebenenfalls durch einen Hydroxylrest substituiert ist,
oder eine CO-Gruppe
oder eine L'-X-Gruppe, wobei L' für einen linearen oder verzweigten Alkylrest mit 1 bis 6 Kohlenstoffatomen steht und X für ein Sauerstoff- oder
Schwefelatom steht;
steht;
wobei die Aryl- und Heteroarylreste gegebenenfalls durch einen oder mehrere gleiche oder verschiedene Reste, die aus Halogenatomen und Hydroxyl-, CN-, Nitro-, -COOH-, -COOalk-, -NRxRy-, -CONRxRy-, -NRxCORy-, -NRxCO₂Rz-, -CORy-, Alkoxy-, Phenoxy-, Alkylthio-, Alkyl-, Cycloalkyl- und Heterocycloalkylresten ausgewählt sind, substituiert sind;
wobei diese letztgenannten Alkoxy-, Phenoxy-, Alkylthio-, Alkyl- und Heterocycloalkylreste selbst gegebenenfalls durch einen oder mehrere gleiche oder verschiedene Reste, die aus Halogenatomen und NRvRw ausgewählt sind, substituiert sind;
wobei die Heterocycloalkyl- und Heteroarylreste außerdem einen Oxorest enthalten können;
R2 für ein Wasserstoffatom oder einen Alkylrest steht;
R3 für einen Alkylrest, der gegebenenfalls durch ein oder mehrere Halogenatome substituiert ist, steht;
R4 für ein Wasserstoffatom oder ein Halogenatom steht;
wobei NRxRy so beschaffen ist, dass Rx für ein Wasserstoffatom oder einen Alkylrest steht und Ry für ein Wasserstoffatom, einen Cycloalkylrest oder einen Alkylrest, der gegebenenfalls durch einen oder mehrere gleiche oder verschiedene Reste, die aus Hydroxyl-, Alkoxy-, NRvRw- und Heterocycloalkylresten ausgewählt sind, substituiert ist, steht; oder Rx und Ry mit dem Stickstoffatom, an das sie gebunden sind, einen 3- bis 10-gliedrigen cyclischen Rest bilden, der gegebenenfalls ein oder mehrere weitere aus 0, S, NH und N-Alkyl ausgewählte Heteroatome enthält und gegebenenfalls substituiert ist;
wobei NRvRw so beschaffen ist, dass Rv für ein Wasserstoffatom oder einen Alkylrest steht und Rw für ein Wasserstoffatom, einen Cycloalkylrest oder einen Alkylrest, der gegebenenfalls durch einen oder mehrere gleiche oder verschiedene Reste, die aus Hydroxyl-, Alkoxy- und Heterocycloalkylresten ausgewählt sind, substituiert ist, steht; oder Rv und Rw mit dem Stickstoffatom, an das sie gebunden sind, einen 3- bis 10-gliedrigen cyclischen Rest bilden, der gegebenenfalls ein oder mehrere weitere aus 0, S, NH und N-Alkyl ausgewählte Heteroatome enthält und gegebenenfalls substituiert ist;
wobei die cyclischen Reste, die Rx und Ry bzw. Rv und Rw bilden können, mit dem Stickstoffatom, an das sie gebunden sind, gegebenenfalls durch einen oder mehrere gleiche oder verschiedene Reste, die aus Halogenatomen und Alkyl-, Hydroxyl-, Oxo-, Alkoxy-, NH₂-, NHalk- und N(alk)₂-Resten ausgewählt sind, substituiert sind;
Rz für die Werte von Ry mit Ausnahme von Wasserstoff steht;
wobei Rx, Ry und Rz in den -NRxCORy-, -CORy- und NRxCO₂Rz-Resten aus den oben für Rx, Ry und Rz angegebenen Bedeutungen ausgewählt sind;
wobei alle obigen Alkyl- (alk-), Alkoxy- und Alkylthioreste linear oder verzweigt sind und 1 bis 6 Kohlenstoffatome enthalten,
wobei die Produkte der Formel (I) in allen möglichen racemischen, enantiomeren und diastereoisomeren isomeren Formen vorliegen, sowie die Additionssalze der Produkte der Formel (I) mit anorganischen und organischen Säuren oder mit anorganischen und organischen Basen.

2. Produkte der Formel (I) gemäß Anspruch 1, worin:
R1 für einen L-Phenyl- oder L-Heteroarylrest steht, wobei L für:
einen linearen oder verzweigten Alkylrest, der 1 bis 6 Kohlenstoffatome enthält und gegebenenfalls durch einen Hydroxylrest substituiert ist,
oder eine CO-Gruppe
oder eine L'-X-Gruppe, wobei L' für einen linearen oder verzweigten Alkylrest mit 1 bis 6 Kohlenstoffatomen steht und X für ein Sauerstoff- oder Schwefelatom steht;
steht;
wobei die Phenyl- und Heteroarylreste gegebenenfalls durch einen oder mehrere gleiche oder verschiedene Reste, die aus Halogenatomen und -NRxRy-, Alkoxy- und Alkylresten ausgewählt sind, substituiert sind;
wobei diese letztgenannten Alkoxy- und Alkylreste selbst gegebenenfalls durch einen oder mehrere Reste, die aus Halogenatomen ausgewählt sind, substituiert sind;
R2 für einen Alkylrest steht;
R3 für einen Alkylrest, der gegebenenfalls durch ein oder mehrere Halogenatome substituiert ist, steht;
R4 für ein Wasserstoffatom oder ein Fluoratom steht;
wobei NRxRy so beschaffen ist, dass Rx für ein Wasserstoffatom oder einen Alkylrest steht und Ry für ein Wasserstoffatom oder einen Alkylrest steht; oder Rx und Ry mit dem Stickstoffatom, an das sie gebunden sind, einen Morpholinorest bilden;
wobei alle obigen Alkyl- (alk-) oder Alkoxyreste linear oder verzweigt sind und 1 bis 6 Kohlenstoffatome enthalten,
wobei die Produkte der Formel (I) in allen möglichen racemischen, enantiomeren und diastereoisomeren isomeren Formen vorliegen, sowie die Additionssalze der Produkte der Formel (I) mit anorganischen und organischen Säuren oder mit anorganischen und organischen Basen.

3. Produkte der Formel (I) gemäß einem der Ansprüche 1 oder 2, die den folgenden Formeln entsprechen:
- (2S)-1-[2-(4-Methoxyphenyl)ethyl]-2-methyl-7-(morpholin-4-yl)-2-(trifluormethyl)-2,3-dihydroimidazo[1,2-a]pyrimidin-5(1H)-on
- 1-[2-(4-Methoxyphenyl)ethyl]-2-methyl-7-(morpholin-4-yl)-2-(trifluormethyl)-2,3-dihydroimidazo[1,2-a]pyrimidin-5(1H)-on
- (2S)-1-Benzyl-2-methyl-7-(morpholin-4-yl)-2-(trifluormethyl)-2,3-dihydroimidazo[1,2-a]pyrimidin-5(1H)-on
- (2S)-2-Methyl-7-(morpholin-4-yl)-1-(2-phenylethyl)-2-(trifluormethyl)-2,3-dihydroimidazo[1,2-a]pyrimidin-5(1H)-on
- (2S)-2-Methyl-7-(morpholin-4-yl)-1-(3-phenylpropyl)-2-(trifluormethyl)-2,3-dihydroimidazo[1,2-a]pyrimidin-5(1H)-on
- (2S)-2-Methyl-7-(morpholin-4-yl)-1-(2-phenoxyethyl)-2-(trifluormethyl)-2,3-dihydroimidazo[1,2-a]pyrimidin-5(1H)-on
- (2S)-2-Methyl-7-(morpholin-4-yl)-1-[2-(phenylsulfanyl)ethyl]-2-(trifluormethyl)-2,3-dihydroimidazo[1,2-a]pyrimidin-5(1H)-on
- (2S)-2-Methyl-7-(morpholin-4-yl)-1-[(2R)-2-phenylpropyl]-2-(trifluormethyl)-2,3-dihydroimidazo[1,2-a]pyrimidin-5(1H)-on
- (2S)-2-Methyl-7-(morpholin-4-yl)-1-[(2S)-2-phenylpropyl]-2-(trifluormethyl)-2,3-dihydroimidazo[1,2-a]pyrimidin-5(1H)-on
- (2S)-1-[(2S)-2-Hydroxy-2-phenylethyl]-2-methyl-7-(morpholin-4-yl)-2-(trifluormethyl)-2,3-dihydroimidazo[1,2-a]pyrimidin-5(1H)-on
- (2S)-1-[(2R)-2-Hydroxy-2-phenylethyl]-2-methyl-7-(morpholin-4-yl)-2-(trifluormethyl)-2,3-dihydroimidazo[1,2-a]pyrimidin-5(1H)-on
- (2S)-2-Methyl-7-(morpholin-4-yl)-1-[(2S)-1-phenylpropan-2-yl]-2-(trifluormethyl)-2,3-dihydroimidazo[1,2-a]pyrimidin-5(1H)-on
- (2S)-2-Methyl-7-(morpholin-4-yl)-1-[(1S)-1-phenylpropyl]-2-(trifluormethyl)-2,3-dihydroimidazo[1,2-a]pyrimidin-5(1H)-on
- (2S)-2-Methyl-7-(morpholin-4-yl)-1-[(1R)-1-phenylpropyl]-2-(trifluormethyl)-2,3-dihydroimidazo[1,2-a]pyrimidin-5(1H)-on
- (2S)-2-Methyl-7-(morpholin-4-yl)-1-{2-[4-(morpholin-4-yl)phenyl]ethyl}-2-(trifluormethyl)-2,3-dihydroimidazo[1,2-a]pyrimidin-5(1H)-on
- (2S)-2-Methyl-7-(morpholin-4-yl)-1-(1-phenylethyl)-2-(trifluormethyl)-2,3-dihydroimidazo[1,2-a]pyrimidin-5(1H)-on
- 1-[2-(4-Methoxyphenyl)ethyl]-2,2-dimethyl-7-(morpholin-4-yl)-2,3-dihydroimidazo[1,2-a]pyrimidin-5(1H)-on
- (2S)-6-Fluor-1-[2-(4-methoxyphenyl)ethyl]-2-methyl-7-(morpholin-4-yl)-2-(trifluormethyl)-2,3-dihydroimidazo[1,2-a]pyrimidin-5(1H)-on
- (2S)-1-Benzyl-6-fluor-2-methyl-7-(morpholin-4-yl)-2-(trifluormethyl)-2,3-dihydroimidazo[1,2-a]pyrimidin-5(1H)-on
- (2S)-1-[(5-Chlor-1-benzothiophen-3-yl)methyl]-2-methyl-7-(morpholin-4-yl)-2-(trifluormethyl)-2,3-dihydroimidazo[1,2-a]pyrimidin-5(1H)-on
- (2S)-2-Methyl-7-(morpholin-4-yl)-1-(phenylcarbonyl)-2-(trifluormethyl)-2,3-dihydroimidazo[1,2-a]pyrimidin-5(1H)-on
- (2S)-1-[(1R oder 1S)-1-(3-Fluorphenyl)ethyl]-2-methyl-7-(morpholin-4-yl)-2-(trifluormethyl)-2,3-dihydroimidazo[1,2-a]pyrimidin-5(1H)-on
- (2S)-1-{[4-Chlor-2-(trifluormethyl)chinolin-6-yl]methyl}-2-methyl-7-(morpholin-4-yl)-2-(trifluormethyl)-2,3-dihydroimidazo[1,2-a]pyrimidin-5(1H)-on-trifluoracetat
- (2S)-1-(3-Brom-4-fluorbenzyl)-2-methyl-7-(morpholin-4-yl)-2-(trifluormethyl)-2,3-dihydroimidazo[1,2-a]pyrimidin-5(1H)-on
- (2S)-1-(2,3-Difluorbenzyl)-2-methyl-7-(morpholin-4-yl)-2-(trifluormethyl)-2,3-dihydroimidazo[1,2-a]pyrimidin-5(1H)-on
- (2S)-1-[2-(3-Methoxyphenyl)ethyl]-2-methyl-7-(morpholin-4-yl)-2-(trifluormethyl)-2,3-dihydroimidazo[1,2-a]pyrimidin-5(1H)-on
- (2S)-1-[2-(2-Chlorphenyl)ethyl]-2-methyl-7-(morpholin-4-yl)-2-(trifluormethyl)-2,3-dihydroimidazo[1,2-a]pyrimidin-5(1H)-on
- (2S)-1-[2-(4-Chlorphenyl)ethyl]-2-methyl-7-(morpholin-4-yl)-2-(trifluormethyl)-2,3-dihydroimidazo[1,2-a]pyrimidin-5(1H)-on
- (2S)-1-[2-(3-Chlorphenyl)ethyl]-2-methyl-7-(morpholin-4-yl)-2-(trifluormethyl)-2,3-dihydroimidazo[1,2-a]pyrimidin-5(1H)-on
- (2S)-1-(1,3-Benzoxazol-2-ylmethyl)-2-methyl-7-(morpholin-4-yl)-2-(trifluormethyl)-2,3-dihydroimidazo[1,2-a]pyrimidin-5(1H)-on
- (2S)-2-Methyl-7-(morpholin-4-yl)-1-[(1R oder 1S)-1-phenylethyl]-2-(trifluormethyl)-2,3-dihydroimidazo[1,2-a]pyrimidin-5(1H)-on
- (2S)-2-Methyl-7-(morpholin-4-yl)-1-[(1R oder 1S)-1-phenylethyl]-2-(trifluormethyl)-2,3-dihydroimidazo[1,2-a]pyrimidin-5(1H)-on
- (2S)-1-(1H-Indol-3-ylmethyl)-2-methyl-7-(morpholin-4-yl)-2-(trifluormethyl)-2,3-dihydroimidazo[1,2-a]pyrimidin-5(1H)-on
- (2S)-1-[(2-Chlorphenyl)carbonyl]-2-methyl-7-(morpholin-4-yl)-2-(trifluormethyl)-2,3-dihydroimidazo[1,2-a]pyrimidin-5(1H)-on
- (2S)-2-Methyl-1-[(2-methylphenyl)carbonyl]-7-(morpholin-4-yl)-2-(trifluormethyl)-2,3-dihydroimidazo[1,2-a]pyrimidin-5(1H)-on
- (2S)-1-[(1R oder 1S)-1-(2-Fluorphenyl)ethyl]-2-methyl-7-(morpholin-4-yl)-2-(trifluormethyl)-2,3-dihydroimidazo[1,2-a]pyrimidin-5(1H)-on
- (2S)-1-[(1R oder 1S)-1-(2-Fluorphenyl)ethyl]-2-methyl-7-(morpholin-4-yl)-2-(trifluormethyl)-2,3-dihydroimidazo[1,2-a]pyrimidin-5(1H)-on
- (S)-1-[2-(2-Fluor-4,5-dimethoxyphenyl)-2-hydroxyethyl]-2-methyl-7-morpholin-4-yl-2-trifluormethyl-2,3-dihydro-1H-imidazo[1,2-a]pyrimidin-5-on
- (S)-1-[(S)-2-Hydroxy-2-(2-methoxyphenyl)ethyl]-2-methyl-7-morpholin-4-yl-2-trifluormethyl-2,3-dihydro-1H-imidazo[1,2-a]pyrimidin-5-on
- (S)-1-[(S)-2-(4-Chlor-2-methoxyphenyl)-2-hydroxyethyl]-2-methyl-7-morpholin-4-yl-2-trifluormethyl-2,3-dihydro-1H-imidazo[1,2-a]pyrimidin-5-on
- (S)-1-[(S)-2-(4-Fluor-2-methoxyphenyl)-2-hydroxyethyl]-2-methyl-7-morpholin-4-yl-2-trifluormethyl-2,3-dihydro-1H-imidazo[1,2-a]pyrimidin-5-on
- (S)-1-[(S)-2-(2-Chlor-4-methoxyphenyl)-2-hydroxyethyl]-2-methyl-7-morpholin-4-yl-2-trifluormethyl-2,3-dihydro-1H-imidazo[1,2-a]pyrimidin-5-on
sowie die Additionssalze der Produkte der Formel (I) mit anorganischen und organischen Säuren oder mit anorganischen und organischen Basen.

4. Verfahren zur Herstellung der Produkte der Formel (I) gemäß einem der Ansprüche 1 bis 3 gemäß Schema 1 gemäß nachstehender Definition: 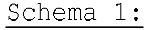 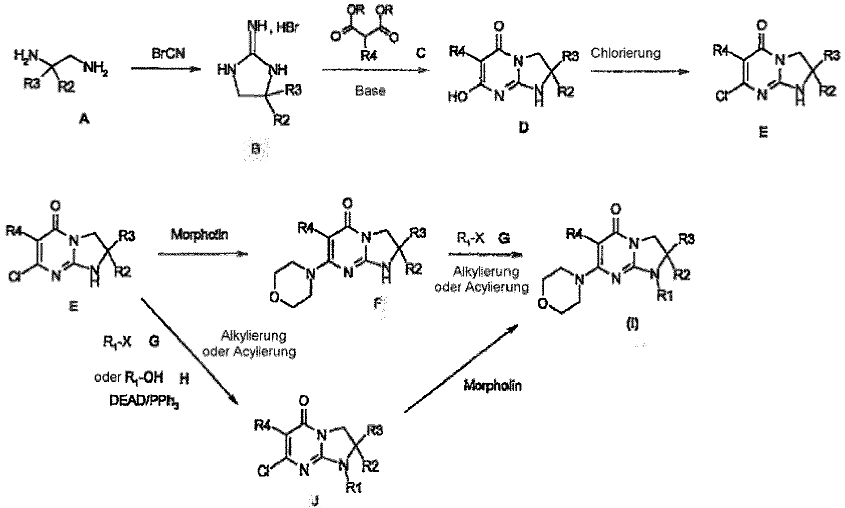 wobei die Substituenten R1, R2, R3 und R4 die in einem der Ansprüche 1 oder 2 angegebenen Bedeutungen besitzen und R für Alkyl steht und X für ein Chlor-, Brom- oder Iodatom oder eine Sulfonyloxygruppe wie Trifluormethylsulfonyloxy steht.

5. Produkte der Formel (I) gemäß einem der Ansprüche 1 bis 3 sowie die Additionssalze der Produkte der Formel (I) mit anorganischen und organischen Säuren oder mit anorganischen und organischen Basen, die pharmazeutisch unbedenklich sind, als Medikamente.

6. Produkte der Formel (I) gemäß Anspruch 3 sowie die Additionssalze der Produkte der Formel (I) mit anorganischen und organischen Säuren oder mit anorganischen und organischen Basen, die pharmazeutisch unbedenklich sind, als Medikamente.

7. Pharmazeutische Zusammensetzungen, die als Wirkstoff mindestens eines der Produkte der Formel (I) gemäß einem der Ansprüche 1 bis 3 oder ein pharmazeutisch unbedenkliches Salz dieses Produkts und einen pharmazeutisch unbedenklichen Träger enthalten.

8. Produkte der Formel (I) gemäß einem der Ansprüche 1 bis 3 als Inhibitoren der AKT-Phosphorylierung.

9. Synthesezwischenprodukte der Formeln D, E, F und J gemäß Anspruch 4, die nachstehend noch einmal aufgeführt sind: 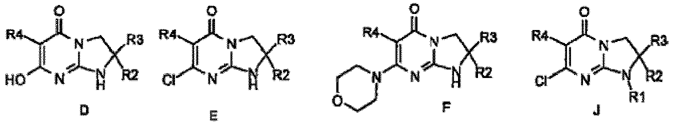 wobei R1, R2, R3 und R4 die in einem der Ansprüche 1 bis 2 angegebenen Bedeutungen besitzen, als technische Produkte.

10. Produkte der Formel (I) gemäß einem der Ansprüche 1 bis 3 zur Verwendung für die Behandlung von Krebserkrankungen.

11. Produkte der Formel (I) gemäß einem der Ansprüche 1 bis 3 zur Verwendung für die Behandlung von soliden oder flüssigen Tumoren.

12. Produkte der Formel (I) gemäß einem der Ansprüche 1 bis 3 zur Verwendung für die Behandlung von Krebserkrankungen, die gegenüber Zytotoxika resistent sind.

13. Produkte der Formel (I) gemäß einem der Ansprüche 1 bis 3 zur Verwendung für die Behandlung von primären Tumoren und/oder Metastasen.

14. Produkte nach dem vorhergehenden Anspruch zur Verwendung für die Behandlung von primären Tumoren und/oder Metastasen bei Krebserkrankungen des Magens, der Leber, der Niere, der Eierstöcke, des Kolons, der Prostata, des Endometriums, der Lunge, Glioblastomen, Krebserkrankungen der Schilddrüse, der Blase, der Brust, bei Melanom, bei lymphoiden oder myeloiden hämatopoetischen Tumoren, bei Sarkomen, bei Krebserkrankungen des Gehirns, des Kehlkopfs, des Lymphsystems, Krebserkrankungen der Knochen und der Bauchspeicheldrüse und bei Hamartomen.

15. Produkte der Formel (I) gemäß einem der Ansprüche 1 bis 3 zur Verwendung für die Chemotherapie von Krebserkrankungen.

16. Produkte der Formel (I) gemäß einem der Ansprüche 1 bis 3 zur Verwendung für die Chemotherapie von Krebserkrankungen, allein oder in Kombination.

17. Produkte der Formel (I) gemäß einem der Ansprüche 1 bis 3 zur Prävention oder Behandlung von lysosomalen Krankheiten.

18. Produkte nach dem vorhergehenden Anspruch zur Prävention oder Behandlung von Glykogenose Typ II oder Pompe-Krankheit.

19. Produkte gemäß Anspruch 17, **dadurch gekennzeichnet, dass** sie allein oder in Kombination verwendet werden.

20. Produkte der Formel (I) gemäß einem der Ansprüche 1 bis 3 zur Behandlung von Parasitenkrankheiten.

21. Produkte nach dem vorhergehenden Anspruch zur Prävention oder Behandlung von Malaria, Schlafkrankheit, Chagas-Krankheit oder Leishmaniosen.

## Claims

1. Products of formula (I): 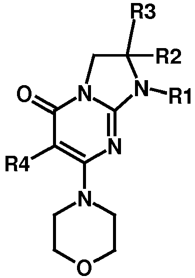 in which:
R1 represents an -L-aryl or -L-heteroaryl radical, such that L represents:
either a linear or branched alkyl radical containing from 1 to 6 carbon atoms and optionally substituted with a hydroxyl radical,
or a CO group,
or an L'-X group where L' represents a linear or branched alkyl radical containing from 1 to 6 carbon atoms, and X an oxygen or sulphur atom;
the aryl and heteroaryl radicals being optionally substituted with one or more radicals, which may be identical or different, chosen from halogen atoms and hydroxyl, CN, nitro-, -COOH, -COOalk, -NRxRy, -CONRxRy, -NRxCORy, -NRxCO₂Rz, -CORy, alkoxy, phenoxy, alkylthio, alkyl, cycloalkyl and heterocycloalkyl radicals;
the latter alkoxy, phenoxy, alkylthio, alkyl and heterocycloalkyl radicals being themselves optionally substituted with one or more radicals, which may be identical or different, chosen from halogen atoms and NRvRw;
it being possible for the heterocycloalkyl and heteroaryl radicals to additionally contain an oxo radical;
R2 represents a hydrogen atom or an alkyl radical;
R3 represents an alkyl radical optionally substituted with one or more halogen atoms;
R4 represents a hydrogen atom or a halogen atom;
NRxRy being such that Rx represents a hydrogen atom or an alkyl radical and Ry represents a hydrogen atom or a cycloalkyl radical or an alkyl radical optionally substituted with one or more radicals, which may be identical or different, chosen from hydroxyl, alkoxy, NRvRw and heterocycloalkyl radicals; or Rx and Ry form, with the nitrogen atom to which they are attached, a cyclic radical containing from 3 to 10 ring members and optionally one or more other heteroatoms chosen from 0, S, NH and N-alkyl, this cyclic radical being optionally substituted;
NRvRw being such that Rv represents a hydrogen atom or an alkyl radical and Rw represents a hydrogen atom or a cycloalkyl radical or an alkyl radical optionally substituted with one or more radicals, which may be identical or different, chosen from hydroxyl, alkoxy and heterocycloalkyl radicals; or Rv and Rw form, with the nitrogen atom to which they are attached, a cyclic radical containing from 3 to 10 ring members and optionally one or more other heteroatoms chosen from 0, S, NH and N-alkyl, this cyclic radical being optionally substituted;
the cyclic radicals that Rx and Ry or Rv and Rw, respectively, can form with the nitrogen atom to which they are attached, being optionally substituted with one or more radicals, which may be identical or different, chosen from halogen atoms, and alkyl, hydroxyl, oxo, alkoxy, NH2, NHalk and N(alk)2 radicals; Rz represents the values of Ry except for hydrogen;
Rx, Ry and Rz, in the -NRxCORy, -CORy and NR_{X}CO₂Rz radicals, being chosen from the meanings indicated above for Rx, Ry and Rz;
all the above alkyl (alk), alkoxy and alkylthio radicals being linear or branched and containing from 1 to 6 carbon atoms,
said products of formula (I) being in all the possible racemic, enantiomeric and diastereoisomeric isomer forms, and also the addition salts with inorganic and organic acids or with inorganic and organic bases, of said products of formula (I).

2. Products of formula (I) as defined in Claim 1, in which:
R1 represents an -L-phenyl or -L-heteroaryl radical, such that L represents:
either a linear or branched alkyl radical containing from 1 to 6 carbon atoms and optionally substituted with a hydroxyl radical,
or a CO group,
or an L'-X group, where L' represents a linear or branched alkyl radical containing from 1 to 6 carbon atoms, and X an oxygen or sulphur atom;
the phenyl and heteroaryl radicals being optionally substituted with one or more radicals, which may be identical or different, chosen from halogen atoms and-NRxRy, alkoxy and alkyl radicals;
the latter alkoxy and alkyl radicals being themselves optionally substituted with one or more radicals chosen from halogen atoms;
R2 represents an alkyl radical;
R3 represents an alkyl radical optionally substituted with one or more halogen atoms;
R4 represents a hydrogen atom or a fluorine atom;
NRxRy being such that Rx represents a hydrogen atom or an alkyl radical and Ry represents a hydrogen atom or an alkyl radical; or Rx and Ry form, with the nitrogen atom to which they are attached, a morpholino radical;
all the above alkyl(alk) or alkoxy radicals being linear or branched and containing from 1 to 6 carbon atoms,
said products of formula (I) being in all the possible racemic, enantiomeric and diastereoisomeric isomer forms, and also the addition salts with inorganic and organic acids or with inorganic and organic bases, of said products of formula (I).

3. Products of formula (I) as defined in either one of Claims 1 and 2, corresponding to the following formulae:
- (2S)-1-[2-(4-methoxyphenyl)ethyl]-2-methyl-7-(morpholin-4-yl)-2-(trifluoromethyl)-2,3dihydroimidazo[1,2-a]pyrimidin-5(1H)one
- 1-[2-(4-methoxyphenyl)ethyl]-2-methyl-7-(morpholin-4-yl)-2-(trifluoromethyl)-2,3-dihydroimidazo[1,2-a]pyrimidin-5(1H)one
- (2S)-1-benzyl-2-methyl-7-(morpholin-4-yl)-2-(trifluoromethyl)-2,3-dihydroimidazo[1,2-a]pyrimidin-5(1H)one
- (2S)-2-methyl-7-(morpholin-4-yl)-1-(2-phenylethyl)-2-(trifluoromethyl)-2,3-dihydroimidazo[1,2-a]pyrimidin-5(1H)one
- (2S)-2-methyl-7-(morpholin-4-yl)-1-(3-phenylpropyl)-2-(trifluoromethyl)-2,3-dihydroimidazo[1,2-a]pyrimidin-5(1H)one
- (2S)-2-methyl-7-(morpholin-4-yl)-1-(2-phenoxyethyl)-2-(trifluoromethyl)-2,3-dihydroimidazo[1,2-a]pyrimidin-5(1H)one
- (2S)-2-methyl-7-(morpholin-4-yl)-1-[2-(phenylsulphanyl)ethyl]-2-(trifluoromethyl)-2,3-dihydroimidazo[1,2-a]pyrimidin-5(1H)one
- (2S)-2-methyl-7-(morpholin-4-yl)-1-[(2R)-2-phenylpropyl]-2-(trifluoromethyl)-2,3-dihydroimidazo[1,2-a]pyrimidin-5(1H)one
- (2S)-2-methyl-7-(morpholin-4-yl)-1-[(2S)-2-phenylpropyl]-2-(trifluoromethyl)-2,3-dihydroimidazo[1,2-a]pyrimidin-5(1H)one
- (2S)-1-[(2S)-2-hydroxy-2-phenylethyl]-2-methyl-7-(morpholin-4-yl)-2-(trifluoromethyl)-2,3-dihydroimidazo[1,2-a]pyrimidin-5(1H)one
- (2S)-1-[(2R)-2-hydroxy-2-phenylethyl]-2-methyl-7-(morpholin-4-yl)-2-(trifluoromethyl)-2,3-dihydroimidazo[1,2-a]pyrimidin-5(1H)one
- (2S)-2-methyl-7-(morpholin-4-yl)-1-[(2S)-1-phenylpropan-2-yl]-2-(trifluoromethyl)-2,3-dihydroimidazo[1,2-a]pyrimidin-5(1H)one
- (2S)-2-methyl-7-(morpholin-4-yl)-1-[(1S)-1-phenylpropyl]-2-(trifluoromethyl)-2,3-dihydroimidazo[1,2-a]pyrimidin-5(1H)one
- (2S)-2-methyl-7-(morpholin-4-yl)-1-[(1R)-1-phenylpropyl]-2-(trifluoromethyl)-2,3-dihydroimidazo[1,2-a]pyrimidin-5(1H)one
- (2S)-2-methyl-7-(morpholin-4-yl)-1-{2-[4-(morpholin-4-yl)phenyl]ethyl}-2-(trifluoromethyl)-2,3-dihydroimidazo[1,2-a]pyrimidin-5(1H)one
- (2S)-2-methyl-7-(morpholin-4-yl)-1-(1-phenylethyl)-2-(trifluoromethyl)-2,3-dihydroimidazo[1,2-a]pyrimidin-5(1H)one
- 1-[2-(4-methoxyphenyl)ethyl]-2,2-dimethyl-7-(morpholin-4-yl)-2,3-dihydroimidazo[1,2-a]pyrimidin-5(1H)one
- (2S)-6-fluoro-1-[2-(4-methoxyphenyl)ethyl]-2-methyl-7-(morpholin-4-yl)-2-(trifluoromethyl)-2,3-dihydroimidazo[1,2-a]pyrimidin-5(1H)one
- (2S)-1-benzyl-6-fluoro-2-methyl-7-(morpholin-4-yl)-2-(trifluoromethyl)-2,3-dihydroimidazo[1,2-a]pyrimidin-5(1H)one
- (2S)-1-[(5-chloro-1-benzothiophen-3-yl)methyl]-2-methyl-7-(morpholin-4-yl)-2-(trifluoromethyl)-2,3-dihydroimidazo[1,2-a]pyrimidin-5(1H)one
- (2S)-2-methyl-7-(morpholin-4-yl)-1-(phenylcarbonyl)-2-(trifluoromethyl)-2,3-dihydroimidazo[1,2-a]pyrimidin-5(1H)one
- (2S)-1-[(1R or 1S)-1-(3-fluorophenyl)ethyl]-2-methyl-7-(morpholin-4-yl)-2-(trifluoromethyl)-2,3-dihydroimidazo[1,2-a]pyrimidin-5(1H)one
- (2S)-1-{[4-chloro-2-(trifluoromethyl)quinolin-6-yl]methyl}-2-methyl-7-(morpholin-4-yl)-2-(trifluoromethyl)-2,3-dihydroimidazo[1,2-a]pyrimidin-5(1H)one trifluoroacetate
- (2S)-1-(3-bromo-4-fluorobenzyl)-2-methyl-7-(morpholin-4-yl)-2-(trifluoromethyl)-2,3-dihydroimidazo[1,2-a]pyrimidin-5(1H)one
- (2S)-1-(2,3-difluorobenzyl)-2-methyl-7-(morpholin-4-yl)-2-(trifluoromethyl)-2,3-dihydroimidazo[1,2-a]pyrimidin-5(1H)one
- (2S)-1-[2-(3-methoxyphenyl)ethyl]-2-methyl-7-(morpholin-4-yl)-2-(trifluoromethyl)-2,3-dihydroimidazo[1,2-a]pyrimidin-5(1H)one
- (2S)-1-[2-(2-chlorophenyl)ethyl]-2-methyl-7-(morpholin-4-yl)-2-(trifluoromethyl)-2,3-dihydroimidazo[1,2-a]pyrimidin-5(1H)one
- (2S)-1-[2-(4-chlorophenyl)ethyl]-2-methyl-7-(morpholin-4-yl)-2-(trifluoromethyl)-2,3-dihydroimidazo[1,2-a]pyrimidin-5(1H)one
- (2S)-1-[2-(3-chlorophenyl)ethyll-2-methyl-7-(morpholin-4-yl)-2-(trifluoromethyl)-2,3-dihydroimidazo[1,2-a]pyrimidin-5(1H)one
- (2S)-1-(1,3-benzoxazol-2-ylmethyl)-2-methyl-7-(morpholin-4-yl)-2-(trifluoromethyl)-2,3-dihydroimidazo[1,2-a]pyrimidin-5(1H)one
- (2S)-2-methyl-7-(morpholin-4-yl)-1-[(1R or 1S)-1-phenylethyl]-2-(trifluoromethyl)-2,3-dihydroimidazo[1,2-a]pyrimidin-5(1H)one
- (2S)-2-methyl-7-(morpholin-4-yl)-1-[(1R or 1S)-1-phenylethyl]-2-(trifluoromethyl)-2,3-dihydroimidazo[1,2-a]pyrimidin-5(1H)one
- (2S)-1-(1H-indol-3-ylmethyl)-2-methyl-7-(morpholin-4-yl)-2-(trifluoromethyl)-2,3-dihydroimidazo[1,2-a]pyrimidin-5(1H)one
- (2S)-1-[(2-chlorophenyl)carbonyl]-2-methyl-7-(morpholin-4-yl)-2-(trifluoromethyl)-2,3-dihydroimidazo[1,2-a]pyrimidin-5(1H)one
- (2S)-2-methyl-1-[(2-methylphenyl)carbonyl]-7-(morpholin-4-yl)-2-(trifluoromethyl)-2,3-dihydroimidazo[1,2-a]pyrimidin-5(1H)one
- (2S)-1-[(1R or 1S)-1-(2-fluorophenyl)ethyl]-2-methyl-7-(morpholin-4-yl)-2-(trifluoromethyl)-2,3-dihydroimidazo[1,2-a]pyrimidin-5(1H)one
- (2S)-1-[(1R or 1S)-1-(2-fluorophenyl)ethyl]-2-methyl-7-(morpholin-4-yl)-2-(trifluoromethyl)-2,3-dihydroimidazo[1,2-a]pyrimidin-5(1H)one
- (S)-1-[2-(2-fluoro-4,5-dimethoxyphenyl)-2-hydroxyethyl]-2-methyl-7-morpholin-4-yl-2-trifluoromethyl-2,3-dihydro-1H-imidazo[1,2-a]pyrimidin-5-one
- (S)-1-[(S)-2-hydroxy-2-(2-methoxyphenyl)ethyl]-2-methyl-7-morpholin-4-yl-2-trifluoromethyl-2,3-dihydro-1H-imidazo[1,2-a]pyrimidin-5-one
- (S)-1-[(S)-2-(4-chloro-2-methoxyphenyl)-2-hydroxyethyl]-2-methyl-7-morpholin-4-yl-2-trifluoromethyl-2,3-dihydro-1H-imidazo[1,2-a]pyrimidin-5-one
- (S)-1-[(S)-2-(4-fluoro-2-methoxyphenyl)-2-hydroxyethyl]-2-methyl-7-morpholin-4-yl-2-trifluoromethyl-2,3-dihydro-1H-imidazo[1,2-a]pyrimidin-5-one
- (S)-1-[(S)-2-(2-chloro-4-methoxyphenyl)-2-hydroxyethyl]-2-methyl-7-morpholin-4-yl-2-trifluoromethyl-2,3-dihydro-1H-imidazo[1,2-a]pyrimidin-5-one
and also the addition salts with inorganic and organic acids or with inorganic and organic bases, of said products of formula (I).

4. Process for preparing the products of formula (I) as defined in any one of Claims 1 to 3 according to Scheme 1 as defined hereinafter. 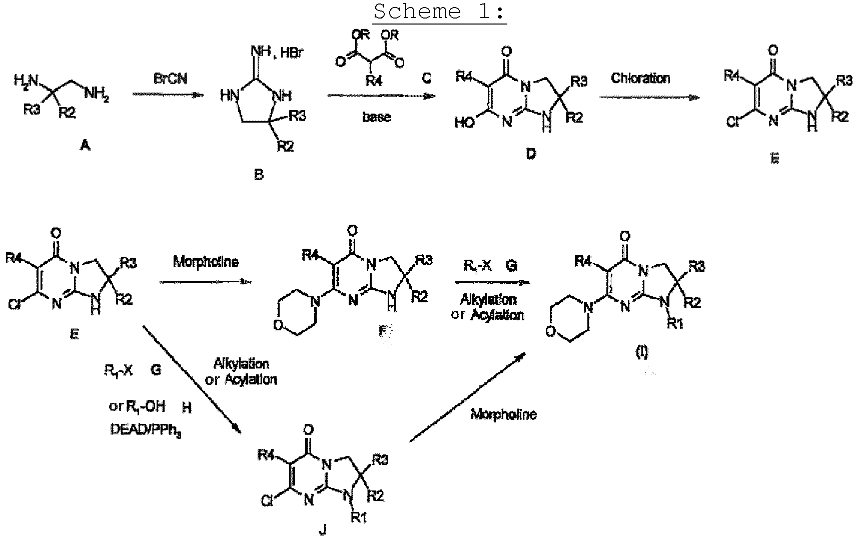 in which the substituents R1, R2, R3 and R4 have the meanings indicated in either one of Claims 1 and 2 and in which R represents alkyl, and X represents a chlorine, bromine or iodine atom or a sulphonyloxy group such as trifluoromethylsulphonyloxy.

5. As medicaments, the products of formula (I) as defined in any one of Claims 1 to 3, and also the pharmaceutically acceptable addition salts with inorganic and organic acids or with inorganic and organic bases, of said products of formula (I).

6. As medicaments, the products of formula (I) as defined in Claim 3, and also the pharmaceutically acceptable addition salts with inorganic and organic acids or with inorganic and organic bases, of said products of formula (I).

7. Pharmaceutical compositions containing, as active ingredient, at least one of the products of formula (I) as defined in any one of Claims 1 to 3, or a pharmaceutically acceptable salt of this product, and a pharmaceutically acceptable carrier.

8. Products of formula (I) as defined in any one of Claims 1 to 3, as inhibitors of AKT phosphorylation.

9. As industrial products, the synthesis intermediates of formulae D, E, F and J as defined in Claim 4 above and recalled below: 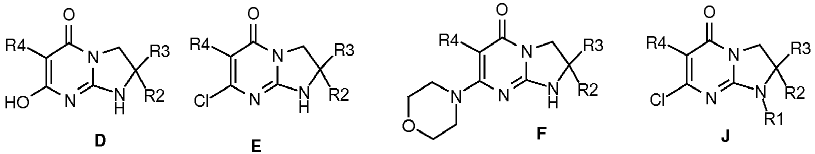 in which R1, R2, R3 and R4 have the definitions indicated in either one of Claims 1 and 2.

10. Products of formula (I) as defined in any one of Claims 1 to 3, for the use thereof in the treatment of cancers.

11. Products of formula (I) as defined in any one of Claims 1 to 3, for the use thereof in the treatment of solid or liquid tumours.

12. Products of formula (I) as defined in any one of Claims 1 to 3, for the use thereof in the treatment of cancers resistant to cytotoxic agents.

13. Products of formula (I) as defined in any one of Claims 1 to 3, for the use thereof in the treatment of primary tumours and/or metastases.

14. Products according to the preceding claim, for the use thereof in the treatment of primary tumours and/or metastases in gastric, hepatic, renal, ovarian, colon, prostate, endometrial and lung cancers, glioblastomas, thyroid, bladder and breast cancers, in melanoma, in lymphoid or myeloid haematopoietic tumours, in sarcomas, in brain, larynx and lymphatic system cancers, bone and pancreatic cancers, and in hamartomas.

15. Products of formula (I) as defined in any one of Claims 1 to 3, for the use thereof in cancer chemotherapy.

16. Products of formula (I) as defined in any one of Claims 1 to 3, for the use thereof in cancer chemotherapy, alone or in combination.

17. Products of formula (I) as defined in any one of Claims 1 to 3, for the prevention or treatment of lysosomal diseases.

18. Products according to the preceding claim, for the prevention or treatment of glycogenosis type II or Pompe disease.

19. Products as defined in Claim 17, **characterized in that** they are used alone or in combination.

20. Products of formula (I) as defined in any one of Claims 1 to 3, for the treatment of parasitic diseases.

21. Products according to the preceding claim, for the prevention or treatment of malaria, sleeping sickness, Chagas disease or leishmaniasis.
